# EUROPEAN PATENT SPECIFICATION

(11) **EP 1 979 000 B1**
(45) Date of publication and mention of the grant of the patent: **13.07.2011**
(21) Application number: 06847911.2
(22) Date of filing: 21.12.2006
(51) Int. Cl.: C12Q 1/68

(54) **METHOD FOR DETECTION OF CANCER CELLS USING VIRUS**
VERFAHREN ZUR ERFASSUNG VON KREBSZELLEN UNTER VERWENDUNG EINES VIRUS
PROCEDE DE DETECTION DE CELLULES CANCEREUSES UTILISANT UN VIRUS

(30) Priority: 22.12.2005 US 753702 P
(43) Date of publication of application: 15.10.2008
(73) Proprietor: MEMORIAL SLOAN-KETTERING CANCER CENTER, New York, NY 10021 (US)
(72) Inventor: FONG, Yuman, New York, NY 10021 (US); ADUSUMILLI, Prasad S., New York, NY 10065 (US)
(74) Representative: Glawe, Delfs, Moll
(86) International application number: PCT/US2006/048777
(87) International publication number: WO 2007/075879

(56) References cited:
- EP-A1- 1 281 772
- WO-A2-2005/047458
- STANZIALE STEPHEN F ET AL: "Oncolytic herpes simplex virus-1 mutant expressing green fluorescent protein can detect and treat peritoneal cancer" HUMAN GENE THERAPY, vol. 15, no. 6, June 2004 (2004-06), pages 609-618, XP002435635 ISSN: 1043-0342
- STILES BRENDON M ET AL: "The replication-competent oncolytic herpes simplex mutant virus NV1066 is effective in the treatment of esophageal cancer." SURGERY (ST LOUIS), vol. 134, no. 2, August 2003 (2003-08), pages 357-364, XP002435636 ISSN: 0039-6060
- WOLLMANN G ET AL: "Targeting Human Glioblastoma Cells: Comparison of Nine Viruses with Oncolytic Potential" JOURNAL OF VIROLOGY, THE AMERICAN SOCIETY FOR MICROBIOLOGY, US, vol. 79, no. 10, May 2005 (2005-05), pages 6005-6022, XP003000393 ISSN: 0022-538X
- STANZIALE STEPHEN F ET AL: "Oncolytic herpes simplex virus-1 mutant expressing green fluorescent protein can detect and treat peritoneal cancer" HUMAN GENE THERAPY, vol. 15, no. 6, June 2004 (2004-06), pages 609-618, XP002435635 ISSN: 1043-0342
- STILES BRENDON M ET AL: "The replication-competent oncolytic herpes simplex mutant virus NV1066 is effective in the treatment of esophageal cancer." SURGERY (ST LOUIS), vol. 134, no. 2, August 2003 (2003-08), pages 357-364, XP002435636 ISSN: 0039-6060
- WOLLMANN G ET AL: "Targeting Human Glioblastoma Cells: Comparison of Nine Viruses with Oncolytic Potential" JOURNAL OF VIROLOGY, THE AMERICAN SOCIETY FOR MICROBIOLOGY, US, vol. 79, no. 10, May 2005 (2005-05), pages 6005-6022, XP003000393 ISSN: 0022-538X
- FONG S M B ET AL: "Fluorescence-expressing viruses allow rapid identification and separation of rare tumor cells in spiked samples of human whole blood" SURGERY, C.V. MOSBY CO., ST. LOUIS, US, vol. 146, no. 3, 1 September 2009 (2009-09-01), pages 498-505, XP026518400 ISSN: 0039-6060 [retrieved on 2009-08-26]

## Description

### FIELD OF THE INVENTION

The invention relates to compositions and methods for cancer cell detection in bodily samples wherein a cancer cell can be detected within a mixed population of cancer cells and non-cancer cells. The invention also relates to compositions and methods that may be used in cancer cell detection, specifically viruses that are replication-competent conditional to a cancer cell, in particular an oncolytic herpes virus, such as NV1066 and a vaccinia virus, such as GLV-1h68. The invention relates to methods and kits for using these viruses that preferentially replicate in cancer cells and may also preferentially infect cancer cells for specific identification of such cancer cells, even when a cancer cell is present, for example, at a ratio of one infected cancer cell in a background of ten thousand non-cancer cells, thus further providing a reproducible and sensitive screening method for cancer detection, monitoring and prognosis.

### BACKGROUND

Screening methods for detecting cancer in high-risk individuals aim to find cancers at an early and potentially curable stage. For one example, cytological analysis of body fluids such as sputum or urine is currently used in screening for lung and bladder cancers, but is hindered by the difficulties in detecting the rare tumor cell within the background of vast numbers of non-cancerous cells.

Early detection of cancer before it has had a chance to metastasize remains the best strategy for reducing cancer mortality. In bladder and lung cancer, for example, 5 - 15% of patients have tumor localized to the organ of origin at the time of diagnosis (The Early Detection Research Network second report, October 2002. United States Department of Health and Human Services, National Institutes of Health, National Cancer Institute 2003; ). The cure rate for lung cancer in patients without distant or loco-regional tumor spread is > 70%, compared to < 10% survival when the cancer is diagnosed in stage 4 (Kennedy et al., 2000, Chest 117(4 Suppl 1):72S-79S; ).

Screening methods directed at early detection of cancers in individuals at high risk have been used with the aim of identifying cancers at a potentially curable stage (Bunn 2002, Lung Cancer 38(1):S5-S8). One routinely used method for screening high risk patients involves microscopic examination of body fluids (*e.g*. sputum, urine, and the like) for the presence of tumor cells (Thunnissen 2003, J Clin Pathol 56(11):805-810). Such cytological tests are labor-intensive and are highly dependent on the skill of the cytopathologists. The sensitivity of such sputum or urinary cytology studies is also governed by technical limitations of identifying the few cancer cells in the background of many normal cells.

There is also a commercially available method for detecting and quantifying circulating cancer cells (Cristofanilli et al., 2004, N Engl J Med. 351(8):781-91; ). However this method is based on cytokeratin staining, which has the limitation that it does not identify every cancer cell type. Added to that, the staining has physical limitations since staining is not uniform in the presence of cell clumps, which are often found in the type of preparations obtained from blood or other sources.

Therefore, there is a need for routine and simple methods that will identify a rare cancer cell in large populations of non-cancer cells, particularly in cell mixtures obtained from bodily fluids.

### SUMMARY OF THE INVENTION

The invention is defined in the claims and relates to compositions and methods for cancer cell detection in bodily samples wherein a cancer cell can be detected within a mixed population of cancer cells and non-cancer cells. The invention also relates to compositions and methods that may be used in cancer cell detection, specifically viruses that are replication-competent conditional to a cancer cell, in particular an oncolytic herpes virus, such as NV1066 and a vaccinia virus, such as GLV-1h68. Provided are methods for using these viruses that preferentially replicate in cancer cells and may also preferentially infect cancer cells for specific identification of such cancer cells, even when a cancer cell is present, for example, at a ratio of one infected cancer cell in a background of ten thousand non-cancer cells, thus further providing a reproducible and sensitive screening method for cancer detection, monitoring and prognosis.

The present inventions also provide methods for early stage cancer detection, cancer monitoring and prognosis, comprising: providing a virus that is replication-competent conditional to a cancer cell, said virus further comprising a reporter gene for expressing a reporter molecule.

The inventions also relate to compositions and methods that can be used in cancer cell detection, specifically compositions comprising an attenuated oncolytic herpes virus, such as NV1066, wherein said virus further comprises a reporter gene and methods for using such viruses for detecting a cancer cell. The inventions also relate to kits and methods for using said kits comprising a virus that is replication-competent conditional to a cancer cell, wherein said virus preferentially infects and/or preferentially replicates in cancer cells, allowing the detection of cancer cells by expression of a reporter gene, to be specifically detected even when present, for example, at a ratio of one cancer cell in a background of ten thousand non-cancer cells. In some embodiments, the inventions provide a virus that allows the detection of one cancer cell in a background of one million non-cancer cells. Thus providing a reproducible and sensitive screening method for early cancer detection and cancer monitoring and prognosis.

The inventions are not limited to any particular compositions and methods for cancer cell detection in the screening of early stage cancer and cancer monitoring and prognosis, wherein a cancer cell may be detected within a mixed population of cancer cells and non-cancer cells. Various compositions and methods are contemplated.

For example, the present invention relates to a method for detection of a cancer cell in a cell sample, comprising, a) providing, i) a virus that is replication-competent conditional to a cancer cell, said virus comprising a reporter gene capable of expressing a reporter molecule, and ii) a cell sample, b) contacting said cell sample *in vitro* with said virus, and c) detecting the expression of the reporter molecule for detecting a cancer cell in a cell sample. The present inventions are not limited by the type of virus used in introducing a reporter gene for replication-competent expression of a reporter molecule. Indeed, a variety of viruses that are replication-competent conditional to a cancer cell are contemplated, including, but not limited to oncolytic viruses that are replication-competent conditional to a cancer cell. In some embodiments, a virus that is replication-competent conditional to a cancer cell is one or more of an infectious virus, a virus infectious to a cancer cell, an oncolytic virus, a herpes virus, a vaccinia virus, and an engineered oncolytic virus that is replication-competent conditional to a cancer cell, such as NV1066. In some exemplary embodiments, the virus is NV1066. In some exemplary embodiments, the virus is GLV-1h68.

The present inventions are not limited by the type of contacting of said cell sample with said virus that is replication-competent conditional to a cancer cell. Indeed a variety of contacting is contemplated, including, but not limited to infection and transfection. The present inventions are not limited by the amount of contacting said cell sample to said virus that is replication-competent conditional to a cancer cell. Indeed a variety of amounts are contemplated, including, but not limited to multiplicity of infection and plaque forming units of virus. In some embodiments, contacting of said virus is at a multiplicity of infection of 0.00001-5.0. In some embodiments, contacting of said virus is at a multiplicity of infection of at least 0.0001-2.0. In some embodiments, contacting of said virus is at a multiplicity of infection of at least 0.0001-1.0. In some embodiments, contacting of said virus is at a multiplicity of infection of at least 0.5-1.0. In some embodiments, contacting of said herpes virus is at a multiplicity of infection of 0.1-5.0. In some embodiments, contacting of said herpes virus is at a multiplicity of infection of 0.1-2.0. In some embodiments, contacting of said herpes virus is at a multiplicity of infection of 0.5-1.0. In some embodiments, contacting of said vaccinia virus is at a multiplicity of infection of 0.00001-5.0. In some embodiments, contacting of said vaccinia virus is at a multiplicity of infection of at least 0.0001-1.0. In some embodiments, contacting of said vaccinia virus is at a multiplicity of infection of at least 0.0001-0.1. The present inventions are not limited by the type of reporter gene. Indeed a variety of reporter genes are contemplated, including, but not limited toGreen Fluorescent Protein (GFP), enhanced Green Fluorescent Protein (eGFP), Blue Fluorescent Protein (BFP), Cyan Fluorescent Protein (CFP), Yellow Fluorescent Protein (YFP), firefly luciferase, renilla luciferase (RUC), β - galactosidase, CAT (chloramphenicol acetyltransferase), alkaline phosphatase (AP), and horseradish peroxidase (HRP). In some preferred embodiments, the reporter gene is selected from the group consisting of an enhanced green fluorescent protein gene and a β-galactosidase gene. In some embodiments, the reporter gene is under the control of a promoter. The present inventions are not limited to the use of any particular reporter gene promoter. In some preferred embodiments, the promoter is operably linked to the reporter gene. The present inventions are not limited by the type of promoter used to drive expression of the reporter gene. Indeed, the use of a variety of promoters active in cancer cells are contemplated, including, but not limited to inducible, constitutive, tissue specific, and cancer cell specific promoters. In some embodiments the promoter includes but is not limited to a constitutive promoter, for example, a cytomegalovirus (CMV) promoter and, a Simian Virus 40 (SV40) promoter. In some embodiments the promoter is a synthetic vaccinia virus early/late promoter. In some embodiments of the inventions the promoter that controls the expression of the reporter gene is a promoter of the replication-competent virus. As such, in some preferred embodiments the promoter of the replication-competent virus is an early expression gene promoter. In some preferred embodiments the promoter of the replication-competent virus is a late expression gene promoter. In some exemplary embodiments, promoters of the replication-competent virus are contemplated, including but not limited to herpes simplex virus-1 (HSV-1) promoters and vaccinia virus promoters. In some preferred embodiments, HSV-1 promoters include but are not limited to a thymidine kinase (TK) β-promoter, a unique short₁₁ (US11) γ-promoter, and an α-promoter of the infected cell protein₄ (ICP4) gene. In some preferred embodiments, a vaccinia virus promoter includes but is not limited to a synthetic vaccinia virus early/late promoter. A variety of cell samples are contemplated, including, but not limited to cell samples derived from patients. The present inventions are not limited to the type of patient. Various types of patients are contemplated. An exemplary patient is a patient not suspected of having cancer. Another exemplary patient is a patient suspected of having cancer. Another exemplary patient is a patient with a diagnosis of cancer whose cancer aggressiveness and progression, or lack thereof, needs to be assessed and monitored. Thus said cell sample is collected from a patient not suspected of having cancer or said cell sample is collected from a patient having cancer. The present inventions are not limited by the type of cancer. Indeed, various types of cancer are contemplated for use with the detection methods of the present inventions including but not limited to lung cancer, bladder cancer, head and/or neck cancer, breast cancer, esophageal cancer, mouth cancer, tongue cancer, gum cancer, skin cancer (*e.g*., melanoma, basal cell carcinoma, Kaposi's sarcoma, etc.), muscle cancer, heart cancer, liver cancer, bronchial cancer, cartilage cancer, bone cancer, stomach cancer, prostate cancer, testicular cancer, ovarian cancer, cervical cancer, endometrial cancer, uterine cancer, pancreatic cancer, colon cancer, colorectal, gastric cancer, kidney cancer, bladder cancer, lymphoma cancer, spleen cancer, thymus cancer, thyroid cancer, brain cancer, neuronal cancer, mesothelioma, gall bladder cancer, ocular cancer (*e.g*., cancer of the cornea, cancer of uvea, cancer of the choroids, cancer of the macula, vitreous humor cancer, *etc*.), joint cancer (such as synovium cancer), glioblastoma, white blood cell cancer (*e.g*., lymphoma, leukemia, etc.), hereditary non-polyposis cancer (HNPC), colitis-associated cancer, etc. Cancers are further exemplified by sarcomas (such as osteosarcoma and Kaposi's sarcoma). The present inventions are not limited by the species of cells in a cell sample. Indeed a variety of species of cells in a cell sample are contemplated, including, but not limited to human, monkey, murine, rat, and the like. In some preferred embodiments, a cell sample may comprise a cancer cell, a normal cell or a mixture of cancer cells and normal cells. In some exemplary embodiments, a cell sample comprises a cancer cell. Further, the present inventions are not limited by the type of cancer cell. Indeed a variety of cancer cell types are contemplated, including but not limited to a gastrointestinal cancer cell, a hepatobiliary cancer cell, a gall bladder cancer cell, a pancreatic cancer cell, a lung cancer cell, a mesothelioma cancer cell, a bladder cancer cell, a prostate cancer cell, a breast cancer cell, a head cancer cell, a neck cancer cell, a thyroid cancer cell, a uterine cancer cell, a cervix cancer cell, a uterine- cervix cancer cell, a blood cancer cell, a white blood cancer cell, a bone marrow cancer cell, pleura cancer cell, and a pleural fluid cancer cell. The present inventions are not limited by the ways of obtaining a cell sample. In some embodiments, a cell sample may derive from a bodily fluid. In some embodiments, a cell sample may derive from a biopsy. Indeed a variety of ways of obtaining a cell sample are contemplated, including but not limited to phlebotomy, aspiration, biopsy, brush biopsy, cystoscopy, endoscopy, lavage, pleural effusion, lumbar puncture, swabbing, and brushing. In some embodiments, said cell sample is obtained from fluids expelled by a patient. In further embodiments, said cell sample is obtained from expelled fluids from spitting, coughing, sneezing, nasal discharging, and dripping or drippage. In some embodiments, a cell sample may derive from saliva, sputum, mucus, amniotic fluid urine, cerebrospinal fluid, blood, plasma, or serum. In some embodiments, a cell sample comprises one or more of a secreted cell, a discharged cell, and a collected cell. In some embodiments, methods of the present inventions further comprise the step of contacting the cell sample with a nuclear stain. The present inventions are not limited by the types of nuclear stains used for contacting a cell sample. Indeed a variety of nuclear stains are contemplated, including but not limited to a Hoechst stain, ethidium bromide, and the like. In some embodiments, methods of the present inventions further comprise the step of contacting the cell sample with a counterstain. Indeed, various types of counterstains are contemplated, including but not limited to a Hoechst stain, a trypan blue stain, an ethidium bromide stain, a 7-amino actinomycin D stain and an antibody stain. In one embodiment, an antibody stain identifies a cancer cell and/or a non-cancer cell. In one embodiment, an antibody stain identifies molecules expressed by a cancer cell. In one embodiment, an antibody stain identifies molecules expressed by a cancer cell at higher levels than a non-cancer cell. The present inventions are not limited by the types of antibody stains for identifying molecules expressed by a cancer cell. Indeed a variety of cancer cell molecules are contemplated, including but not limited to a cytokeratin molecule, a cytokeratin molecule expressed on the cell surface, an integrin CD51/61 molecule, a TAG-72, a p53 molecule and the like. The methods of the present inventions are not limited by the time for detecting a reporter molecule. In some embodiments of the inventions the detecting of the reporter molecule is between one hour and forty-eight hours after contacting the cell sample with the virus. In some embodiments the detecting of the reporter molecule is between twenty-four hours and forty-eight hours after contacting the cell sample with the virus. In some embodiments the detecting of the reporter molecule is between one hour and twenty-four hours after contacting the cell sample with the virus. In some embodiments the detecting of the reporter molecule is between one hour and eighteen hours after contacting the cell sample with the virus. In some embodiments the detecting of the reporter molecule is between one hour and twelve hours after contacting the cell sample with the virus. In some embodiments the detecting of the reporter molecule is between one hour and six hours after contacting the cell sample with the virus. In some embodiments the detecting of the reporter molecule is between one hour and three hours after contacting the cell sample with the virus. In some embodiments the detecting of the reporter molecule is between six hours and eighteen hours after contacting the cell sample with the virus. The present inventions are not limited by the type of detecting method for determining the presence, abundance or absence of a cancer cell in said sample, wherein determining the presence, abundance or absence of a cancer cell is by detecting a reporter molecule, detecting the amount of reporter molecule or not detecting a reporter molecule, respectively. Indeed various types of detecting methods are contemplated, indlucing but not limited to fluorescence assisted cytological testing (FACT). For example, in exemplary embodiments, the detecting method comprises using an instrument selected from the group consisting of a microscope, a luminometer, a fluorescent microscope, a confocal laser-scanning microscope, and a flow cytometer. It is not intended that the sensitivity of the viruses or vectors be limited by the ratio of cancer cells to non-cancer cells in a mixed population. Many sensitivities for detecting a cancer cell in a population of cells are contemplated. For example, in exemplary embodiments, said sensitivity comprises detecting one cancer cell in a background of normal cells in a mixture. In one embodiment, said sensitivity preferably detects 1 cancer cell in a mixture of at least 10 normal cells in a mixture, for example, 1:10. Accordingly in some embodiments, said sensitivity comprises detecting cancer cells and in a population of normal cells in a ratio that is more preferably 1:1000. In one embodiment, said ratio is even more preferably 1:10,000. In one embodiment, said ratio is still more preferably 1:100,000. In one embodiment, said ratio is 1:1,000,000. In further embodiments said ratio ranges from 1:1 to 1:1,000,000. In further embodiments, the mixed cell population contains a cancer cell that is not limited to any one type of cancer cell.

The invention also relates to a kit comprising an isolated virus of the present inventions. Some embodiments of the inventions relate to a kit for cancer detection, further comprising an isolated virus of the present inventions. For example a kit for cancer cell detection, comprises, providing, a virus that is replication-competent conditional to a cancer cell, said virus comprising a reporter gene, wherein said virus has the capability of allowing detection of a cancer cell. While is not intended that the present invention be limited to any specific magnitude of resolution, said virus has the capacity of allowing detection of a cancer cell in a cell sample having a ratio of one cancer cell in a background of ten thousand non-cancer cells, in another embodiment, said virus has the capacity of allowing detection of a cancer cell in a cell sample having a ratio of one cancer cell in a background of one million non-cancer cells. The kits are not limited by the types of replication-competent virus provided for cancer detection. In some embodiments of the inventions said virus is NV1066. In some embodiments of the inventions said virus is GLV-1h68. The replication-competent virus provided for cancer detection are not limited by the types of reporter genes. In some embodiments of the inventions the reporter gene is selected from the group consisting of an enhanced green fluorescent protein gene and a B - galactosidase gene. The kit may further comprises a reagent for performing a detection assay selected from the group consisting of fluorescence assisted cytological testing. The kits are not limited by the types of detection methods. In some embodiments of the inventions said detection comprises using an instrument selected from the group consisting of a microscope, a luminometer, a fluorescent microscope, a confocal laser scanning microscope, and a flow cytometer. The kits may further comprise one or more reagents or components (e.g., antibodies, stains, devices, software, instructions, etc.) useful for, necessary for, or sufficient for, conducting any of the methods described herein.

### BRIEF DESCRIPTION OF THE DRAWINGS

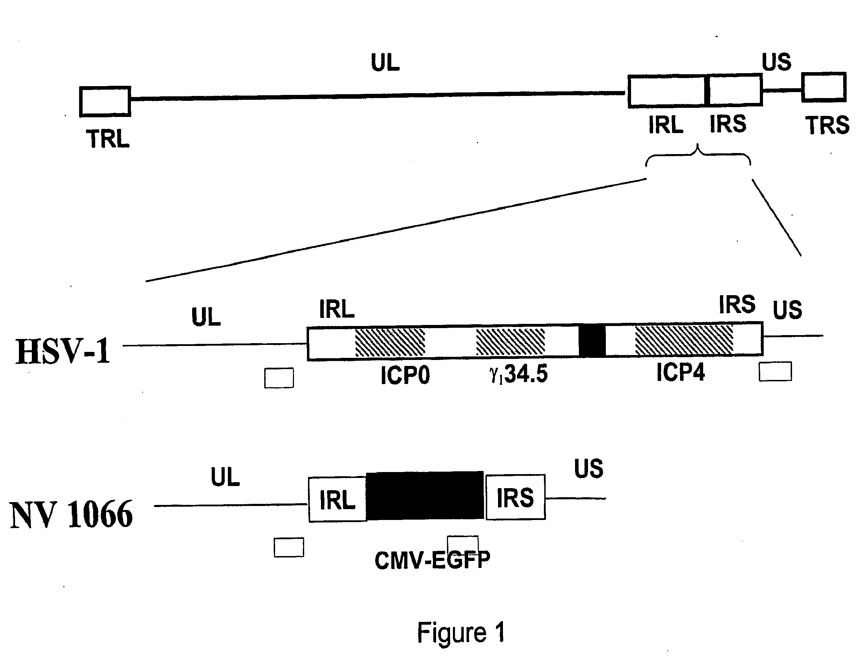

Figure 1 shows exemplary embodiments of a molecular structure of NV 1066. A wild-type HSV-1 (herpes simplex virus type-1) genome consists of the unique long (UL) and unique short (US) sequences, flanked by inverted repeats, terminal and internal repeats long (TRL and IRL) and terminal and internal repeats short (TRS and IRS). NV 1066 comprises deletions from the internal repeat sequences, with loss of one copy each of the ICP-0, ICP-4, and γ₁34.5 genes. A sequence for enhanced green fluorescent protein (eGFP) has been inserted into the viral backbone under the control of a constitutive cytomegalovirus (CMV) promoter.

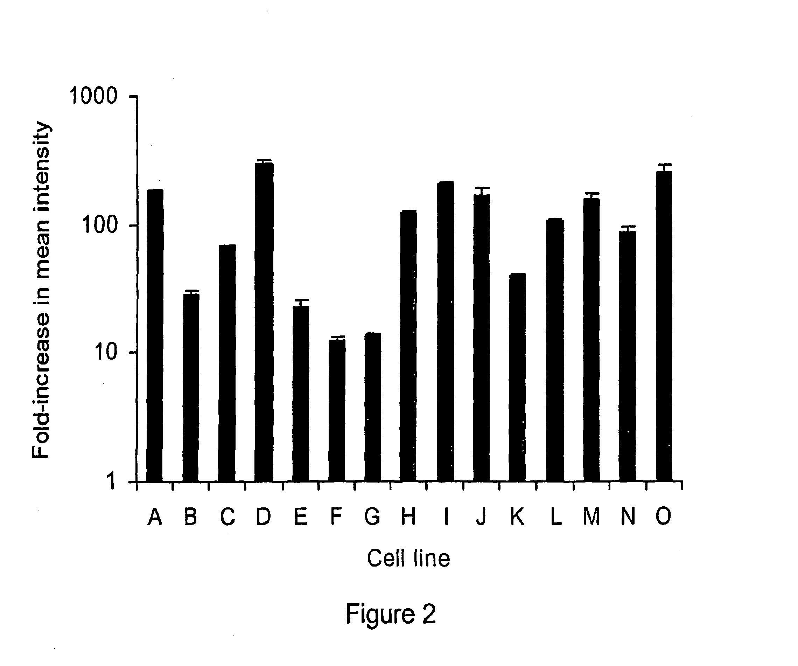

Figure 2 shows exemplary embodiments of a mean intensity of NV1066 infected cancer cells at 11 - 344 -fold higher than background autofluorescence. Fifteen representative human cancer cell lines (A - O, see below) were infected *in vitro* at an MOI of 1 (multiplicity of infection, such as a ratio of viral particles per cancer cell), incubated for 18 hours, and analyzed by flow cytometer. Compared to background autofluorescence, infected cancer cells have a higher mean intensity of green fluorescence (11 - 344 -fold higher, represented in logarithmic scale). Because of this strong expression of eGFP, cancer cells in body fluids can be easily identified even in a background of millions of cells or cell clumps. (A - O cancer cells: lung - A549, H1299; bladder - UMUC-3, KU19-19; stomach - OCUM-2MD3; colorectal - HT29; hepatoma - HepG2; mesothelioma - MSTO-211H (MSTO211H), JMN, H-Meso, H-28; breast MCF-7; head and neck - SCCVII, SCC25, MG 11).

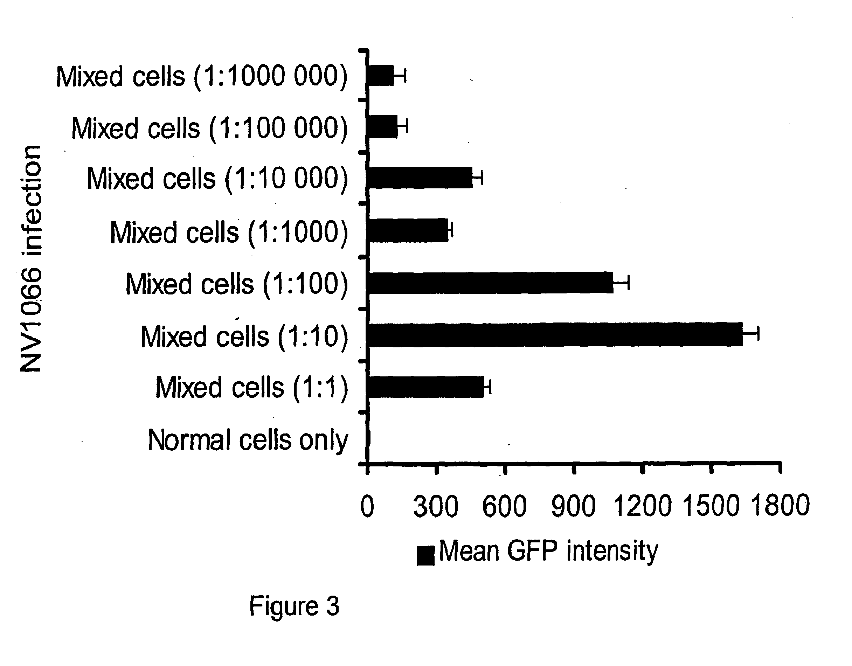

Figure 3 shows exemplary embodiments of NV 1066 infected cancer cells highly expressing eGFP even when mixed with millions of normal cells. Lung cancer cells were mixed with normal cells from bronchoalveolar lavage in ratios from 1:10 to 1:1,000,000 and incubated with NV1066 for 18 hours. Cancer cells mixed with NV1066 served as positive control, and normal cells mixed with NV1066 served as negative controls. The mean intensities of eGFP expressing cells in each sample were plotted. Cancer cells were detected by higher intensity of green fluorescence in up to one in a million without any difficulty. A mean intensity of fluorescence at a dilution of one cancer cell in a million normal cells is fifteen times higher than autofluorescence of cells.

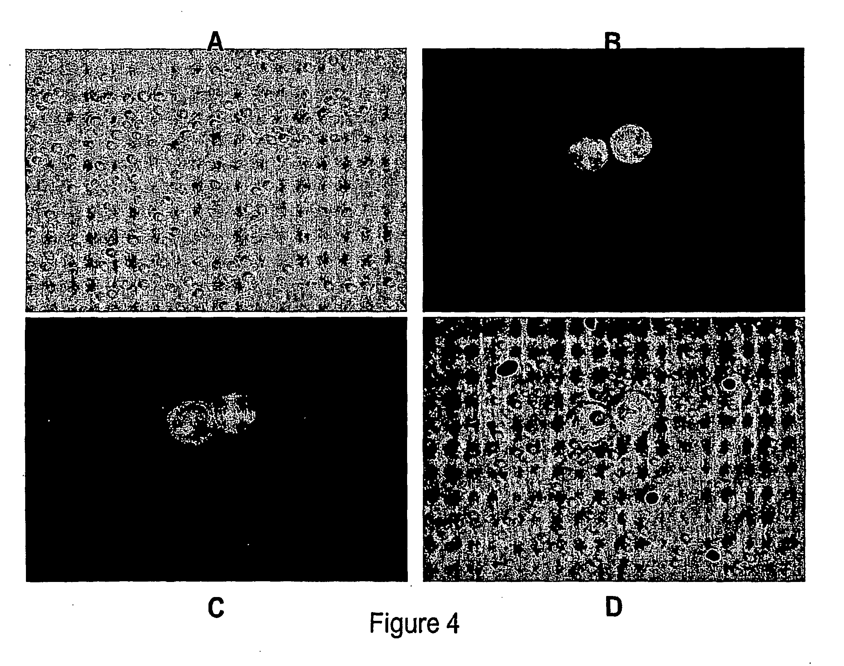

Figure 4 shows exemplary embodiments of NV1066 selectively infecting human mesothelioma cancer cells and not infecting normal cells. Shown is a NV1066 selective infection of cancer cells among a mixture of millions of normal cells confirmed by counterstaining with immunohistochemistry. Human mesothelioma cancer cells were mixed with normal pleural cells (Figure 4A) and were incubated with NV1066 for 18 hours. Examination under fluorescence microscope identified cancer cells by expression of strong green fluorescence (Figure 4B). These cancer cells express integrin (CD 51/61) surface antigen. Incubation with R-Phycoerythrin (R-PE) conjugated mouse anti-human CD51/61 monoclonal antibody confirmed that eGFP expression is selective to cancer cells (identified by red fluorescence, Figure 4C). Overlap of fluorescent pictures with bright-field identifies cancer cells amongst normal cells (Figure 4D). Live cells amongst the cell clumps were identified by nuclear Hoechst staining (blue in color, black in black & white).

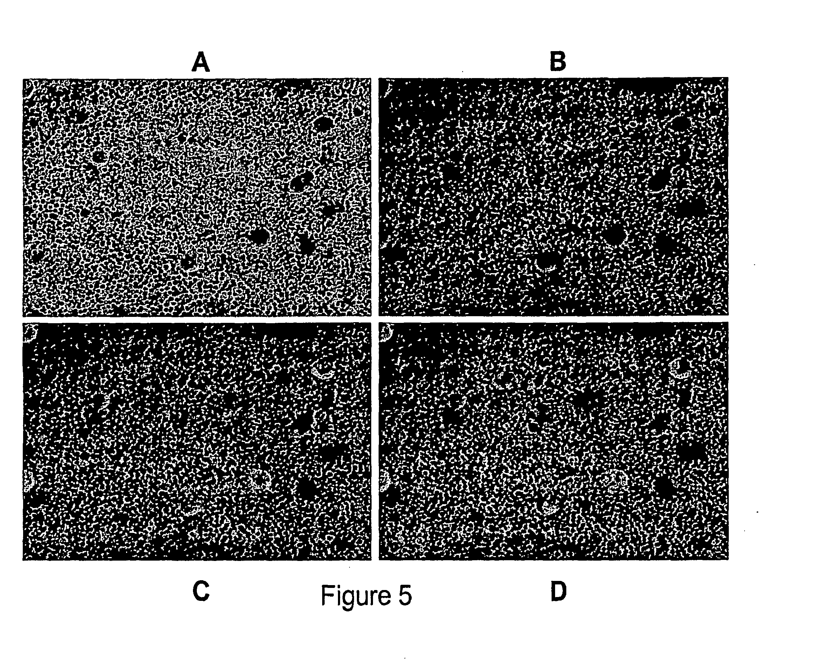

Figure 5 shows exemplary embodiments of NV1066 selectively infecting human lung cancer cells and not infecting normal cells. Shown is a NV1066 selective infection of cancer cells among a mixture of millions of normal cells is confirmed by counterstaining with immunohistochemistry. Human lung cancer cells were mixed with normal bronchoalveolar cells (Figure 5A) and were incubated with NV1066 for 18 hours. These cancer cells express integrin (CD 51/61) surface antigen. Incubation with R-Phycoerythrin (R-PE) conjugated mouse anti-human CD51/61 monoclonal antibody identified cancer cells by red fluorescence (Figure 5B, overlap of bright-field and red fluorescence). Cancer cells were detected by expression of strong green fluorescence (Figure 5C, overlap of bright-field and green fluorescence). Overlap of fluorescent pictures with bright-field identifies cancer cells amongst normal cells (Figure 5D). Live cells amongst the cell clumps were identified by nuclear Hoechst staining (blue in color, black in black & white).

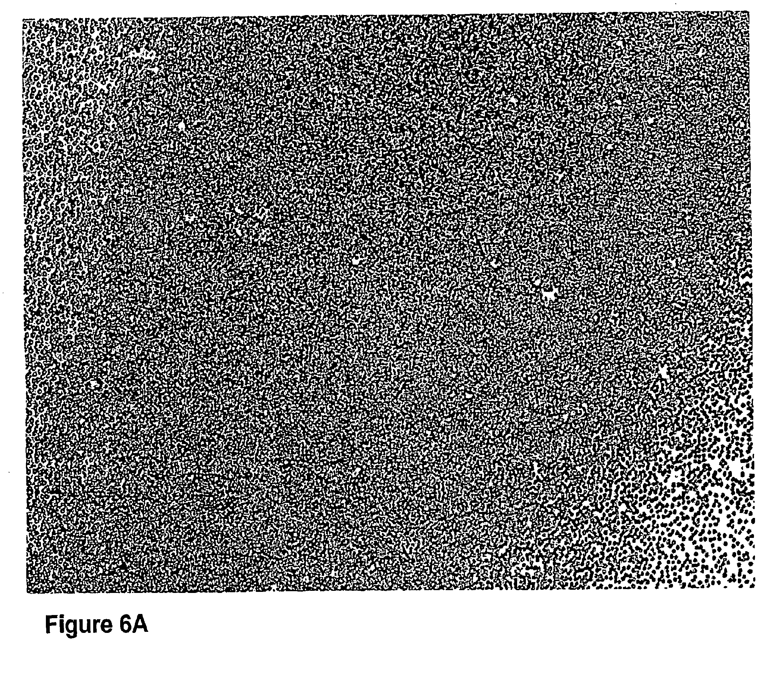

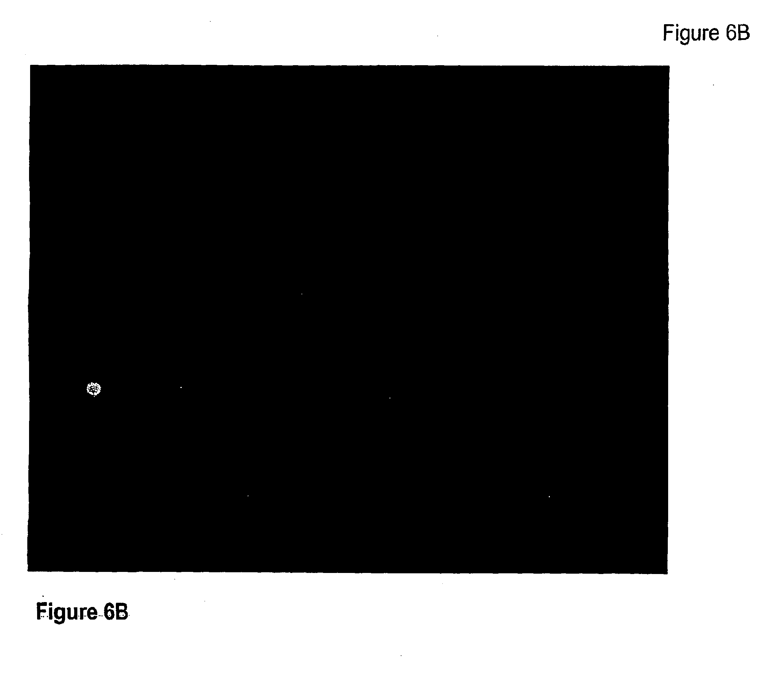

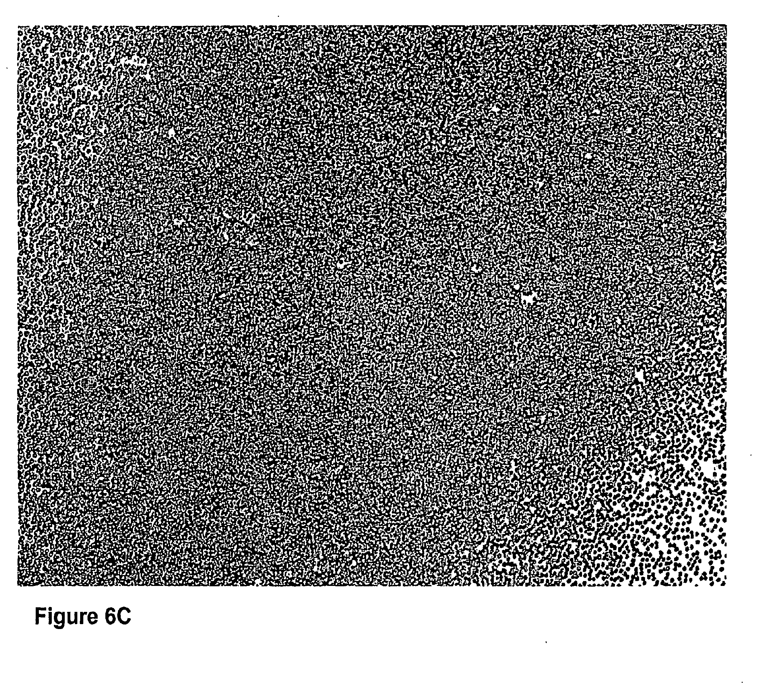

Figure 6 shows exemplary embodiments of eGFP positive lung cancer cells, identified against a background of millions of bronchoalveolar lavage cells. Rare cancer cell in a mixture of millions of normal cells is difficult to identify under bright-field microscopy and is time consuming (Panel 6A). Under fluorescent microscopy, eGFP positive NV 1066 infected cancer cells can be easily identified by means of green fluorescence (Panel 6B). Overlap of a fluorescent image with a bright-field image identifies the cancer cell (Panel 6C) for further studies.

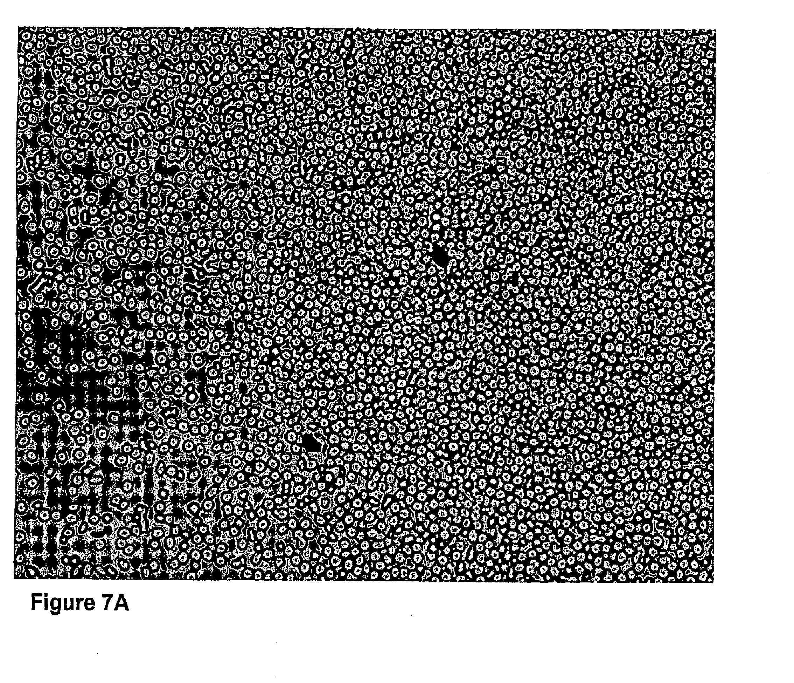

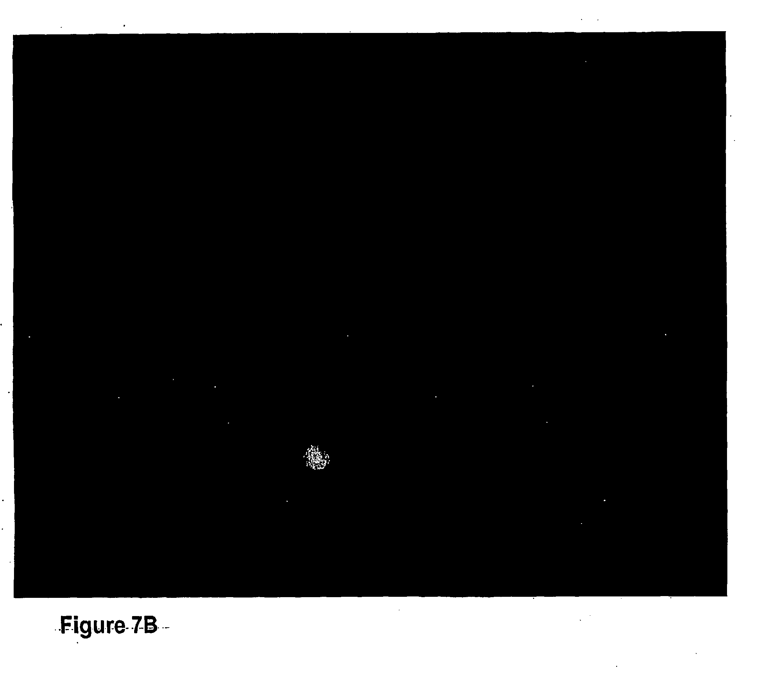

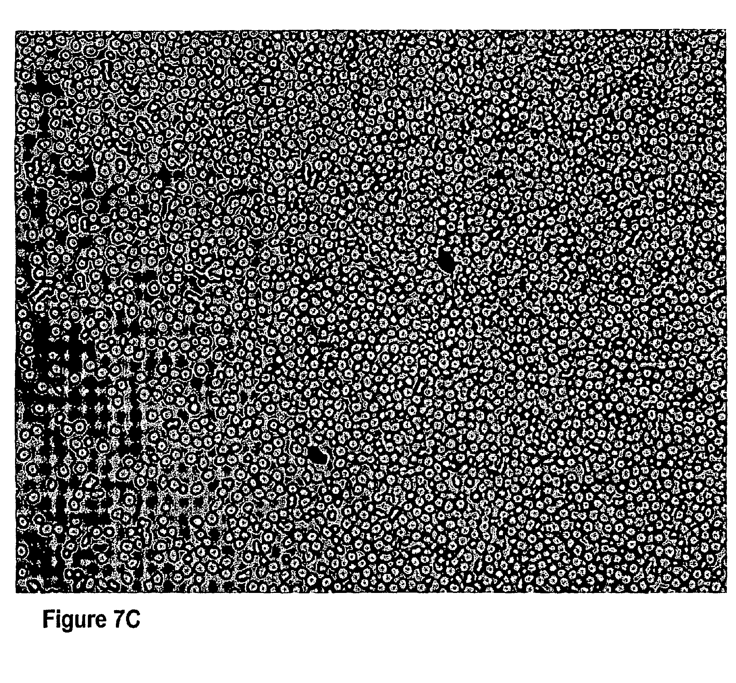

Figure 7 shows exemplary embodiments of eGFP positive bladder cancer cells can be identified against a background of millions of normal bladder cells. A rare cancer cell in a mixture of millions of normal cells is difficult to identify under bright-field microscopy and is time consuming (Figure 7A). Under fluorescent microscopy, eGFP positive NV1066 infected cancer cells can be easily identified by means of green fluorescence expression (Figure 7B). Overlap of a fluorescent image with a bright-field image identifies a cancer cell (Figure 7C) for further studies.

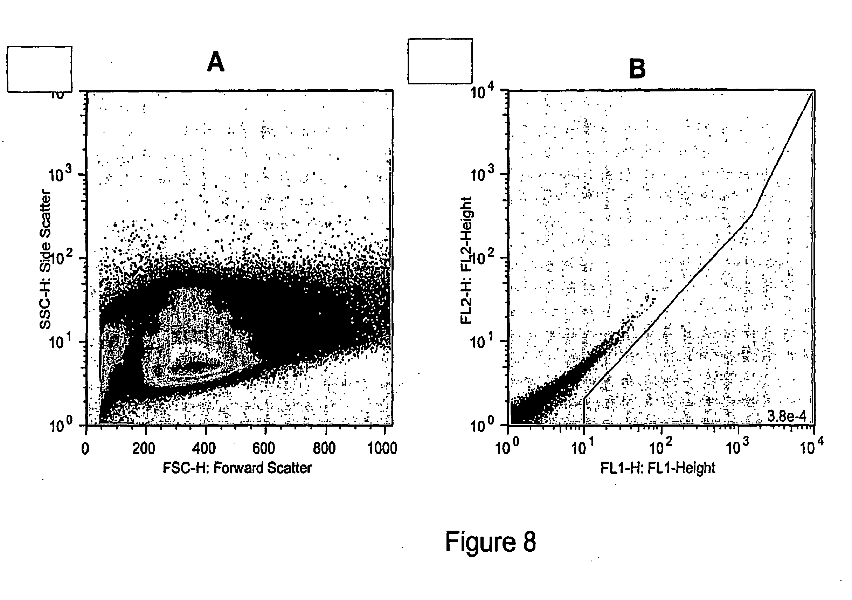

Figure 8 shows exemplary embodiments of a rare cancer cell amongst millions of cells that is detected and separated out for further studies by flow cytometry. Because of the strong emission of green fluorescence by NV1066 infected cancer cells compared to the background autofluorescence of normal cells, a rare cancer cell amongst millions of normal cells can be easily identified by flow cytometry by gating in FL-1 channel. In Panel 8A, two million cells were sorted out by flow cytometry. In Panel 8B, amongst the same cell population, cancer cells were identified by strong green fluorescence in FL-1 channel. These rare cancer cells can be separated out for further histological studies by flow cytometric sorting.

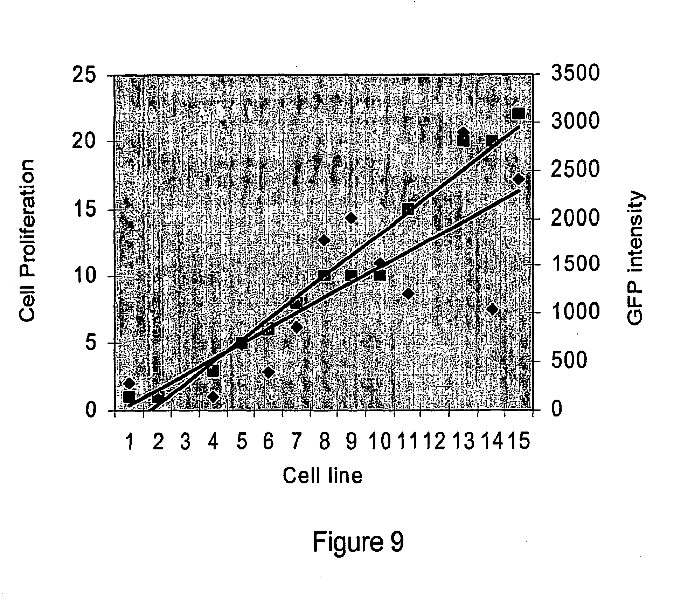

Figure 9 exemplary embodiments of fifteen different cancer cell lines, of lung cancer and mesothelioma, that were infected in parallel with NV1066 (MOI at 0.5 or 1.0). Eighteen hours later, positive - green cells were identified and their mean intensity measured by flow cytometry. Cellular proliferation was measured in each cell line by determining the relative (fold) increase in cell number five days after plating over the initial plating cell number. ■= GFP intensity and ◆ = Cell proliferation.

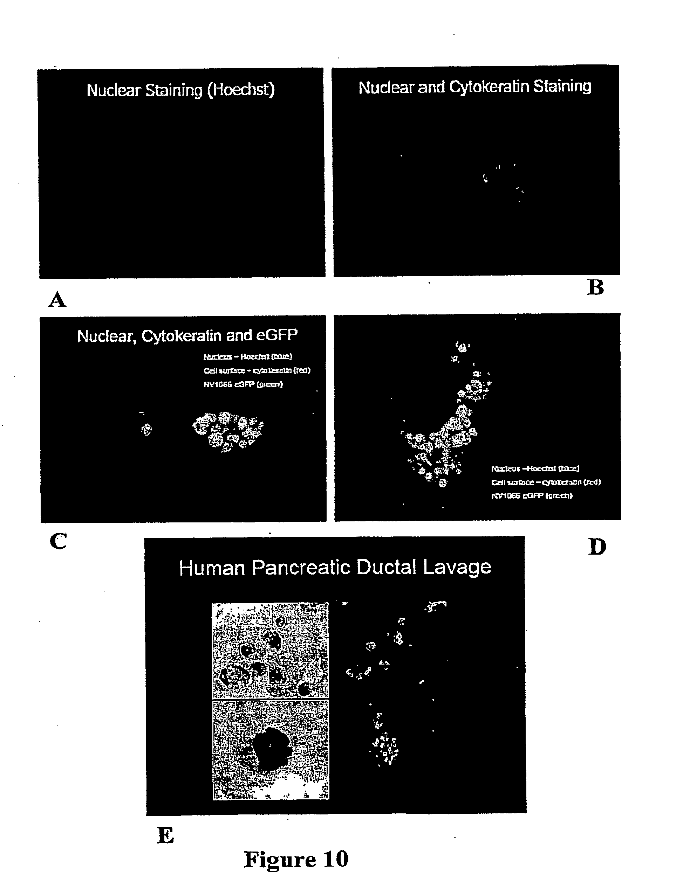

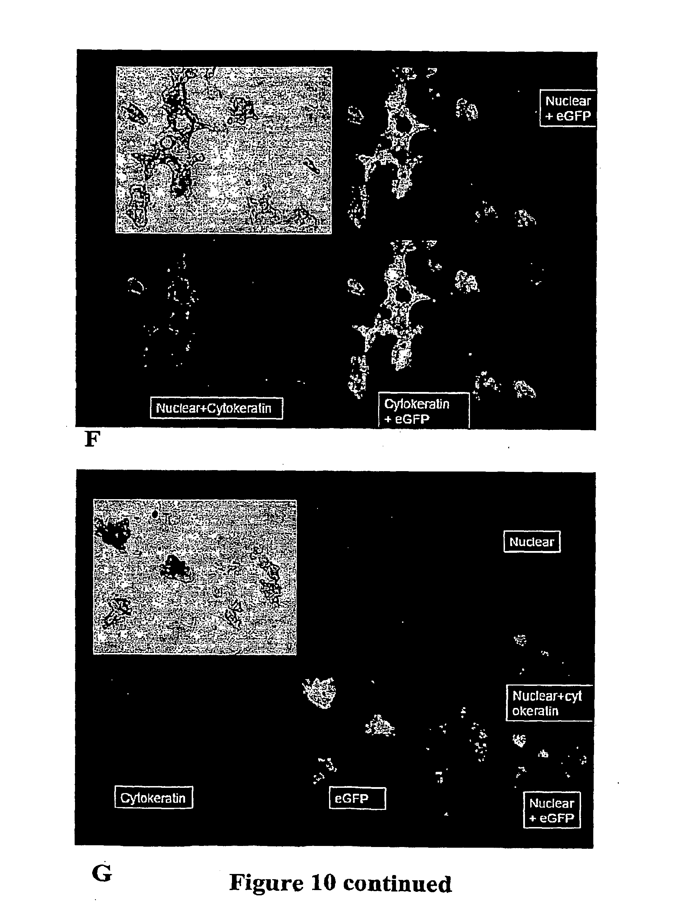

Figure 10 shows exemplary embodiments of NV1066 infected cells from samples obtained from pancreatic cancer resections. Samples were stained with Hoechst (for nucleus - Blue), cytokeratin (for a cancer cell surface marker- Red) and GFP (for a cancer cell, Green). A-D) microscopic pictures showing that human cancer cells expressing cytokeratin are infected by NV1066 producing GFP; E) human pancreatic cancer cells are infected by NV1066 and produce GFP (right panels) compared to staining with "Diff-Quick" (left panels); and F & G) a mixture of cells (cell nuclei stained with Hoechst, blue), cancer cells stained with cytokeratin (red) showing cancer cells are infected by NV1066 and produce GFP (green; green positive; positive) while normal cells (blue positive, red negative) are not infected by NV1066 and do not produce GFP (green negative; negative).

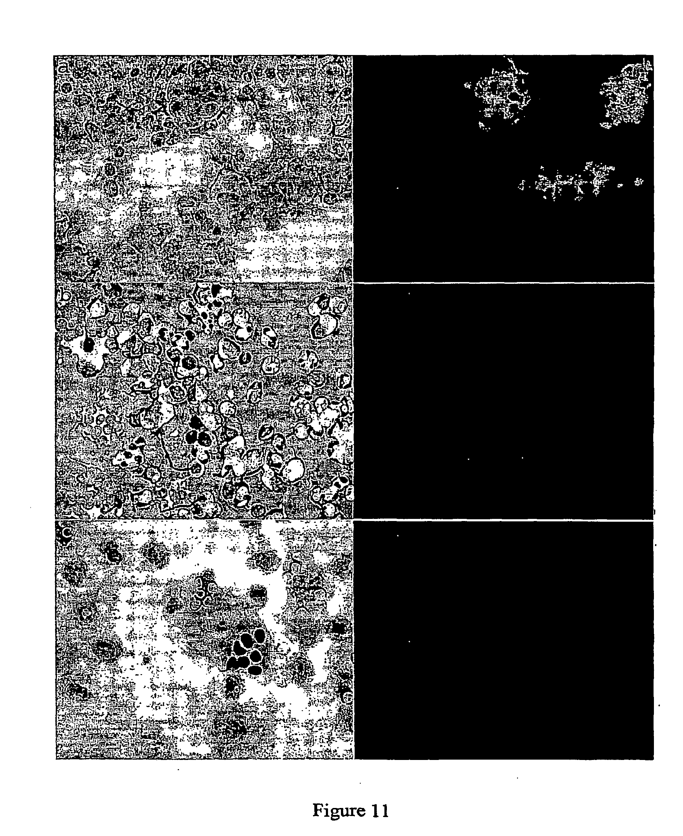

Figure 11 shows exemplary detection of NV1066 transduced green fluorescence that distinguished malignant from benign cells (left panels show light micrographs while right panels show fluorescent micrographs). Panel a: green fluorescent cells viewed with a fluorescent microscope were determined malignant by conventional cytology under light microscopy, panel b: inflammatory cells did not fluoresce green, and panel c: benign epithelial cells did not fluoresce green. (Magnification, 40x).

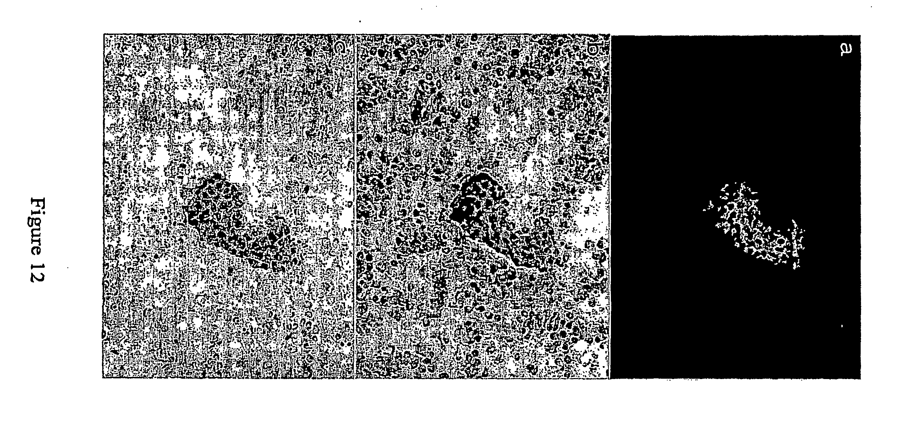

Figure 12 shows an exemplary identification of cancer cells in pancreatic juice from a patient with pancreatic ductal adenocarcinoma and an exemplary comparison of NV1066 cancer cell detection compared to conventional cytological identification as confirmed by immunohistochemistry using a carcinoma cell marker, antibody B72.3. Immunohistochemistry with B72.3 confirms that green fluorescent cells are cancer cells: panel a: pancreatic juice from a patient with pancreatic ductal adenocarcinoma was positive for green fluorescence, panel b: conventional cytology of the same slide yielded an indeterminate diagnosis by three independent, attending pathologists, and panel c: immunohistochemical staining with B72.3 (brown) confirmed that green fluorescent cells were malignant. (Magnification, 40x).

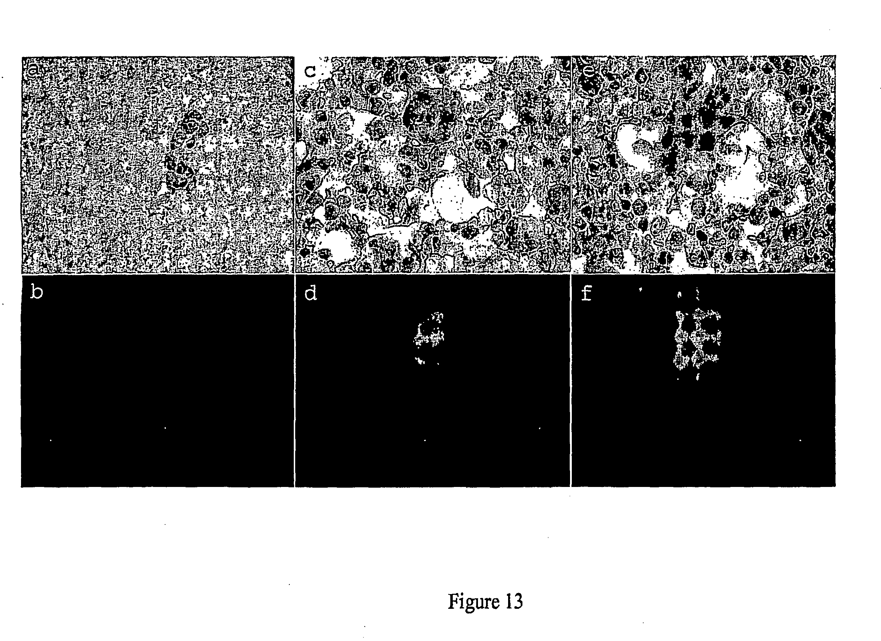

Figure 13 shows exemplary fluorescent micrographs of cancer cells detected in human pleural fluid, (a) cytologic examination showed benign mesothelial cells, as read by an attending pathologist, (b) These benign cells did not fluoresce when viewed under the eGFP filter, (c and e) cytologic examination showed malignant cells in samples from patients with non-small cell lung cancer, (d and f). Malignant cells expressed green fluorescence under the eGFP filter. (Magnification, 40x).

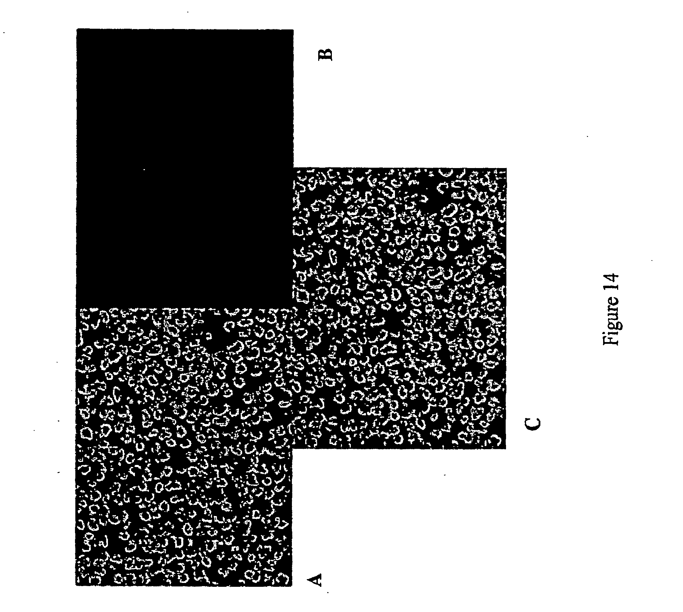

Figure 14 shows exemplary 1x10² (1e2) MST0211H mesothelioma cancer cells mixed with 1x10⁸ (1e8) rat hepatocytes, plated and infected with 1x10⁴ (1e4) pfus of GLV-1h68, 24-48 hours after infection, visualized under fluorescent microscopy.

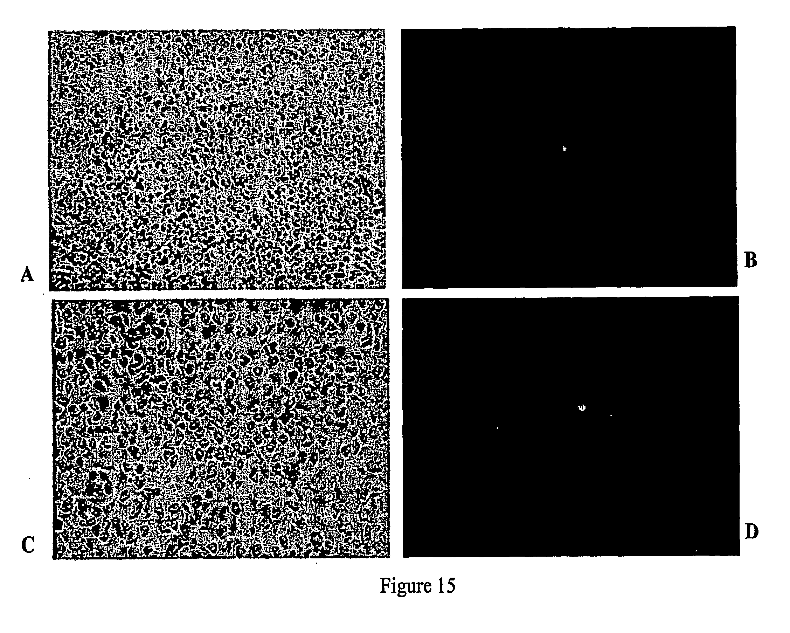

Figure 15 shows exemplary 1x10³ (1e3) MSTO211H mesothelioma cancer cells mixed with 1x10⁶ (1e6) rat hepatocytes, plated and infected with 1x10³ (1e3) pfus of GLV-1h68, 24-48 hours after infection, visualized under fluorescent microscopy.

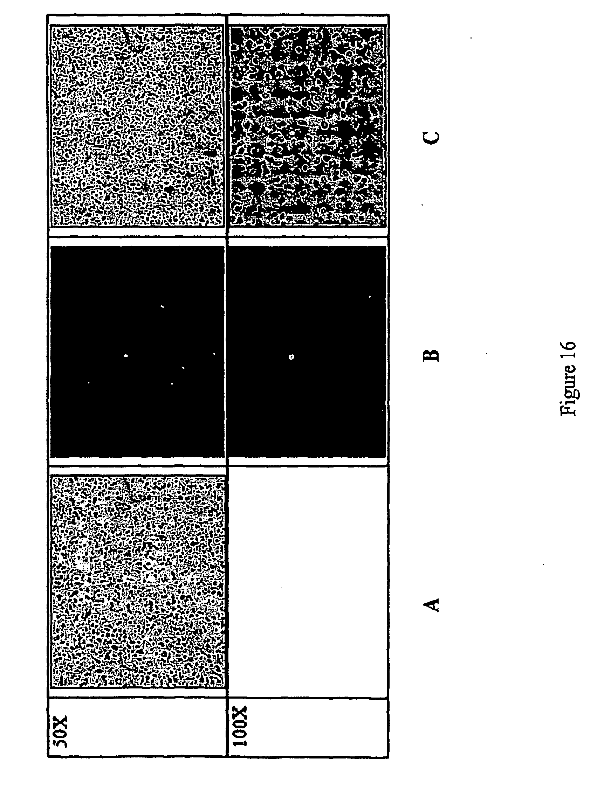

Figure 16 shows exemplary 1x10³ (1e3) MSTO211H mesothelioma cancer cells mixed with 1x10⁶ (1e6) rat hepatocytes, plated and infected with 1x10³ (1e3) pfus of GLV-1h68, 24-48 hours after infection, visualized under fluorescent microscopy.

### DEFINITIONS

To facilitate understanding of the invention, a number of terms are defined below.

The use of the article "a" or "an" is intended to include one or more.

As used herein, the term "replication-competent" refers to the capability of a vector or virus to replicate within a cancer cell. In preferred embodiments, replication-competent refers to the capability of a vector or virus to replicate within a cancer cell but not a non-cancer cell, in other words "replication-competent conditional to a cancer cell." In one embodiment, a vector or virus is "replication-competent conditional to a cancer cell" (*e.g.,* NV1066).

As used herein, the terms "replication-competent" and "replication-competent conditional to a cancer cell" in reference to a herpes virus refer to any herpes based virus, comprising any of a Herpes simplex virus type 1 (HSV-1), cytomegalovirus (CMV), *etc*., in any form such as a virus, virion, plasmid, phage, transposon, cosmid, chromosome, *etc*., which is capable of replication when associated with the proper control elements and which can transfer gene sequences between cells. Thus, the terms include cloning and expression vehicles, as well as viral vectors. *See*, Oncolytic herpes simplex viruses review by Hu JCC and Coffin RS (2003) Internat. Review of Neurobiology 55:165-184; . An example of replication-competent attenuated infectious herpes viral vector of the present inventions is a NV1066, for example, a mutant herpes virus that carries a reporter gene, such as a gene for enhanced Green Fluorescent Protein (eGFP) (Wong et al., 2002, Oncolytic herpesvirus effectively treats murine squamous cell carcinoma and spreads by natural lymphatics to treat sites of lymphatic metastases Hum Gene Ther 13:1213-23; ). In one embodiment, a HSV-1 virus is based on existing HSV-1 strains such as strain F and 17. In one embodiment, HSV-1 is based upon a clinical isolate. Examples of replication-competent herpes viruses that can be used as a vector for a reporter gene are recombinant herpes viruses that are incapable of expressing a functional ICP34.5, for example, NV1066 of the present inventions, and/or a functional thymidine kinase. Further replication-competent herpes viruses are recombinant herpes viruses that are incapable of expressing an active gene product from one copy of each ICP0, ICP4, ORFO, ORFP and ICP34.5 genes. These viruses can be further attenuated by mutation, deletion or inactivation of one or more of the 46 genes found dispensable for viral replication in cell culture (*see*, Table 1 in Roizman B, 1996, Proc Natl Acad Sci 93:11307-12;). Among genes suitable for mutation, deletion or inactivation to decrease virulence are UL16, UL24, UL40, UL41, UL55, UL56, α22, US4, US8 and US11 genes, especially UL24 and UL56. The aforementioned viruses may further include an inactivating mutation in the ICP47 locus of the viruses. Further embodiments include attenuated herpes viruses based on, for example, HSV1716 (MacLean et al., 1991, J Gen Virol 72:631-639;), NV1023 (Wong et al., 2001, Hum Gene Ther 12:253-265;), NV1020 (Delman et al., 2000, Hum Gene Ther 11(18):2465-72;), G207 (Yazaki et al., 1995, Cancer Res 55(21):4752-6;), G47Δ (Todo et al., 2001, Proc Natl Acad Sc. 98(11):6396-6401;), hrR3 (Spear et al., 2000, Cancer Gene Ther 7(7):1051-59;), HF (ATCC VR-260), MacIntyre (ATCC VR-539;), MP (ATCC VR-735;), HSV-2 strains G (ATCC VR-724; ) and MS (ATCC VR-540;), as well as any viruses having mutations (*e.g*., inactivating mutations, deletions, or insertions) in any one or more of the following genes: the immediate early genes ICP0, ICP22, and ICP47 (for example *see*, U.S. Patent No. 5,658,724,); the γ34.5 gene (one or both copies); the ribonucleotide reductase gene; and the VP16 gene (*i.e*., Vmw65, for example, *see,* WO 91/02788, WO 96/04395, and WO 96/04394 ). Further examples of viruses described in U.S. Patent Nos. 6,106,826 and 6,139,834 as well as other replication-competent, attenuated herpes viruses, can also be used as a basis for the construction of the viruses of the present inventions. Another example is a vaccinia virus, such as GLV-1h68.

It is contemplated that, in some embodiments, a vector used in the present invention is labeled with a "reporter molecule," so that the reporter molecule is detectable in any detection system, including, but not limited to eyeball, microscopic, fluorescent, luminescent and radioactive systems. It is not intended that the present invention be limited to any particular detection system or label. Exemplary "reporter" gene sequences (e.g.,. sequences that encodes a molecule such as polypeptide, stain, DNA, RNA, *etc*., that is detectable in enzyme-based histochemical assays, fluorescent, radioactive, and luminescent systems, *etc*.) include green fluorescent protein gene, enhanced green fluorescent protein gene, luciferase gene, *E. coli* β-galactosidase gene, human placental alkaline phosphatase gene, and chloramphenicol acetyltransferase gene.

The terms "reporter gene" and "reporter molecule" refer to an expressible gene and its expressed protein wherein a "reporter gene" is an "expressible gene" and a "reporter expressible gene" that for the purposes of the present inventions refers to a gene capable of being expressed with a cancer cell and further a "reporter molecule" is an "expressed protein" that for the purposes of the present inventions refers to a reporter molecule that may be assayed. Examples of expressible reporter genes include, but are not limited to, green fluorescent protein (GFP) (*e.g*., U.S. Patent Nos. 5,360,728; 5,491,084; GenBank Accession Number U43284;), a number of eGFP molecules (enhanced green fluorescent protein, *see below*) and variants are commercially available from BD Biosciences Clontech Laboratories, (Palo Alto, Calif.) such as found within a pEGFP-Cl vector from BD Biosciences Clontech; (Heim et al., PNAS 91: 12501-4 (1994); Zernicka-Goeta et al., Development 124: 1133-7 (1997)) ), blue, cyano, yellow fluorescent proteins (BFP, CFP, YFP) (Mitra RD et al., Gene 173: 13-7 (1996)), firefly luciferase (*See, e.g.,* de Wet et al., Mol. Cell. BioL 7:725 (1987) and U.S. Patent Nos. 6,074,859; 5,976,796; 5,674,713; and 5,618,682 ), renilla luciferase, such as derived from a sea panzy (*Renilla reniformis*) (for example, Srikantha et al., J Bacteriol. 178:121-9 (1996), β-galactosidase, specifically a *lacZ* gene, in one embodiment detected using luminescent / fluorescent detection systems such as commercially available from Promega Corporation, for example, a Beta-Glo™ Assay System), chloramphenicol acetyltransferase (CAT) (Fordis and Howard, (1987) Methods Enzymol. 151:382-97 ), alkaline phosphatase, for example, human placental alkaline phosphatase (PLAP) anchored protein, Fields-Berry et al. (1992) PNAS, USA, 89:693-697; ), and horseradish peroxidase. Further examples of reporter molecules include genes encoding luminescenct molecules derived from any source of beetle, bacterial, marine bacterial and Cypridina species that naturally produces bioluminescence.

The terms "enhanced green fluorescent protein" or "eGFP" or "EGFP" refer to synthetically modified green fluorescent proteins (GFP). Green fluorescent protein derived from a jellyfish may fluoresce green when exposed to blue light. Enhanced green fluorescent protein refers to numerous mutants and variants, natural and synthetic, of the eGFP gene and encoded proteins that have been produced whose proteins have enhanced fluorescence, for example, a "humanized" mutant of wild-type eGFP for enhanced expression in a mammalian cell as described for eGFP commercially available from BD Biosciences Clontech wherein chromaphore mutations in the EGFP gene sequence correspond to the GFPmut1 variant (Cormack et al. FACS-optimized mutants of the green fluorescent protein (GFP) Gene. 1996;173(1 Spec No):33-8); ) which contains the double-amino-acid substitution of Phe-64 to Leu and Ser-65 to Thr, further comprising silent base pair changes that result in human codon optimization and further provides enhancement of fluorescent intensity GFP is approximately 35X greater when compared to wildtype GFP expressed in mammalian cells (*see,* Clonetechniques, Application Notes, p.22, January 1997) and further U.S. Patent Nos. 5,874,304; 5,968,750 6,020,192; 6,265,548 ) with additional examples of eGFP variants disclosed in U.S. Pat. Nos. 5,625,048; 5,741,668; 5,804,387; 6,020,192; 6,414,119; 6,638,732 ).

The term "FACT" stands for "Fluorescence Assisted Cytological Testing," that for the purposes of the present inventions, refers to the capability of detecting a ratio of one cancer cell in the background of at least one hundred thousand cells, and more preferably in a background of one million normal cells, *see,* EXAMPLE 11. In one embodiment a detecting of a ratio of one cancer cell in the background of one million normal cells is with a sensitivity of > 92%, *see,* Table 8 and EXAMPLE 12. The term "fluorescence assisted" refers to using "fluorescent imaging." Fluorescent imaging includes but is not limited to a luminometer, for example, a Veritas™ Microplate Luminometer (Turner BioSystems), fluorescent microscopy, such as when using a fluorescent microscope that may or may not include bright-field imaging, a confocal laser scanning microscope, a one-photon laser microscope, a two-photon laser microscope, and flow cytometry such as when using a flow cytometer, for example, a FACScan flow cytometer (BD Biosciences).

As used herein, the term "kit" refers to any delivery system for delivering materials. In the context of cancer cell detection, a kit may refer to a combination of materials for detecting one cancer cell in a background of normal cells. In the context of cancer cell detection, such delivery systems include systems that allow for the storage, transport, or delivery of reaction reagents (e.g., virus, detection agents, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials.

As used herein, the term "fragmented kit" refers to delivery systems comprising two or more separate containers that each contains a subportion of the total kit components. The containers may be delivered to the intended recipient together or separately. For example, a first container may contain a virus for use in an assay, while a second container contains control reagents. The term "fragmented kit" is intended to encompass kits containing Analyte Specific Reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

As used herein, the term "container" in reference to a virus refers to any composition that will contain a virus within a confined space. Examples of containers include but are not limited to vials, tubes, flasks, and the like.

The terms "purified," "to purify," "purification," "isolated," "to isolate," "isolation," and grammatical equivalents thereof as used herein, refer to the reduction in the amount of at least one contaminant from a sample. For example, a nucleotide sequence is purified by at least a 10%, preferably by at least 30%, more preferably by at least 50%, yet more preferably by at least 75%, and most preferably by at least 90%, reduction in the amount of undesirable proteins and/or undesirable nucleic acids, such as those present in a nuclear and/or cytoplasmic cell extract. Thus purification of a nucleotide sequence results in an "enrichment," *i.e*., an increase in the amount, of the nucleotide sequence in the sample.

The term "altering" and grammatical equivalents as used herein in reference to the level of any reporter molecule (*e.g*., "enhanced green fluorescent protein, eGFP," "β-galactosidase," *etc*.) and/or counterstain (*e.g*., nuclear Hoechst staining, trypan blue, 7-amino actinomycin D, a R-PE conjugated anti-human CD51/61 monoclonal antibody, a TAG-72, a detected p53 antibody (Ab), for example an antibody for a mutant p53, such as (Ab-3), an antibody for detecting wild-type p53 for detecting altered amounts of conformationally intact p53, such as (Ab-4) and (Ab-5), an antibody for detecting altered amounts of total p53 such as (Ab-1) and (Ab-6), of which these p53 antibodies are supplied by ONCOGENE RESEARCH PRODUCTS, Cambridge, MA ) and/or phenomenon (*e.g*., binding, expression, transcription, enzyme activity, pain, *etc*.) refers to an increase and/or decrease in the quantity of the substance and/or phenomenon, regardless of whether the quantity is determined quantitatively, for example, by flow cytometry, and the like, or qualitatively, for example, when viewed with the eye, when viewed by a microscope, a luminometor, a fluorescent microscope, a confocal laser scanning microscope and the like.

The terms "increase," "elevate," "raise," "greater," "higher" and grammatical equivalents when in reference to the level of a reporter molecule in a cancer cell (*e.g*., "eGFP" *etc.)* and/or phenomenon (*e.g*., infecting, replication, binding, expression, transcription, enzyme activity, pain, *etc.*) in a first sample relative to a second sample, means that the quantity of the substance and/or phenomenon in the first sample is higher than in the second sample by any amount that is statistically significant using any art-accepted statistical method of analysis. In another embodiment, the quantity of the substance and/or phenomenon in the first sample is at least 10 fold greater than the quantity of the same substance and/or phenomenon in a second sample. In another embodiment, the quantity of the substance and/or phenomenon in the first sample is at least 11 fold greater than the quantity of the same substance and/or phenomenon in a second sample. In yet another embodiment, the quantity of the substance and/or phenomenon in the first sample is at least 100 fold greater than the quantity of the same substance and/or phenomenon in a second sample. In a further embodiment, the quantity of the substance and/or phenomenon in the first sample is at least 200 fold greater than the quantity of the same substance and/or phenomenon in a second sample. In yet another embodiment, the quantity of the substance and/or phenomenon in the first sample is at least 344 fold greater than the quantity of the same substance and/or phenomenon in a second sample. (For example, *see,* Fig. 2 and EXAMPLE 3). Further, the increase of a reporter molecule detected in a cancer cell may be relative to the expression of a reporter molecule detected in a non-cancer cell within the same sample. In other words, in one embodiment, the increase of a reporter molecule may be determined qualitatively, for example when a cancer cell is positive and a non-cancer cell is negative refers to a subjective perception of infection, such as color, fluorescence, density, *et cetera. See,* for example, Figures 14-17.

The terms "reduce," "inhibit," "diminish," "suppress," "decrease," and grammatical equivalents when in reference to the level of a substance and/or phenomenon (*e.g*., binding, expression, transcription, enzyme activity, pain, *etc.)* in a first sample relative to a second sample, mean that the quantity of substance and/or phenomenon in the first sample is lower than in the second sample by any amount that is statistically significant using any art-accepted statistical method of analysis. The quantity of substance and/or phenomenon in the first sample is at least 10% lower than the quantity of the same substance and/or phenomenon in a second sample. In another embodiment, the quantity of the substance and/or phenomenon in the first sample is at least 25% lower than the quantity of the same substance and/or phenomenon in a second sample. In yet another embodiment, the quantity of the substance and/or phenomenon in the first sample is at least 50% lower than the quantity of the same substance and/or phenomenon in a second sample. In a further embodiment, the quantity of the substance and/or phenomenon in the first sample is at least 75% lower than the quantity of the same substance and/or phenomenon in a second sample. In yet another embodiment, the quantity of the substance and/or phenomenon in the first sample is at least 90% lower than the quantity of the same substance and/or phenomenon in a second sample. Further, the decrease of a reporter molecule detected in a non-cancer cell may be relative to the expression of a reporter molecule detected in a cancer cell within the same sample. In other words, in one embodiment, the decrease of a reporter molecule may be determined qualitatively, for example when a non-cancer cell is negative and a cancer cell is positive refers to a subjective perception of infection, such as color, fluorescence, density, *et cetera. See,* for example, Figures 14-17.

Reference herein to any specifically named protein or molecule (such as "integrin surface molecule (CD 51/61)," or "TAG-72," or "a p53 molecule," *etc.),* unless specified otherwise, refers to any and all equivalent fragments, fusion proteins, and variants of the specifically named protein having at least one of the biological activities (such as those disclosed herein and/or known in the art) of the specifically named protein, wherein the biological activity is detectably by any method.

The term "fragment" when in reference to a protein refers to a portion of that protein that may range in size from four (4) contiguous amino acid residues to the entire amino acid sequence minus one amino acid residue. Thus, a polypeptide sequence comprising "at least a portion of an amino acid sequence" comprises from four (4) contiguous amino acid residues of the amino acid sequence to the entire amino acid sequence.

The term "variant" of a protein as used herein is defined as an amino acid sequence that differs by insertion, deletion, and/or conservative substitution of one or more amino acids from the protein.

The term "conservative substitution" of an amino acid refers to the replacement of that amino acid with another amino acid that has a similar hydrophobicity, polarity, and/or structure. For example, the following aliphatic amino acids with neutral side chains may be conservatively substituted one for the other: glycine, alanine, valine, leucine, isoleucine, serine, and threonine. Aromatic amino acids with neutral side chains which may be conservatively substituted one for the other include phenylalanine, tyrosine, and tryptophan. Cysteine and methionine are sulphur-containing amino acids which may be conservatively substituted one for the other. Also, asparagine may be conservatively substituted for glutamine, and *vice versa,* since both amino acids are amides of dicarboxylic amino acids. In addition, aspartic acid (aspartate) may be conservatively substituted for glutamic acid (glutamate) as both are acidic, charged (hydrophilic) amino acids. Also, lysine, arginine, and histidine may be conservatively substituted one for the other since each is a basic, charged (hydrophilic) amino acid. Guidance in determining which and how many amino acid residues may be substituted, inserted or deleted without abolishing biological and/or immunological activity may be found using computer programs well known in the art, for example, DNAStar^{™} software. In one embodiment, the sequence of the variant has at least 95% identity with the sequence of the protein in issue. In another embodiment, the sequence of the variant has at least 90% identity with the sequence of the protein in issue. In yet another embodiment, the sequence of the variant has at least 85% identity with the sequence of the protein in issue. In a further embodiment, the sequence of the variant has at least 80% identity with the sequence of the protein in issue. In yet another embodiment, the sequence of the variant has at least 75% identity with the sequence of the protein in issue. In another embodiment, the sequence of the variant has at least 70% identity with the sequence of the protein in issue. In another embodiment, the sequence of the variant has at least 65% identity with the sequence of the protein in issue.

Reference herein to any specifically named nucleotide sequence (such as enhanced green fluorescence protein, *etc.)* includes within its scope any and all equivalent fragments, homologs, and sequences that hybridize under high and/or medium stringent conditions to the specifically named nucleotide sequence, and that have at least one of the biological activities (such as those disclosed herein and/or known in the art) of the specifically named nucleotide sequence, wherein the biological activity is detectably by any method. The "fragment" may range in size from an exemplary 6, 7, 8, and 9 contiguous nucleotide residues to the entire nucleic acid sequence minus one nucleic acid residue. Thus, a nucleic acid sequence comprising "at least a portion of" a nucleotide sequence comprises from six (6) contiguous nucleotide residues of the nucleotide sequence to the entire nucleotide sequence.

The term a "composition comprising a particular nucleotide sequence" as used herein refers broadly to any composition containing the recited nucleotide sequence. The composition may comprise an aqueous solution containing, for example, salts (*e.g*., NaCl), detergents (*e.g*., SDS), and other components (*e.g*., Denhardt's solution, dry milk, salmon sperm DNA, *etc.).*

The term "naturally occurring" as used herein when applied to an object (such as cell, *etc*.) and/or chemical (such as amino acid, amino acid sequence, nucleic acid, nucleic acid sequence, codon, *etc*.) means that the object and/or compound can be found in nature. For example, a naturally occurring polypeptide sequence refers to a polypeptide sequence that is present in an organism (including viruses) that can be isolated from a source in nature, wherein the polypeptide sequence has not been intentionally modified by man in the laboratory.

As used herein the term, *"in vitro"* refers to an artificial environment and to processes or reactions that occur within an artificial environment. *In vitro* environments exemplified, but are not limited to, test tubes and cell cultures.

As used herein the term, *"in vivo"* refers to the natural environment (*e.g*., an animal or a cell) and to processes or reactions that occur within a natural environment.

As used herein, the term "proliferation" refers to an increase in cell number.

As used herein, the term "ligand" refers to a molecule that binds to a second molecule. A particular molecule may be referred to as either, or both, a ligand and second molecule. Examples of second molecules include a receptor of the ligand, and an antibody that binds to the ligand.

The terms "derived from" and "established from" when made in reference to any cell disclosed herein refer to a cell which has been obtained from (*e.g*., isolated, purified, *etc*.) the parent cell in tissue or fluids using any manipulation, such as, without limitation, infection with virus, transfection with DNA sequences, treatment and / or mutagenesis using for example chemicals, radiation, *etc*., selection (such as by serial culture) of any cell that is contained in cultured parent cells. A derived cell can be selected from a mixed population by virtue of response to a growth factor, cytokine, selected progression of cytokine treatments, adhesiveness, lack of adhesiveness, sorting procedure, and the like.

As used herein, the term "biologically active," refers to a molecule (*e.g*. peptide, nucleic acid sequence, carbohydrate molecule, organic or inorganic molecule, and the like) having structured, regulatory, and/or biochemical functions.

The terms "antibody" and "immunoglobulin" are interchangeably used to refer to a glycoprotein or a portion thereof (including single chain antibodies), which is evoked in an animal by an immunogen and which demonstrates specificity to the immunogen, or, more specifically, to one or more epitopes contained in the immunogen. The term "antibody" includes polyclonal antibodies, monoclonal antibodies, naturally occurring antibodies as well as non-naturally occurring antibodies, including, for example, single chain antibodies, chimeric, bifunctional and humanized antibodies, as well as antigen-binding fragments thereof, including, for example, Fab, F(ab')₂, Fab fragments, Fd fragments, and Ev fragments of an antibody, as well as a Fab expression library. It is intended that the term "antibody" encompass any immunoglobulin (*e.g*., IgG, IgM, IgA, IgE, IgD, *etc*.) obtained from any source (*e.g*., humans, rodents, non-human primates, caprines, bovines, equines, ovines, *etc*.). The term "polyclonal antibody" refers to an immunoglobulin produced from more than a single clone of plasma cells; in contrast "monoclonal antibody" refers to an immunoglobulin produced from a single clone of plasma cells. Monoclonal and polyclonal antibodies may or may not be purified. For example, polyclonal antibodies contained in crude antiserum may be used in this unpurified state.

Naturally occurring antibodies may be generated in any species including murine, rat, rabbit, hamster, human, and simian species using methods known in the art. Non-naturally occurring antibodies can be constructed using solid phase peptide synthesis, can be produced recombinantly or can be obtained, for example, by screening combinatorial libraries consisting of variable heavy chains and variable light chains as previously described (Huse et al. (1989) Science 246:1275-1281;). These and other methods of making, for example, chimeric, humanized, CDR-grafted, single chain, and bifunctional antibodies are well known to those skilled in the art (Winter and Harris, (1993) Immunol. Today 14:243-246 ; Ward et al. (1989) Nature 341:544-546,; Hilyard et al. Protein Engineering: A practical approach (IRL Press 1992); and Borrabeck. Antibody Engineering, 2d ed. (Oxford University Press 1995); .

Those skilled in the art know how to make polyclonal and monoclonal antibodies which are specific to a desirable polypeptide. For the production of monoclonal and polyclonal antibodies, various host animals can be immunized by injection with me peptide corresponding to any molecule of interest in the present inventions, including but not limited to rabbits, mice, rats, sheep, goats, chickens, *etc*. In one preferred embodiment, the peptide is conjugated to an immunogenic carrier (*e.g*., diphtheria toxoid, bovine serum albumin (BSA), or keyhole limpet hemocyanin (KLH)). Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and *Corynebacterium parvum.*

For preparation of monoclonal antibodies, directed toward molecules of interest in the present inventions, any technique that provides for the production of antibody molecules by continuous cell lines in culture may be used (*See, e.g*., Harlow and Lane, Antibodies: A Laboratory Manual, Cold Spring Harbor Laboratory Press, Cold Spring Harbor, NY; ). These include but are not limited to the hybridoma technique originally developed by Köhler and Milstein (1975) (Köhler and Milstein, Nature 256:495-497,;), as well as the trioma technique, the human B-cell hybridoma technique (*See e.g.,* Kozbor et al. (1993) Immunol. Today 4:72; ), and the EBV-hybridoma technique to produce human monoclonal antibodies (Cole et al., in Monoclonal Antibodies and Cancer Therapy, Alan R. Liss, Inc., pp. 77-96, 1985). In some particularly preferred embodiments of the present inventions, the present inventions provide monoclonal antibodies of the IgG class.

In additional embodiments of the invention, monoclonal antibodies can be produced in germ-free animals utilizing technology such as that described in WO 90/13678 . In addition, human antibodies may be used and can be obtained by using human hybridomas (Cote et al. (1993) Proc. Natl. Acad. Sci. U.S.A.80:2026-2030) or by transforming human B cells with EBV virus in vitro (Cole et al in Monoclonal Antibodies and Cancer Therapy, Alan R. Liss, pp. 77-96, 1985).

Furthermore, techniques described for the production of single chain antibodies (*See e.g.,* U.S. Patent 4,946,778) can be adapted to produce single chain antibodies that specifically recognize a molecule of interest An additional embodiment of the invention utilizes the techniques described for the construction of Fab expression libraries (Huse et al. (1989) Science 246:1275-1281 ) to allow rapid and easy identification of monoclonal Fab fragments with the desired specificity for a particular protein or epitope of interest

As used herein, the term "primary cell" is a cell that is directly obtained from a tissue (*e.g*. blood) or organ of an animal in the absence of culture. Typically, though not necessarily, a primary cell is capable of undergoing ten or fewer passages *in vitro* before senescence and/or cessation of proliferation. In contrast, a "cultured cell" is a cell that has been maintained and/or propagated *in vitro* for ten or more passages.

As used herein, the term "cultured cells" refer to cells that are capable of a greater number of passages *in vitro* before cessation of proliferation and/or senescence when compared to primary cells from the same source. Cultured cells include "cell lines" and "primary cultured cells."

As used herein, the term "cell culture" refers to any in vitro culture of cells. Included within this term are continuous cell lines (*e.g.*, with an immortal phenotype), primary cell cultures, finite cell lines (*e.g.*, non-transformed cells), and any other cell population maintained in vitro, including oocytes and embryos.

As used herein, the term "cell line," refers to cells that are cultured in vitro, including primary cell lines, finite cell lines, continuous cell lines, and transformed cell lines, but does not require, that the cells be capable of an infinite number of passages in culture. Cell lines may be generated spontaneously or by transformation.

As used herein, the terms "primary cell culture," and "primary culture," refer to cell cultures that have been directly obtained from cells *in vivo*, such as from animal tissue. These cultures may be derived from adults as well as fetal tissue.

As used herein, the terms "monolayer," "monolayer culture," and "monolayer cell culture," refer to a cell that has adhered to a substrate and grow as a layer that is one cell in thickness. Monolayers may be grown in any format, including but not limited to flasks, tubes, coverslips (*e.g.*, shell vials), roller bottles, *et cetera.* Cells may also be grown attached to microcarriers, including but not limited to beads.

As used herein, the terms "suspension" and "suspension culture" refer to cells that survive and proliferate without being attached to a substrate. Suspension cultures are typically produced using hematopoietic cells, transformed cell lines, and cells from malignant tumors.

As used herein, the terms "culture media," and "cell culture media," refer to media that are suitable to support the growth of cells *in vitro* (*i.e.*, cell cultures). It is not intended that the term be limited to any particular culture medium. For example, it is intended that the definition encompass outgrowth as well as maintenance media. Indeed, it is intended that the term encompass any culture medium suitable for the growth of the cell cultures of interest.

The term, "cell biology" or "cellular biology" refers to the study of a live cell, such as anatomy and function of a cell, for example, a cell's physiological properties, structure, organelles, interactions with their environment, their life cycle, division and death.

As used herein, the term "cell" refers to a single cell as well as to a population of (*i.e.*, more than one) cells. The population may be a pure population comprising one cell type, such as a population of normal cells or a population of cancer cells. Alternatively, the population may comprise more than one cell type, for example a mixed cell population. It is not meant to limit the number of cells in a population, for example, a mixed population of cells may comprise at least one cancer cell. In one embodiment a mixed population may comprise at least one non-cancer cell. In the present inventions, there is no limit on the number of cell types that a cell population may comprise.

The term, "cytology" refers to a study of loose cells, such as a cell sample, for example, cells taken from the cervix during a cervicovaginal smear (pap smear).

The term, "cytologic" refers to relating to cytology.

The term, "pancreatic juice cytology" refers to a cytologic examination of cells obtained from pancreatic juice.

The term, "cytologic examination" refers to an analysis of cells under a microscope.

The term, "cytologic smear" refers to a thin tissue or blood sample spread on a glass slide and stained for cytologic examination and diagnosis under a microscope, for example, a pleural smear, a bronchoscopic smear, a lower respiratory tract smear, sputum smear, an alimentary tract smear, also referred to as a "cytologic specimen." The term, "cytosmear" refers to a cells that were directly spread on a glass slide, also referred to as a cytospin slide or cytospun slide.

The term, "cytopathology" refers to a branch of pathology that studies and diagnoses diseases on the cellular level, such as a Pap smear, and the like.

The term, "cystoscopy" refers to a procedure to see the inside of an organ or structure, such as a bladder, urethra etc.

The term, "histology" refers to microscopic anatomy. Histology also refers to a study of tissue sectioned as a thin slice, wherein the tissue was infiltrated with wax or plastic or frozen in cryopreservation medium.

The term, "histopathology" refers to a microscopic study of diseased tissue.

The term, "histopathology" refers to a field of pathology which specializes in the histological study of diseased tissue.

The term, "histochemistry" refers to a science of using chemical reactions between laboratory chemicals and components within tissue.

The term, "Diff-Quick" refers to a stain used by cytopathologists or histologists for cancer cell identification.

As used herein, the term "normal cell" refers to a non-cancer cell.

As used herein, the term "abnormal cell" refers to a cancer cell or a "benign" cell.

The term, "benign" refers to a cell or medical condition or anatomical malformation which, untreated or with symptomatic therapy, will not become life-threatening, for example, benign pancreatic lesions. Benign is used particularly in relation to a cell or a tumor, which is either a benign cell or benign tumor, as opposed to a malignant cell or malignant tumor.

The term, "premalignant condition" refers to a disease, syndrome, or finding that, if left untreated, may lead to cancer. Examples of pre-malignant conditions include actinic keratosis, Barrett's esophagus and cervical dysplasia.

The term, "malignant" is a clinical term that is used to describe a clinical course that progresses rapidly to death, such as a periampullary malignancy. The change of cells from benign to malignant behavior is called "malignant transformation."

The term, "invasive" in reference to a tumor or cancer, such as an "invasive periampullary tumor," refers to a disease or condition that has a tendency to spread, in other words "metastasize" especially a malignant cancer that spreads into healthy tissue. The term, "metastasis" refers to a movement or spreading of cancer cells from one organ or tissue to another or from one area of the body to another, such that a "metastatic cancer" is a cancer that has spread from its primary site into another area.

The term, "noninvasive" in reference to a tumor or cancer, such as a "noninvasive pancreatic carcinoma," not invading adjacent healthy cells, blood vessels, or tissues; localized: a noninvasive tumor or a "nonmetastatic" tumor.

The term, "neoplasia" refers to an abnormal, disorganized growth in a tissue or organ, usually forming a distinct mass, such a growth is referred to as a neoplasm. Neoplasms can be benign or malignant.

The term "cancer" refers to a "malignant neoplasm" or "tumor" that contains at least one cancer cell. The term "cancer" is used herein to refer to a neoplasm, which may or may not be metastatic. Exemplary cancers include but are not limited to tumor cells from various tumor types, including lung, bladder, head and neck, breast, esophageal, mouth cancer, tongue cancer, gum cancer, skin cancer (*e.g.*, melanoma, basal cell carcinoma, Kaposi's sarcoma, *etc.*), muscle cancer, heart cancer, liver cancer, bronchial cancer, cartilage cancer, bone cancer, stomach cancer, prostate cancer, testis cancer, ovarian cancer; cervical, endometrial cancer, uterine cancer, pancreatic cancer, colon cancer, colorectal, gastric cancer, kidney cancer, bladder cancer, lymphoma cancer, spleen cancer, thymus cancer, thyroid cancer, brain cancer, neuron cancer, mesothelioma, gall bladder cancer, ocular cancer (*e.g.*, cancer of the cornea, cancer of uvea, cancer of the choroids, cancer of the macula, vitreous humor cancer, *etc.*), joint cancer (such as synovium cancer), glioblastoma, lymphoma, leukemia, and hereditary non-polyposis cancer (HNPC), colitis-associated cancer. Cancers are further exemplified by sarcomas (such as osteosarcoma and Kaposi's sarcoma).

The term, "pancreatic cancer" refers to a group of cancers arising from pancreatic cells including but not limited to broad types of exocrine pancreatic cancer and endocrine pancreatic cancer. Examples of exocrine pancreatic cancer includes acinar cell carcinoma, adenocarcinoma periampullary malignancy, adenosquamous carcinoma, giant cell tumor, intraductal papillary-mucinous neoplasm (IPMN), mucinous cystadenocarcinoma, pancreatoblastoma, serous cystadenocarcinoma, solid tumors, and pseudopapillary tumors. Examples of endocrine pancreatic cancer include gastrinomas, insulinomas, somatostatinomas, VIPomas, and glucagonomas.

The term, "periampullary tumor" or "periampullary carcinoma" refers to a heterogeneous group of neoplasms arising from the head of the pancreas, the distal common bile duct and the duodenum. Periampullary carcinoma should be distinguished from ampullary carcinoma as a tumor topographically centered in the region of the ampulla of Vater, which is formed by three anatomical components: the ampulla (common channel), the intraduodenal portion of the bile duct and the intraduodenal portion of the pancreatic duct.

The term, "pancreatic adenocarcinoma" or "adenocarcinoma" refers to cancerous cells that involve cells lining the pancreatic duct.

The term, "cholangiocarcinoma" or "bile duct cancer" refers to a malignancy of the bile duct.

The term, "pancreatic juice" refers to a secretion of the pancreas containing enzymes that aid in the digestion of proteins, carbohydrates, and fats, wherein examples of such enzymes include trypsinogen, chymotrypsinogen, pancreatic lipase, and amylase.

As used herein, the terms "cancer cell" and "tumor cell" refer to a cell undergoing early, intermediate or advanced stages of multi-step neoplastic progression as previously described (H.C. Pitot (1978) in "Fundamentals of Oncology," Marcel Dekker (Ed.), New York pp 15-28), including pre-neoplastic cell (*i.e.*, hyperplastic cell and dysplastic cell) and neoplastic cell. Exemplary cancer cells within the scope of the invention include but are not limited to a lung cell, a bronchoalveolar cell, a bronchial cell, an alveolar cell, an esophageal cell, a peritoneal cell, a liver cell, a kidney cell, urinary bladder cell, a stomach cell, a gallbladder cell, a gastrointestinal cell, such as a stomach cell, a colorectal cell, *etc.*, a pancreatic cell, a hepatobiliary cell, such as a hepatoma cell, a mesothelioma cell, a bladder cell, a prostate cell, a breast cell, a head cell, a neck cell, a thyroid cell, a uterine cell, a cervix cell, a uterine-cervix cell, a blood cell, a bone marrow cell, a breast cell, a colon cell, a brain tumor cell, a lymph node cell, a skin cell, an adenocarcinoma cell, a fecal cell, a urinary cell, a bodily fluid cell, sputal cell, a pleural cell, such as a cell from the lining of a lung, and a cell found in a pleural effusion. It is not intended that the present inventions be limited by the nature of the cancer cells used for screening. Both i) cancer cells from established cancer cell lines and ii) cancer cells obtained from patients (*e.g.* from a biopsy) are contemplated. Exemplary cells may be obtained as "samples" by a variety of techniques such as phlebotomy, aspiration, biopsy, brush biopsy, cystoscopy, endoscopy, lavage, pleural effusion, lumbar puncture, swabbing, and brushing, for example, swabbing to provide a Pap smear, or expelled from a patient, such as when a patient is spitting, coughing, sneezing, nasal discharging, and dripping or drippage, further including but not limited to a secreted cell, a discharged cell, a collected cell, and the like.

As used herein, the term "secreted cell" refers to any cell released from an animal in a bodily secretion, such as tears, sweat, pus, mucus, and the like.

As used herein, the term "discharged cell" refers to any cell expelled from an animal such as urine, feces, sputum, uterine material, ejaculate and the like.

As used herein, the term "collected cell" refers to any cell obtained from an animal such as a cytology sample, a cytological specimen, a pleural sample, a biopsy sample, a blood sample, and the like.

As used herein, the terms "sample," "bodily sample," "bodily samples," "cell sample," "bodily fluid cell sample" and "cell sample from a bodily fluid" refer to a "population" and a "cell population" from any material being tested for the presence of cancer cells using methods of the present inventions. In one sense, a bodily sample is meant to include a specimen or culture obtained from any area of an animal such as a secreted cell, a discharged cell, a collected cell, and the like. A sample, including a bodily sample, may also be any population of cells such as those obtained as *in vitro* cell cultures, for example, continuous cell lines (*e.g.*, with an immortal phenotype), primary cell cultures, finite cell lines (*e.g.*, non-transformed cells), and any other cell population maintained *in vitro*, including oocytes and embryos. Such examples are not however to be construed as limiting the sample types applicable to the present inventions.

As used herein, the terms "mixture" and "mixture of at least one cancer cell and at least one non-cancer cell" in a cell population refer to a mixture of two or more types of cells. In some embodiments, the cells are not cancer cells, while in other embodiments the cells are cancer cells. In some embodiments the cells contain infectious engineered viruses or engineered vectors. The present inventions encompasses any combination of cell types suitable for the detection, identification, and/or quantitation of a cancer cell in samples, including mixed cell cultures in which all of the cell types used are cancer cells, mixtures in which one or more of the cell types are cancer cells and the remaining cell types are non-cancer cells, and mixtures in which all of the cell types are non-cancer cells.

As used herein, the term "patient" refers to an animal (*e.g.*, a human, a domestic animal, a livestock animal, an exotic animal, *etc.*).

As used herein, the term "mammal" refers to an organism comprising functional or non-functional mammary glands.

As used herein, the term "patient suspected of having cancer" refers to an animal suspected of having any cancer, including but not limited to, leukemia, gastrointestinal cancer, such as of the esophagus, stomach, colorectal, *etc.*, hepatobiliary, such as hepatocellular carcinoma, cholangiocarcinoma, such as of the gall bladder, *etc.*, cancer of one or more of the following, pancreas, lung, mesothelioma, urinary tract, bladder, prostate, breast, head and neck, thyroid, uterine, cervix, lymph node, bone marrow, brain, nervous system, skin, *et cetera*. Types of cancers include, for example, localized tumors as well as diffuse soft tissue types.

As used herein, the terms "infecting" and "infection" with a microorganism (such as virus and bacterium) refer to co-incubation, *e.g.* for contacting, of a target biological sample, (*e.g.*, cell, tissue, *etc.*) with the microorganism under conditions such that nucleic acid sequences contained within the microorganism are introduced into one or more cells of the target biological sample. Infection may be *in vitro* and/or *in vivo*.

As used herein, the term "infectious" refers to the ability of a microorganism to infect a cell.

The term, "plaque forming unit" or "pfu" or "PFU" refers to a measure of infectious virus particles, such that one plaque forming unit is equivalent to one infectious virus particle.

As used herein, the term "multiplicity of infection" or "MOI" refers to the ratio of infecting vectors or viruses to host cells, virus:host cell, used during transfection or transduction of host cells. For example, if 1,000,000 vectors or viruses are used to transduce 100,000 host cells, the multiplicity of infection is 10. Additionally, an MOI is also calculated using pfu values, such that MOI=PFU/number of cells. The use of this term is not limited to events involving transduction, as it further encompasses introduction of a vector or virus into a host by methods such as lipofection, microinjection, calcium phosphate precipitation, and electroporation.

As used herein, the term "contacting" or "treating" cells with a stain, counterstain, or microbe refers to placing the stain, counterstain, or microbe in a location that will allow it to touch the cell in order to produce "contacted" or "treated" cells. The contacting may be accomplished using any suitable method. For example, in one embodiment, contacting is by adding the stain, counterstain, or microbe to a tube of cells. Contacting may also be accomplished by adding the stain, counterstain, or microbe to a slide chamber of the cells. Contacting may also be accomplished by adding the stain, counterstain, or microbe to cells in a microtiter plate. Contacting may also be accomplished by adding the stain, counterstain, or microbe to a culture of the cells. It is not meant to limit how the stain, counterstain, or microbe contacts the cells. In one embodiment, contacting may be accomplished by administration of stain, counterstain, or microbe to an *animal in vivo.* In one embodiment, contacting may be accomplished by conventional transfection methods, for example, contacting a cell with a virus using one or more of liposomal-mediate transfection, calcium phosphate mediated transfection, electroporation, and the like. For the purposes of the present inventions, wherein by contacting a cell with a virus the virus may enter a normal cell and a cancer cell but whose replication and/or expression of a reporter gene is cancer cell specific, *e.g.* replication-competent conditional to a cancer cell, and thus detecting the reporter gene or molecule, for example, detecting eGFP expression, would be specific for cancer cells.

As used herein, the term "virus" refers in the broadest sense to a virally based nucleic acid construct comprising an expressible reporter gene.

As used herein, the term "infectious virus" refers to a virus comprising a virally based nucleic acid construct, further comprising one or more of a capsid protein and optionally a lipid envelope, adenovirus is such an example of a virus that does not have a lipid envelope, that enables said virus to infect one or more of a cancer cell. In one embodiment, the infectious viruses are attenuated viruses.

As used herein, the term "attenuated virus" refers to a weakened virus that may not produce disease but still stimulate a strong immune response, a response similar to a natural virus. Examples of attenuated viruses include but are not limited to herpes, polio, measles, mumps, adenoviruses, poxviruses, reoviruses, retroviruses and rubella.

In one embodiment the infectious viruses are derived from naturally occurring viruses comprising an expressible reporter gene. Such infectious viruses are contemplated as tumor therapeutics when found to specifically lyse tumor cells due to tumor specific infection and/or replication. Therefore, such viruses are referred to as "oncolytic viruses"..

As used herein, the terms "oncolytic" and "oncolytic viruses" refer to cancer killing; i.e. "onco" meaning cancer and "lytic" meaning "killing."

As used herein, where oncolytic refers to an "oncolytic virus" and an "OV," oncolytic refers to a virus that may kill a cancer cell. Oncolytic viruses are well known in the field A wide range of viruses are contemplated as oncolytic viruses, such as but not limited to herpes viruses, adenovirus, adeno-associated virus, influenza virus, reovirus, vesicular stomatitis virus (VSV), Newcastle virus, vaccinia virus, poliovirus, measles virus, mumps virus, sindbis virus (SIN), sendai virus (SV), see Tables 1 - 7 below, providing an overview of published oncolytic viruses from THE ONCOLYTIC VIRUS WEB PAGE; All Rights Reserved. Copyright © 2004 E.A. Chiooca.

As used herein, the term "herpes virus" refers to any of the animal viruses that cause painful blisters on the skin of an animal. Examples of herpes viruses include but are not limited to Herpes simplex virus type 1 (HSV-1), i.e. a herpes virus that causes old sores and fever, herpes simplex, *i.e.* a herpes virus that affects the skin and nervous system, herpes zoster, *i.e.* a herpes virus that causes shingles, Epstein Barr virus (EBV), *i.e.* a herpes virus that causes infectious mononucleosis; associated with specific cancers in Africa and China, cytomegalovirus (CMV)" refers to any of a group of herpes viruses that infect and enlarge epithelial cells and can cause birth defects, further, such viruses also cause a disease of infants characterized by circulatory dysfunction and microcephaly, and can affect humans with impaired immunological systems, varicella zoster virus, *i.e.* a member of the herpes virus family that is responsible for chickenpox.

As used herein, the term "vaccinia virus" or "VV" refers to any DNA virus in a poxvirus family of viruses, for examples, a natural VV, an engineered VV, such as an engineered GLV-1h68.

As used herein, the term "ICP" refers to "infected cell protein," for example, ICP 34.5, wherein said ICP 34.5 protein is encoded by a_{γ1}34.5 gene.

As used herein, the term "ORF" refers to "open reading frame" that for the present inventions refers genes within a 8.5 kb region of DNA transcribed during latent viral infections comprising 16 ORFs encoding at least 50 codons, which have been designated ORF A through ORF P (*see*, Lagunoff and Roizman, (1994) J Virol. Sep;68(9):6021-8; ).

As used herein, the term "vector" refers to any genetic element, such as a virus, virion, plasmid, phage, transposon, cosmid, chromosome, *etc.*, which is capable of replication when associated with the proper control elements and which can transfer gene sequences between cells.

As used herein, the term "promoter" is defined as an array of nucleic acid control sequences that direct transcription of a nucleic acid, such as a reporter gene.

As used herein, a promoter includes necessary nucleic acid sequences near the start site of transcription, such as, in the case of a polymerase II type promoter, a TATA element. A promoter also optionally includes distal enhancer or repressor elements which can be located as much as several thousand base pairs from the start site of transcription.

A "constitutive" promoter refers to a promoter which is active under most environmental and developmental conditions.

As used herein, the term "constitutive promoter" in reference to a replication-competent virus and a virus that is replication-competent conditional to a cancer cell vector construct refers to a promoter that allows for continual transcription of its associated gene in a cancer cell, for example an SV40 promoter, a CMV promoter and the like. When a promoter is in reference a promoter active for a virus, that promoter is referred to a viral promoter, for example, HIV-1 viral promoters for driving the transcription of HIV-1 genes.

An "inducible" promoter is a promoter which is under environmental or developmental regulation. The term "operably linked" refers to a functional linkage between a nucleic acid expression control sequence (such as a promoter, or array of transcription factor binding sites) and a second nucleic acid sequence, wherein the expression control sequence directs transcription of the nucleic acid corresponding to the second sequence, that for the purposes of the present inventions a promoter is operably linked to a reporter gene. A promoter of the present inventions includes promoters active in any cancer cell and includes promoters expressing a reporter gene in a specific cancer cell, for example, an SV40 promoter may express a reporter gene in any cancer cell whereas an SV40 construct, such as a SV40 / Tyrosinase, Invitrogen, may specifically express a reporter gene in a melanoma cancer cell.

It is contemplated that when "counterstaining," at least one cell of the present inventions will be counterstained by any "counterstain," so that either at least one cancer cell or at least one non-cancer cell or both types of cells are stained using counterstains selected from the group consisting of a Hoechst stain, a trypan blue stain, an ethidium bromide stain, a 7-amino actinomycin D stain and an antibody stain. In one embodiment, a counterstain identifies a cancer cell and a non-cancer cell. In one embodiment, a counterstain identifies a cancer cell but not a normal cell. In one embodiment, a counterstain identifies a live cell and a dead cell. In one embodiment, a counterstain identifies a live cell but not a dead cell. In one embodiment, a counterstain identifies a dead cell but not a live cell. In one embodiment, no stain is used and cells in a mixture are detected with bright-field microscopy.

As used herein, when referring to a "nuclear stain," at least the nucleus of a cell is stained by any one of a stain and a counterstain for example, a Hoechst stain, ethidium bromide, acridine orange (AO) and the like.

As used herein, the term "positive cell" in relation to eGFP refers to a cancer cell, wherein a cancer cell expresses a fluorescent green molecule that is detectable quantitatively and/or qualitatively above an autofluorescent backgound. A positive cell may also refer to a cell that stains for a molecule such as CD16, *et cetera*.

As used herein, the term "negative cell," refers to a cell absent detectable signal, such as following contacting with a virus or vector, *e.g.* eGFP expression, or following counterstaining, such as CD16 detection, *et cetera*.

As used herein, the term "detecting a positive cell" refers to the detecting of the expression of the reporter gene by a cell (*e.g.*, detecting a molecule encoded by a reporter gene, a protein, an mRNA, the activity of a protein encoded by the reporter gene) that for the purposes of the present inventions includes "detecting a cancer cell" detecting cancer" and "cancer detection." In preferred embodiments, the detecting involves the diagnostic methods of the present inventions, also referred to as "sensitivity" or "sensitivities" of a virus for detecting a cancer cell or a sensitivity of a diagnostic method or methods for detecting a cancer cell. In one embodiment, said detecting comprises detecting one cancer cell in a background of normal cells wherein said detecting of one cancer cell is at least 15 fold greater than detecting background detecting of either uninfected cells or infected normal cells. In one embodiment, said detecting comprises detecting within a mixture of cells a ratio of cancer cells to normal cells wherein said ratio is preferably 1 cancer cell in a mixture of 10 normal cells (1:10). Accordingly in some embodiments, said detecting comprises detecting wherein the ratio of cancer cells to normal cells is more preferably 1:1000. In one embodiment, said detecting comprises detecting a ratio of cancer cells to normal cells wherein said ratio is even more preferably 1:10,000. In one embodiment, said detecting comprises detecting a ratio of cancer cells to normal cells wherein said ratio is still more preferably 1:100,000. In one embodiment, said detecting comprises detecting a ratio of cancer cells to normal cells wherein said ratio is 1:1,000,000. While not limiting the invention to any method for detecting a positive cell, in one embodiment, the detecting comprises using methods known in the art, including, but not limited to, detection instruments such as a bright-field microscope, a luminometer, a fluorescent microscope, a confocal microscope (*e.g.*, such as a scanning confocal microscope, a fluorescence correlation spectroscopy (FCS) systems), flow cytometers, microfluidic devices, Fluorometric Imaging Plate Reader (FLIPR) systems (See, *e.g.*, Schroeder and Neagle, J. Biomol. Screening 1:75-80 [1996]; herein incorporated by reference), and plate-reading systems. In some preferred embodiments, the response (*e.g.*, increase in fluorescent intensity) caused by the expression of a reporter molecule from at least one infected cancer cell in a mixture of cells that is compared to the response generated by a known number of cancer cells in a mixture of a known number of cells. The minimum response caused by 100% non-cancer cells is defined as a 0% response (for example, 0% mean eGFP intensity). Likewise, the maximal response recorded after addition of a cancer cell to a sample containing a known number of non-cancer cells is detectably higher than the 0% response (for example, greater than 0% mean eGFP intensity). In one embodiment, mean eGFP intensity is 150, in another embodiment, mean eGFP intensity is 300, in yet another embodiment mean eGFP intensity is 600, in yet another embodiment, mean eGFP intensity is 1,000, in yet a further embodiment, mean eGFP intensity is 1700. For example, *see*, Fig. 3. In one embodiment the detecting comprises using a plurality of reaction compartments. Preferably, each of the reaction compartments comprises one mixed cell sample. More preferably, the mixed cell sample in each of the reaction compartments is different from the mixed cell sample in other reaction compartments. In one embodiment, the plurality of reaction compartments comprises a micro-well titer plate. Alternatively, the plurality of reaction compartments comprises at least 48 or at least 96 of the reaction compartments.

The term "level of expression" refers to the quantity of protein and /or RNA that is produced following transcription of a DNA sequence that encodes the protein and /or RNA. A protein may be a transfected protein, such as eGFP and β-galatosidase, and an endogenous protein, such as CD51, TAG-72, and p53. Methods for determining the level of expression of proteins are known in the art such as using fluorescence, as described herein, (*e.g.*, enhanced green fluorescent protein encoded by the *eGFP* gene), assays wherein the mixture of cell are contacted with a conjugated antibody that is specific for an expressed protein, and such as using immunofluorescence wherein said antibody is a fluorescent conjugate, such as CD51 as described herein, or a non-fluorescent conjugate, such as for detecting TAG-72 or p53, as in assays wherein the cells are incubated with a first antibody that is specific for the expressed protein and fluorescently labeled second antibody that is specific for the immunoglobulin of the first antibody followed by observation of immunofluorescence under the microscope.

The term, "surgical resection" in reference to a specific organ or structure, refers to a surgical removal of part of an organ or structure, for example, "pancreatic resection" refers to a surgical removal of part of a pancreas.

The term, "radiographically occult" in reference to a cancer, refers to a cancer, a tumor or a cancer cell that is hidden from view.

### GENERAL DESCRIPTION OF THE INVENTION

The invention is defined in the claims and relates to compositions and methods for cancer cell detection in bodily samples wherein a cancer cell can be detected within a mixed population of cancer cells and non-cancer cells. The invention also relates to compositions and methods that may be used in cancer cell detection, specifically viruses that are replication-competent conditional to a cancer cell, in particular an oncolytic herpes virus, such as NV1066 and a vaccinia virus, such as GLV-1h68. Provided are methods and kits for using these viruses that preferentially replicate in cancer cells and may also preferentially infect cancer cells for specific identification of such cancer cells, even when a cancer cell is present, for example, at a ratio of one infected cancer cell in a background of ten thousand non-cancer cells, thus further providing a reproducible and sensitive screening method for cancer detection, monitoring and prognosis.

When cancers develop, they are often not found until they have metastasized and are no longer curable. Thus active ongoing investigations seek to develop methods for finding these cancers at an earlier stage in time to provide a cure (Smith et al., 2004, CA Cancer J Clin 54(1):41-52). One screening technique that is already in use is the collection and examination of bodily fluids, such as sputum, urine, or tissue samples, such as cervical cytological specimens from high-risk patients to look for cancer cells. These tests are attractive because they are easily performed and pose little risk to the patients. However, examination of these specimens by current cytological techniques is labor intensive and is low in sensitivity. Furthermore, conventional cytology relies on the morphological identification of malignant cells by a skilled pathologist and is subject to interpretive error and interpathologist variability (Khalid et al., (2005) Clin Lab Med;25(1):101-16; Harewood et al., (2004) Am J Gastroenterol;99(8):1464-9 ).

### I. Current Cancer Cell Detection Methods, Sensitivity, and Limitations.

The following examples of cancer cell detection methods are provided as examples and intended to limit either the example or the types of cancer cells detected using compositions and methods of the present inventions.

### A. Lunn Cancer Cell Detection.

Sputum analysis is widely used to screen high-risk populations for lung cancer. The United States National Cancer Institute (NCI) early lung cancer study gave sputum cytology sensitivity a score of 23% and 10% for prevalence and incidence reporting in cancerous patients, respectively (Flehinger et al., 1984, Am Rev Respir Dis 130(4):555-560 ). The accuracy of conventional sputum cytology is even worse for very early lung cancers, as well as for peripheral lung cancers, particularly adenocarcinoma, which is becoming the predominant cancer type in women and non-smokers (Charloux et al., 1997, Lung Cancer 16(2-3):133-143; Charloux et al., 1997, Int J Epidemiol 26(1):14-23).

### B. Bladder Cancer Cell Detection.

The overall sensitivity of positive urine cytology in diagnosis of bladder cancer is slightly higher, at approximately 40 to 60%, due to the general poor nature of urine specimens that typically contain significant amounts of skin and vaginal contamination.

Many investigators have attempted to enhance the sensitivity of cancer cell detection in cells obtained from bodily fluids, including sputum and urine, by several methods, such as DNA ploidy, and evaluating numerous types of DNA markers. The following is a brief review of current detection methods and their limitations. Detection of lung or bladder cancer by measuring DNA ploidy (the total amount of DNA in a given cell measured by flow cytometry) has limitations inherent to the technique. These tests are difficult to perform and standardize, and even a few lysed white blood cells can confound the DNA measurements (Bunn, 2002, Lung Cancer 38(1):S5-S8). Because of these limitations, measuring DNA ploidy from urine has a sensitivity of 45% and specificity of 87% in diagnosing bladder cancer (Pattari et al., 2002, Diagn Cytopathol 27(3):139-142). Studies of other DNA markers such as measuring gene expression levels using quantitative PCR techniques, hypermethylation of CpG-islands in promoter regions of various tumor suppressor genes (Tsou et al., 2002, Oncogene 21(35):5450-5461), microsatellite alterations using several markers, and mutations in specific oncogenes, are limited by the heterogenicity of expression and false-positive test results due to low-level transcription of the marker genes in normal cells. Many of these markers are also elevated in patients with inflammatory diseases.

Detection of messenger ribonucleic acid (mRNA) coding for a specific marker protein by reverse transcription-polymerase chain reaction (RT-PCR), advocated as the most sensitive approach, has not translated into clinical practice due to the high dependence of the result on the purity of RNA preparations, and the false-positive rate occurring from low levels of illegitimate transcription. Tumor markers such as Lewis X antigen, demonstrated immunohistochemically in 85% to 89% of transitional cell bladder cancers, is not a sensitive early detection biomarker as 51% of reactive urothelial cells also express Lewis X antigen (Brown et al., 2002, Urol Clin North Am 27(1):25-37 ). Bladder tumor antigen (BTA) sensitivity in a multicenter trial is up to 40%, with 10% of the patients having false positive results. Similarly, nuclear matrix protein, fibrin degradation products, telomerase, and hyaluronic acid/hyaluronidase in urine did not translate into clinical practice because of lack of sensitivity (Brown et al., 2002, Urol Clin North Am 27(1):25-37). Detection of early bladder cancer by RT-PGR for cytokeratin is limited by skin and vaginal contamination. Conventional fluorescence in situ hybridization (FISH) methods require fixation, which reduces cell recovery as much as 50% and makes detection of small subpopulations of cancer cells difficult (Gozzetti et al., 2000, Semin Hematol 37(4):320-333 ). Current methods of flow cytometric detection of malignant cells, developed to avoid above constraints were limited by the lack of uniform biomarker expression.

### C. Cervical Cancer Cell Detection.

In case of cervical smears, the Agency for Health Care Policy Research (AHCPR) concluded that the sensitivity of a single smear of conventional cervical cytology is 51% (Spitzer et al., 2002, Obstet Gynecol Clan North Am 29(4):673-683 ). Three consecutive yearly Pap smears are required to increase the sensitivity to 88.2% (Spitzer et al., 2002, Obstet Gynecol Clin North Am 29(4):673-683 ).

### D. Pancreatic Cancer Cell Detection.

Pancreatic cancer, the most common periampullary malignancy, is a fatal disease with most patients dying within two years of diagnosis. Surgical resection provides the greatest chance for cure, but most patients present with locally advanced or metastatic disease at the time of diagnosis, precluding surgery. Early detection of resectable tumors is necessary to improve patients' outcome. However, despite advances in imaging and endoscopy, periampullary neoplasms are among the most challenging tumors to detect early (Walsh et al., (2003) Surg Endosc; 17(10):1514-20).

Cytologic examination of pancreatic juice is advocated as a diagnostic tool for early and potentially curable periampullary tumors (Nakaizumi et al., (1999) Hepatogastroenterology; 46(25):31-7) and can provide a tissue diagnosis to guide treatment, such as neoadjuvant chemoradiation. However, current cytologic methods are inadequate, with sensitivity as low as 30% (Ohuchida et al., (2004) Cancer; 101(10):2309-17; Hiyama et al., (1997) Cancer Res; 57(2):326-31 ). Pancreatic juice cytology is limited by scant cellularity in specimens, difficulty differentiating neoplastic from inflammatory changes, and technical errors in sample preparation (Enayati et al., (1996) Am J Surg; 171(5):525-8; Henke et al., (2002) Adv Anat Pathol; 9(5):301-8; Mitchell et al., (1985) Am J Clin Pathol;83(2):171-6; ). In contrast to the sensitivity of using compositions and methods of the present inventions that provide accuracy and sensitivities of at least 75% and up to 92%.

Besides the usefulness for early cancer detection, determinination of the presence and abundance of cancer cells in bodily fluids is useful in the monitoring of cancer progression, cancer prognosis and determination of cancer treatment effectiveness. Studies suggested that the presence of circulating tumor cells in patients with metastatic carcinoma is associated with short survival (Cristofanilli et al., N Engl J Med. 2004 Aug 19;351(8):781-91). Therefore, sensitive detection and quantification of cancer cells in bodily fluids, particularly prior to metastasis,can be used as tools to determine cancer presence and further to determine treatment effectiveness, cancer progression and cancer prognosis.

### II. Oncolytic Viruses.

Many attempts have been made to exploit the cell killing properties of normal viruses, such as measles, vesicular stomatitis, bovine enterovirus, and Newcastle disease virus for the treatment of cancer. Because of concerns related to the toxicities of wild type viruses, recent studies of oncolytic viral therapy have focused on genetically engineered viruses that are more specific in infecting cancer cells and thus less toxic to man. One such promising candidate virus for human therapy is the herpes simplex virus (HSV). The herpes virus of the present inventions is a second-generation, genetically engineered multimutated herpes virus that has high specificity for infection of tumor cells.

A number of studies by the inventors and others have determined that these viruses are highly selective in infecting many tumor types, including lung, bladder, head and neck, breast, esophageal, cervical, colorectal, gastric cancer and mesothelioma, and spare normal cells (Bennett et al., Interleukin 12 secretion enhances antitumor efficacy of oncolytic herpes simplex viral therapy for colorectal cancer, Ann Surg 2001; 233(6):819-826; Jamagin et al. Neoadjuvant treatment of hepatic malignancy: an oncolytic herpes simplex virus expressing IL-12 effectively treats the parent tumor and protects against recurrence-after resection, Cancer Gene Therapy 2003; 10(3):215-223; Bennett et al. Comparison of safety, delivery, and efficacy of two oncolytic herpes viruses (G207 and NV1020) for peritoneal cancer, Cancer Gene Therapy 2002; 9(11):935-945; Cozzi et al. Oncolytic viral gene therapy for prostate cancer using two attenuated, replication-competent, genetically engineered herpes simplex viruses, Prostate 2002; 53(2):95-100; Stanziale et al. Ionizing radiation potentiates the antitumor efficacy of oncolytic herpes simplex virus G207 by upregulating ribonucleotide reductase, Surgery 2002; 132(2):353-359; Carew et al. A novel approach to cancer therapy using an oncolytic herpes virus to package amplicons containing cytokine genes, Molecular Therapy 2001; 4(3):250-256; Stanziale et al. Oncolytic herpes simplex virus-1 mutant expressing green fluorescent protein can detect and treat peritoneal cancer, Hum Gene Ther 2004; 15(6):609-618; Stiles et al. The replication-competent oncolytic herpes simplex mutant virus NV1066 is effective in the treatment of esophageal cancer, Surgery 2003; 134(2):357-364; Bennett et al. Up-regulation of GADD34 mediates the synergistic anticancer activity of mitomycin C and a gamma134.5 deleted oncolytic herpes virus (G207), FASEB J 2004; 18(9):1001-1003 ).

Stanziale et al., Hum Gene Ther 2004; 15(6): 609-618 and Stiles et al., Surgery 2003; 134(2): 357-364 tested NV1066 infectivity of human cancer cell lines in the background of normal mouse tissue, wherein the present invention for the first time tested and surprisingly showed that said virus was capable of specifically identifying a primary cancer cell when infected with said virus *in vitro* in the background of normal human cells efficiently and it was noticed that the intensity of the reporter molecule within a cancer cell correlates with cancer proliferation rate (cancer aggressiveness).

The present invention provides that the tumor specificity of this class of viruses may be used for early detection of a wide spectrum of cancers. Examples of oncolytic viruses (OV) are provided in the Tables below.

**Table 1. Oncolytic Viruses (Ovs) targeting oncogenic ras or defective Interferon pathways.**

| **Virus (Company, if known)** | **Viral gene defect** | **Cellular Target** | **Tumor models** | **References*** |
|---|---|---|---|---|
| Influenza A | NS1 | PKR | Melanoma | Bergmann, et al., 2001, Cancer Res, 61:8188-8193 |
| HSV1mutants: R3616, 1716, ICP34.5 G207 (Medigene, Inc.), MGH1 | ICP34.5 | Protein phosphatase 1a, Defective interferon signaling. | Brain, Colorectal, ovarian, lung, prostate, breast | Leib, et al., 2000, Proc Natl Acad Sci 97:6097-6101; Farassati, et al., 2001, Nat Cell Biol;745-750 |
| Reovirus (Oncolytics Biotech. Inc.) | None | Overactive Ras pathway | Brain, ovarian, breast colorectal | Strong, et al., 1998, Embo J, 173351-3362; Coffey, et al., 1998, Science, 282:1332-1334; Norman, et al, 2002 Ther, Hum Gene Ther, 13:641-652; Wilcox, et al., 2001, J Natl Cancer Inst, 93:903- |
| HSV | None | Defective Interferon signaling | Melanoma | Stojdl, et al., 2000 Nat Med, 6:821-825 |
| Newcastle disease virus (Provirus) | None | Overactive Ras pathway 86:1228-1233 | Fibrosarcoma, Neuroblastoma | Lorence, *et al.*, 1994, J Natl cancer Inst, |

| | | | | |
|---|---|---|---|---|
| * References listed are herein incorporated in their entirety. | | | | |

**Table 2. OVs targeting defective p16 tumor suppressor pathways.**

| **Virus (Company, if known)** | **Mutated viral gene** | **Cellular target** | **Effect** | **References*** |
|---|---|---|---|---|
| Adenovirus D24 and d1922-947 (Onyx Phannaceuticals) | E1A-CR2 domain | PRB | Viral replication restricted to defective | Fueyo, et al., 2000, Oncogene, pRB- 19:2-12; Heise, et mutants al., 2000, Nat Med, 6:1134-1139 |
| Adenovirus CB106 | E1A-CR1and CR2 domains | PRB, p300, p107, viral replication p130 | In keratinocytes, restricted to papillomavirus 75:7602-7611 E6/E7 expressors | Balague, et al., 2001, J Virol, |
| Adenovirus ONYX-411(Onyx Pharmaceuticals) | a) E1A-CR1 b) E2F promoterPRB driving E1A E4 genes c) E3 genes deletion | PRB and upregulated E2F transcription factor | Increased dependence of virus replication on overactive E2F | Johnson, et al., 2002, Cancer Cell, 1:325-337 |
| HSV: hrR3, rRp450, HSV1yCD, MGH1, G207 (Medigene, Inc.), G47Δ) | U139 (ICP6) | RR activity elevating dNTP pools | Viral replication depends on 330; Chase, *et al.*, pools | Carroll, et al., 1996, Ann Surg, 224:323-329; discussion 329-1998, Nat Biotechnol, 16:444-448 |
| HSV Myb34.5 (Prestwick Scientific, Inc.) | a)UL39(ICP6) b) B-Myb (E2F-responsive) promoter γ34.5 gene) | RR activity elevating dNTP pools and driving upregulated E2F transcription | Increased viral replicative dependence on E2F activity | Chung, et al., 1999, J Virol, 73:7556-7564; Nakamura, et al., 2002, J Clin Invest, 109:871-882 |
| Vaccinia vvDD-GFP | TK gene | Elevated dTTP (due to cellular TK?) | Viral replication restricted to cells with dTTP pools | McCart, et al., 2001, Cancer Res, 61:8751-8757; Puhlmann, et al., 1999, Hum Gene Ther, 10:649-657 |

| | | | | |
|---|---|---|---|---|
| * References listed are herein incorporated in their entirety. | | | | |

**Table 3. OVs targeting defective p53 tumor suppressor pathway.**

| **Virus (Company, if known)** | **Mutated viral gene** | **Cellular target** | **Effect** | **References*** |
|---|---|---|---|---|
| Adenovirus ONYX-015 (Onyx Pharmaceuticals) | E1B-55Kd | mutants | Viral replication restricted to defective | Bischoff, et al., 1996, Science, 274:373-376; Tollefson, et al., 1996, J Virol, 70: 22962306; Ramachandra, et al., 2001, Nat Biotechnol, 19:1035-1041; Raj, et al., 2001, Nature, p53- 412:914-917; Rodriguez, et al., 1997, Cancer Res, 57:2559-2563; Yu, et al., 1999, Cancer Res, 59:4200-4203; Chen, et al., 2001, Cancer Res, 61:5453-5460; Li, et al., 2001, Cancer Res, 61:6428-6436 |
| Adenovirus 01/PEME(Canji) | 1) p53 promoter expression of E2F antagonist 2)E1A-CR1 p300 binding-domain 3) E3 deletion 4) Extra Major Late Promoter driving expression of E3-11.6 Kd | p53, p300. | expression of E2 and subsequent viral genes dependent on loss of p53 function; wild-type p53 function enhanced by p300 coactivation; increased adenoviral release and cell death by adenoviral death protein (21) | Ramachandra, et al., 2001, Nat Biotechnol, 19:1035-1041 |
| AAV | AAV unusual DNA structure is precipitating factor | p53/ p21 | Lack of G2/M arrest in p53-defective cells, infected with AAV, causes cell death | Raj, et al., 2001, Nature, 412:914-917 |

| | | | | |
|---|---|---|---|---|
| * References listed are herein incorporated in their entirety. | | | | |

**Table 4. Targeting of OV with tumor-specific promoters.**

| **Virus (Company, if known)** | **Tumor-specific Promoter** | **Viral gene** | **Effect** | **References*** |
|---|---|---|---|---|
| Adenovirus CV706 (Calydon, Inc.) | PSA (prostate) | E1A | Replication restricted to prostate tissue | Rodriguez, et al., 1997, Cancer Res, 57:2559-2563 |
| Adenovirus CN787 (Calydon, Inc.) | a) Rat probasin promoter for E1A b) PSA for E1B | E1A and E1B | Same as above | Mu, et al., 1999, Cancer Res, 59:4200-4203; Chen, et al.,2001, Cancer Res, 61:5453-5460; |
| Adenovirus CV980(Calydon, Inc.) | AFP (hepatocellular carcinoma) | E1A and E1B | Replication restricted to hepatic Cancer tumors. | Li, et al., 2001, Res, 61:6428-6436 |
| Adenovirus ONYX-411 Pharmaceuticals) | (Onyx E2F1 promoter (most tumors) | E1A and E4 | Increased dependence of virus replication on overactive E2F | Johnson, et al., 2002, Cancer Cell, 1: 325-337 |
| Adenovirus 01/PEME (Canji Inc.) | p53 promoter (most tumors) | E2F antagonist viral | Expression ofE2 and subsequent genes dependent on loss of p53 function | Ramachandra, et al., 2001, Nat Biotechnol, 19:1035-1041; |
| CG8840 (Cell Genesys, Inc.) Inc. | Uroplakin II (bladder) | E1A and E1B | Replication restricted to bladder cancer | Zhang, et al., 2002, Cancer Res, 62:3743-3750; |
| KD1-SPB | Surfactant protein | E4 | Replication improved in lung tumors | Doronin, et al., 2001, J Virol, 75:3314-3324; |
| HSV Myb34.5 (Prestwick Scientific, Inc.) | B-Myb promoter (most tumors) | g34.5 (ICP34.5) | Improved replication in tumors | Chung, et al., 1999, J Virol, 73:7556-7564; Nakamura, et al., 2002, J Clin Invest, 109:871-882 |
| HSV DF3g34.5 | DF3 promoter | g34.5 | Improved replication in (ICP34.5) MUC1-positive pancreatic and breast tumor cells. | Mullen, *et al.* Annals of Surgery, in press |
| HSV G92A promoter | Albumin promoter | ICP4 | Replication restricted in hepatoma | Miyatake, et al., 1999, Gene Ther, 6:564-572 |

| | | | | |
|---|---|---|---|---|
| * References listed are herein incorporated in their entirety. | | | | |

**Table 5. Targeting with "tumor-selective" infection.**

| **Virus** | **Redirected viral ligand** | **Cellular target** | **effect** | **References*** |
|---|---|---|---|---|
| Dual Adenovirus system: AdsCAR-EGF +Δ24 | antibody binding adenovirus fiber adenovirus fiber to EGFR | EGFR | Redirects viral infection to EGFR-expressing cells | Hemminki, et al., 2001, Cancer Res, 61:6377-6381 |
| Adenovirus: Ad5- of D24RGD | H1-loop in Fiber Ad modified by incorporation of RGD | Integrin | Redirects viral infection to integrin-expressing 1998, J Virol, cells. | Dmitriev, *et al.,* 72:9706-9713 |
| D24 or ONYX-015 | Infusion of bispecific antibodies to and EGFR | fiber EGFR | Redirects viral infection to EGFR- 2002, expressing cells | van der Poel, et al., J Urol, 168:266-272 |
| And 5/35 | Fiber of adenovirus serotype 35 substituted into adenovirus serotype 5 | Unknown | Redirects viral infection away from CAR and towards an unidentified cellular receptor present in human breast cancer | Shayakhmetov, et al., 2002, Cancer Res, 62:1063-1068 |

| | | | | |
|---|---|---|---|---|
| * References listed are herein incorporated in their entirety. | | | | |

**Table 6. Other mechanisms of OV targeting.**

| **Virus** | **Defect in viral gene** | **Effect** | **Oncolytic mechanism** | **References*** |
|---|---|---|---|---|
| Vaccinia vvDD-GFP | Vaccinia Growth Cannot Factor normal cells | prime to divide | Dividing tumor cells will replicate cells, because normal cells are not "primed" by VGF | McCart, *et al.,* 61:8751-8757 not |
| PV1 (RIPO) | Substitutes poliovirus IRES element with IRES | Loss of neurovirulence, because neurons cannot translate rhinovirus 2 with substituted IRES | Tumor cells can still propagate | Gromeier, et al., Acad Sci, 97:6803-2000, Proc Natl Acad Sci, 97:6803-6808 |
| Adenovirus E1- | E1 | Does not replicate | Tumor cells can complement the defect | Nevins, 1981, Cell, 26:213-220; E1 Steinwaerder, et al., 2000, Hum Gene Ther, 11:1933-1948 |
| Adenovirus Ad.IR.BG | E1 defect with inverted repeats flanking reporter transgene in antisense orientation to promoter | DNA replication rearranges the construct so that promoter is 5' to reporter | Adenoviral replication occurs in tumor cells that complement the E1 defect hut not in defect cells transgene to complement the E1 defect | Steinwaerder, et al., 2001, Nat Med, 7:240-243 unable |
| Measles, mumps, Sindbis, Sendai | None | Tumor lysis | Blood, Unknown | Grote, et al., 2001, 97:3746-3754; Asada, 1974, Cancer, 34: 907-1928 |

| | | | | |
|---|---|---|---|---|
| * References listed are herein incorporated in their entirety. | | | | |

**Table 7. OVs that express anti-cancer cDNAs.**

| **Virus** | **Viral gene defect** | **Anticancer cDNA** | **Prodrug> Metabolite** | **Effect** | **Reference*** |
|---|---|---|---|---|---|
| HSV1: hrR3, MGH1, G207 (Medigene, Inc.) | ICP6 and/or ICP34.5 | TK | Ganciclovir>GCV-Phosphate | Predominant anticancer action 8:2057-2068; some situations, increased antiviral action in others | Boviatsis, et al., 1994, Cancer Res, 54:5745-5751; Kramm, et al., 1996, Hum Gene Ther, 7:1989-1994; Kasuya, et al., 1999, J Surg Oncol, 72:136-141; Kramm, et al., 1997, Hum Gene Ther, but Carroll, et al., 1997. J Surg Res, 69:413-417; Yoon, et al., 1998, Ann Surg, 228:366-374; Todo, et al., 2000, Cancer Gene Ther, 7:939-946; Samoto, et al., 2002, Neurosurgery, 50:599-605; discussion 605-596 |
| HSV1. rRp450 | ICP6 | CYP2B1 | Cyclophosphamide> Mustard Phosphoramide | Predominant anticancer action + 1998, Nat immunosuppressive Biotechnol, effects. | Chase, *et al*., 16:444-448 |
| Adenovirus: FGR | E1BSSkD | TK CD geneTK-CD gene | Ganciclovir> GCV-Phosphate + 5-fluorocytosine> 5fluorouracil | Combination of FGR, GCV, 5FC and radiation shows and predominant anticancer action | Freytag, et al., 1998,Hum Gene Ther, 9:1323-1333 |
| HSV1 :Fu-10 | Unknown | Fusogenic glycoprotein | Not applicable | Enhanced fusion of cell membranes caused by replicating virus increases anticancer effect | Fu,and Zhang, 2002, cancer Res 62:2306-2312 |
| Adenovirus: ad5/IFN= | E3 | Interferon | Not applicable | Increased anticancer effect compared to control E3-deleted adenovirus | Zhang, et al., 1996, Proc Natl Acad Sci 93:4513-4518 |
| Adenovirus; Ad.TK^{RC},Ad.OW34 | E1B55KD | TK | Ganciclovir> GCV-Phosphate | Contradictory anticancer effects | Wildner, et al., 1999, Cancer Res, 59:410-413 Morris, and Wildner, 2000, Mol Ther, 1:56-62 |
| Adenovirus: Ig.Ad5E1 + .E3TK | E3-19K | TK | Ganciclovir> GCV-Phosphate | Increased anticancer effect anticancer effect in glioma | Nanda, et al., 2001, Cancer Res, 61:8743-8750 |
| HSV1: Mix of G207 + Defective HSv-IL2 | ICP6 and ICP34.5 | IL2 | Not applicable | At low dose, the mix was more effective than either virus alone | Zager, et al., 2001, Mol Med, 7:561-568 |
| HSV1: NV1042 | Complex | IL12 | Not applicable | Increased anticancer effect | Wong, et al., 2001,Hum Gene Ther, 12:253-265; 58 |
| HSV1: Mix of G207 + Defective HSV-soluble B7-1 | ICP6 and ICP34.5 | Soluble B7-1 | Not applicable | Increased anticancer effect | Todo, et al., 2001. Cancer Res, 61:153- 61 |
| HSV1: HSV1yCD | ICP6 | Yeast deaminase | cytosine 5-fluorocytosine> 5-fluorouracil | Increased anticancer effect minimal antiviral effect | Nakamura, et al.,2001 Cancer Res, 61:5447- 5452 |
| VCD Vaccinia: | TK | Bacterial cytosine deaminase | 5- fluorocytosine> 5-fluorouracil | Increased effect at low viral dose | McCart, 2000, Gene Ther, 7:1217-1223 |
| HSV1 | ICP34.5 | IL4 IL12 IL10 | Not applicable | Increased anticancer effect for IL-12 and II-4, but antagonistic effect for IL-10 | Andreansky, et al., 1998, Gene Ther, 5:121 130; Parker, et al.,2000, Proc Natl Acad Sci 97:2208-2213 |

| | | | | | |
|---|---|---|---|---|---|
| * References listed are herein incorporated in their entirety. | | | | | |

### III. Additional Biopsy Methods for Collecting test cells and cancer cells.

There are numerous methods for collecting fluids and tissue biopsies for providing test cells for evaluation using cancer cell detection compositions and methods of the present inventions. The following are biopsy methods that are provided merely as examples

Brush biopsy methods are provided as ways to obtain cells samples for analyzation using compositions and methods of the present inventions. A brush biopsy of tissue is obtained using a soft nylon brush or steel brush, such as to obtain a full transepithelial biopsy specimen of oral lesions. Brushes can also be used to obtain brush biopsies of bronchial specimens, bronchoalveolar lavage fluid, nasal swabs, biliary duct and pancreatic samples. However, where tissues are difficult to reach by hand, a brush or other cell collection device is used in combination with an endoscope or specifically a cystoscope.

An endoscopic biopsy refers to a type of biopsy that is done through an endoscope, often a fiberoptic endoscope, that is inserted into a body opening, such as the gastrointestinal tract (alimentary tract endoscopy), urinary bladder (cystoscopy), abdominal cavity (laparoscopy), joint cavity (arthroscopy), mid-portion of the chest (mediastinoscopy), or trachea and bronchial system (laryngoscopy and bronchoscopy), either through a natural body orifice or through a small surgical incision. An endoscope refers to a device with a light attached that is used to look inside a body cavity or organ, referred to as "endoscopy." Thus an endoscopist can directly visualize an abnormal area in or on the organ in question. An endoscope may also have a tool to remove tissue or provide a means for cell or tissue removal, such as a channel for insertion of mechanical devices, for example, a tool for tissue or cell removal. A long cable with a brush or forceps attached to the end can either be inserted inside the endoscope or into a parallel tube, or inserted parallel to the endoscope or along a guide wire in order to brush off cells or pinch off tiny bits of tissue, respectively. The cells or tissue are then analyzed using compositions and methods of the present inventions.

A cystoscope refers to an endoscope specialized for us in the digestive tract, comprising a long, thin tube that is placed through a body duct (urethra, ureter, bladder, intestine, esophagus, biliary tract, etc.) in order to visualize and/or reach a target area for collecting cells, such a procedure is referred to as "cystoscopy." After the end of the cystoscope has reached the target, a guide wire may be inserted through the cystoscope and into the tract for inserting additional tools or devices. A small camera at the end of the cystoscope or other tool is used to visualize the inside of an area of interest (bladder, kidney, gallbladder, pancreas, etc) while passed over or beside the guide wire. Once the suspect area is identified, a nylon or steel brush or biopsy forceps is placed through the cystoscope and the lesion is rubbed with the brush, or a cell or tissue sample is collected with the biopsy forceps. When the brush or biopsy forceps is removed, the tissue from the lesion is removed from the instrument and then analyzed using compositions and methods of the present inventions. Following removal of the cell sample, the instrument and guide wire are completely removed from the body.

Colposcopic biopsy refers to a gynecologic procedure that typically is used to evaluate a patient who has had an abnormal Pap smear. The colposcope is actually a close-focusing telescope that allows the physician to see in detail abnormal areas on the cervix of the uterus, so that a good representation of the abnormal area can be identified, removed and analyzed using compositions and methods of the present inventions.

A fine needle aspiration (FNA) biopsy refers to a simple technique by using a needle no wider than that typically used to give routine injections (22 to 25 gauge) to insert into a suspected tumor lump, wherein a few tens to thousands of cells are drawn up (aspirated) into a syringe. These collected cells are smeared onto a slide for analysis using compositions and methods of the present inventions. Tumors of deep, hard-to-get-to structures (pancreas, lung, and liver, for instance) are especially good candidates for FNA as are thyroid lumps, thus avoiding using major surgery for suspected cancer cell collection. Such FNA procedures are typically done by a radiologist under guidance by ultrasound or computed tomography (CT scan) and require no anesthesia and may avoid the need for local anesthesia.

Stereotactic needle biopsy refers to a technique used for evaluating breast lesions by combining the advantages of FNA (no scar, no anesthesia, inexpensive), excisional biopsy (acquisition of solid pieces of tissue rather than smears) and needle localization (precise guidance by x-ray or ultrasound imaging). The patient lies on her abdomen, so that the breast hangs down into a space that can be x-rayed by a computerized imaging device. The computer displays the mammographic image on a screen. The radiologist identifies the abnormality and marks it electronically on the screen. The computer then positions a movable arm directly over the abnormal area. A biopsy device is attached to the arm, and the spring-loaded gun quickly inserts a hollow biopsy needle into the breast. The needle is removed, and the tissue it contains is analyzed using compositions and methods of the present inventions.

### DESCRIPTION OF THE INVENTION

The invention is defined in the claims and relates to compositions and methods for cancer cell detection in bodily samples wherein a cancer cell can be detected within a mixed population of cancer cells and non-cancer cells. The invention also relates to compositions and methods that may be used in cancer cell detection, specifically viruses that are replication-competent conditional to a cancer cell, in particular an oncolytic herpes virus, such as NV1066 and a vaccinia virus, such as GLV-1h68. Provided are methods for using these viruses that preferentially replicate in cancer cells and may also preferentially infect cancer cells for specific identification of such cancer cells, even when a cancer cell is present, for example, at a ratio of one infected cancer cell in a background of ten thousand non-cancer cells, thus further providing a reproducible and sensitive screening method for cancer detection, monitoring and prognosis.

For the purposes of the present inventions, the inventors present simple methods for detection of cancer cells in biological specimens. Using methods of the present inventions, fluorescence assisted cytological testing (FACT), in combination with NV1066 or GLV-1h68, for example, was capable of detecting one cancer cell in the background of one million normal cells. In particular, NV1066 detected cancer cells with a sensitivity of > 92%. Furthermore, the data the inventor presents herein shows that NV1066 is specific for allowing the cancer cell selective expression of a reporter gene a wide panel of cancer cell types comprising one hundred and eleven human cancer cell lines from sixteen different primary organs.

### I. Advantages of using compositions and methods of the present inventions.

The following section describes certain exemplary advantages of the compositions and methods of the present invention. It should be understood, that the invention is not necessarily limited to compositions and methods that take advantage of any one or more of these advantages in any particular application. For example, the compositions and methods of the invention find uses even where the invention is applied such that one or more of the advantages is not used.

Methods of the present inventions have multiple additional advantages over the prior art. One advantage is providing a more sensitive level of cancer cell detection. For example, in one exemplary test on cancer cells in pancreatic juice, patients with invasive pancreatic or bile duct tumors were diagnosed as positive by green fluorescence in 18 of 24 (75%) patients compared to conventional cytology where 12 of 24 (50%) patients were deemed positive, while benign specimens did not express green fluorescence.

Another advantage is that due to a broad host range of the viruses of the present inventions, detection of cancer cells within a cell population is not limited to techniques known in the art that rely upon detecting the expression of certain tumor markers and further is not limited to detecting cancer cell markers.

In contrast to other approaches, the present inventions exploit the cancer cell and thus tumor selective replication with and without cancer cell selective infection, i.e. tumor selective infection, of genetically modified viruses in cancer cells. This leads to the multiplying of the reporter gene due to the tumor selective replication of the viral genome. Therefore, in a cancer cell, or a tumor cell, multiple copies of reporter molecules are expressed from multiple copies of the reporter genes that originate from a single infection event. As a consequence one can observe the high sensitivity of the present methods. Moreover, in the methods of the present inventions, the inventors found that the intensity of the reporter molecule within a cancer cell correlates with such cancer cell proliferation rate. The higher the marker intensity or marker presence in a given cancer cell, the more likely that the cancer, from where the cancer cell originated, is rapidly proliferating, providing an indication of an aggressive cancer. This has important clinical implications: FACT analysis allows the detection of cancer cells in body fluids, in addition to providing information for predicting the aggressiveness of cancer in that patient. If and when a cancer cell is detected in a bodily fluid sample, especially as a rare cell (e.g. one in a million), if that cell has greater fluorescence than neighboring cells and/or higher fluorescence than a non-cancer cell control, that particular patient should be investigated immediately and thoroughly for cancer. Within 24 hours, the methods described herein, can determine if a) the patient has cancer; b) is the cancer cell is considered aggressive; c) this particular patient will respond to oncolytic viral therapy (NV1066) or not, and d) this patient requires higher or lower doses of virus for oncolytic viral therapy.

Another unique feature of one of the embodiments of the present inventions is that the reporter gene is actually cancer specific-specifically amplified by the cancer selective replication of the viral genome as a whole, which leads to a much higher sensitivity compared to the alternative of transfection of a simple reporter construct where the reporter gene may be controlled by a cancer specific promoter/enhancer, but which virus copy number remains constant because the virus does not replicate inside the cancer cell. Thus, in some embodiments of the inventions the promoter that controls the expression of the reporter gene is a promoter of the replication conditional virus with relative cancer cell selectivity. In other words the reporter gene is under the controls of a promoter for the replication-competent virus. As such, in some preferred embodiments the promoter is an early or a late expression promoter of the replication conditional virus, for example, such promoter will be silent in some cells that are infected by the virus such that the virus does not replicate. In cells where the virus is able to replicate, the promoter will be active and drive the expression of the reporter gene. Therefore, the sensitivity of the detection can be enhanced. Useful promoters of the disclosed virus are well known in the art. For HSV-1, preferred promoters are e.g. the β-promoter of the TK gene, the γ-promoter of U_{S}11 or the α-promoter of the ICP4 gene as an example for an early expression gene promoter.

Further, in the methods of the present inventions the presence of clumps or sheets of cells do not cause a problem, unlike in other methods, wherein clumps or sheets of cells causes non-uniform staining by biomarker detection chemicals and variation in fluorescence), because virus or its progeny can spread from cell to cell when cells are in clumps and when cells are confluent, for example, through gap junctions, by forming cell syncytia and the like. The inventors contemplate that FACT technique can be used for early diagnosis of a wide spectra of cancers with a high sensitivity of > 90%, even in the presence of few cancer cells amongst a clump of normal cells.

Normal cells in body fluids are difficult to keep alive in cell culture and generally die from the time of removal from the body. Malignant cells, on the other hand, can generally be kept alive in cell culture for several days or weeks. The present inventions exploit the dynamic nature of malignant cells in retaining their capacity to survive and stay metabolically active *in vitro.* These viable malignant cells in body fluids can be infected by the viruses of the present inventions, e.g. cancer cell specific replication-competent herpes viral mutants that, upon viral replication within a cell, express enhanced amounts of eGFP or any of the other markers recited above, and thus can be detected either by microscopy using fluorescent detection or by flow cytometer or by a luminometer. This detection ability is not compromised even when the sample of cells was preserved in ice or at room temperature for more than 12 hours. Thus, the current proposed methods of the present inventions could be used successfully even when the specimen collection methods are not stringent. Furthermore, the inventors' showed that by combining fluorescent imaging with traditional cytological techniques further enhanced the capability for detecting cancer and tumor cells.

Recently, several investigators proposed detecting cancer cells by automated techniques utilizing the differences in cellular autofluorescence and cell size between malignant and normal cells. Use of cellular autofluorescence as a means of delineating tumor cells from surrounding normal cells is constrained by the narrow range of tumor/normal cell signal intensity ratio and unpredictable cellular morphology and autofluorescence *in vitro.* However, as shown in examples of the present inventions, eGFP expressed following NV 1066 replication within a cancer cell is a stable protein with fluorescence levels several folds higher than cellular autofluorescence and does not require additional substrates or cofactors for its expression, see, for example. Figure 2. As eGFP is an internal stain (cytoplasmic), rather than on the surface, its fluorescence is not susceptible to environment-dependent quenching. Furthermore, the fluorescence intensity of enhanced eGFP is pH-insensitive (Kneen et al., 1998, Biophys J 74(3):1 S91-1599). In addition, the combination of excitation and emission wavelengths for enhanced eGFP is specific and thus eliminates autofluorescence interference.

Inflammatory cells often interfere with the diagnosis of cancer because of cell size, texture, and fluorescence. Because these inflammatory cells have different excitation-emission wavelengths compared to eGFP (GFP 475/509 mM. tryptophan 290/330 nM, NAD(P)H 350/450 nM, FAD 450/530 nM) misdiagnosis of recognizing inflammatory cells as cancer cells is avoided by the FACT method (Heintzelman et al., 2000, Photochem Photobiol 71 (3):327-332).

The inventors used the selectivity of NV 1066 for replicating in tumor cells and its transduction of green fluorescence in order to detect peritoneal and pleural micrometastases in mice using fluorescence laparoscopy and thoracoscopy (Stiles et al., (2006) Cancer Gene Therapy; 13(1):53-64; Stanziale et al., (2004) Hum Gene Ther; 15(6):609-18).

The inventors describe herein an increased capability to provide cytologic diagnosis of human cancer cells, including periampullary cancers, by using fluorescence assisted cytological testing (FACT) in combination with an oncolytic herpes simplex virus 1 (HSV-1) or a vaccinia virus (GLV-1h68) for ex vivo detection of cancer cells. NV1066, an oncolytic HSV-1, and GLV-1h68 were genetically engineered to selectively infect and/or replicate in tumor cells and encodes a transgene for enhanced green fluorescent protein (eGFP) under the control of a constitutive cytomegalovirus (CMV) promoter (Wong et al., (2002) Human Gene Therapy;13(10):1213-23; Stiles et al., (2003) Surgery;134(2):357-64; Yu, et al. Nature Biotechnol. 2004 Mar;22(3):313-20). Green fluorescence is expressed 1-6 hours after viral entry into cells and can be detected and quantified with fluorescence microscopy and flow cytometry (Foster et al., (1998) J Viral Methods;75(2):151-60; Stiles et al., (2006) Cancer Gene Therapy; 13(1):53-64; herein incorporated by reference).

FACT can be implemented with currently available equipment and personnel. The specimens that screen positive by FACT can be sent to the pathologist to further characterize the exact nature of malignancy. After fluorescence microscopic Examination, the results can be confirmed by routine histological methods or the fluorescent cells can be sorted for further characterization, see, for example, Figure 8.

Using FACT analysis in combiantion with compositions of the present invention for cancer cell identification is a practical method of cancer diagnosis. While currently used immunohistochemical and PCR-based methods are handicapped by costliness, multiple steps, and long processing times (Trumper et al., (2002) J Clin Oncol; 20(21):4331: 4331-7), FACT comprises merely two steps - incubation of samples with NV1066 or GLV-1h68 and then examination under fluorescence, either by microscopy or flow cytometry. One example of advantages of using FACT based analysis is provided in an analysis of biliary and pancreatic duct cytology, 17% of false-negative diagnoses resulted from technical errors, specifically air-drying artifact (Logrono et al., (2000) Arch Pathol Lab Med;124(3):387-92 ). In contrast, FACT incurs no risk of air-drying artifact because the specimen undergoes minimal processing and cells are maintained in culture media. Thus, FACT can be performed in clinics that lack specialized cytology facilities, avoiding cumbersome histopathological procedures.

The inventors additionally show the ease and reliability of cancer diagnosis with FACT. General pathologists infrequently evaluate pancreaticobiliary cytology specimens, in which findings can be subtle and difficult to interpret, resulting in high intra- and interobserver variability (Henke, et al., (2002) Adv Anat Pathol;9(5):301-8; Khalid, et al., (2005) Clin Lab Med 2005;25(1):101-16; Logrono, et al., (2000) Arch Pathol Lab Med;124(3):387-92). In a study of interpathologist variability in biliary cytology interpretation, agreement between pathologists was as low as 48% (Harewood et al., (2004) Am J Gastroenterol; 99(8): 1464-9).

Conventional cytology requires the identification of rare cancer cells under a microscope, which can be arduous and may miss the few atypical cells in a background of normal cells or necrotic debris (Nakaizumi et al., (1999) Hepatogastroenterology; 46(25):31-7). In contrast,observers quickly identified a single green fluorescent cell among many nonfluorescent cells. In order to test the reliability of NV1066-transduced eGFP expression as a method of cancer cell detection, the inventor performed a blinded study of 15 inexperienced observers who lacked pathology training. The inventors found that inexperienced observers diagnosed malignancy based on green fluorescence with 97% accuracy. Further, ninety-nine percent of benign samples were correctly identified by the absence of green fluorescence.

Thus FACT based methods of the present inventions were used to successfully identify many cancer cell types, regardless of stage and grade. In contrast, other methods that aim to increase the sensitivity of cytology such as immunohistochemical and molecular assays require tumor-specific antibodies or genetic targets, restricting their use to specific tumors and requiring previous knowledge of antigenic or molecular changes. As shown herein, green fluorescence was detected in patient samples of multiple periampullary tumors, including pancreatic adenocarcinoma and cholangiocarcinoma. Notably, NV1066-mediated detection of cancer from pancreatic juice was more sensitive for tumors of the pancreas and bile duct than the duodenum and ampulla, although the invention may be used in any of these settings. This is likely due to the lower number of exfoliated cancer cells present in the pancreatic duct from duodenal and ampullary tumors. The detection of various tumor types by FACT show its applicability in other body fluids, such as sputum and urine for the diagnosis of lung and bladder cancers.

Thus, the inventors developed novel methods of the present inventions to label tumor cells with a reporter protein, for example an enhanced green fluorescence protein that will improve detection of cancer cells in a background of non-cancer cells. SpecificaUy, the inventors used a herpes virus that shows specific infection and/or replication in cancer cells, to label cancer cells *in vitro.* NV1066, a mutant herpes virus that carries a gene for green fluorescent protein, is used to treat cell mixtures, for example, that resemble sputum or urine specimens, and results in specific expression of eGFP in tumor cells [which aids in their detection]. This method is demonstrated to be feasible among a wide range of cancers from different primary organs. The results of the composition and methods of the present inventions would encourage clinical testing of FACT in the early detection of human malignancies.

### EXPERIMENTAL

The following examples serve to illustrate certain preferred embodiments and aspects of the present inventions and are not to be construed as limiting the scope thereof.

In the experimental disclosure which follows, the following abbreviations apply: M (molar); mM (millimolar); µM (micromolar); nM (nanomolar); mol (moles); mmol (millimoles); µmol (micromoles); nmol (nanomoles); gm (grams); mg (milligrams); µg (micrograms); pg (picograms); L (liters); ml (milliliters); µl (microliters); cm (centimeters); mm (millimeters); µm (micrometers); nm (nanometers); and °C (degrees Centigrade/Celsius).

### EXAMPLE 1

### Materials And Methods

### I. Construction of viruses for use in the present inventions:

### A. Construction of a Herpes Simplex Virus for use in the present inventions, for example. NV1066. is briefly described below.

NV1066 refers to an attenuated oncolytic herpes virus that expresses enhanced green fluorescent protein (eGFP). *See,* Figure 1. Specifically, an engineered herpes virus was constructed when a transgene encoding eGFP, under a constitutive CMV promoter, was inserted into the internal repeat sequence of the parent virus, resulting in deletions in one copy each of the viral genes encoding *ICP-4, ICP-0,* and γ*₁34.5.* These deletions rendered the virus selective for infection and replication in tumor cells while attenuating its' potential neurovirulence.

More specifically, NV1066 was constructed by transfecting a BAC mid (BAC 17-28), a cosmid (cos12a), and a plasmid (pUL56-GFP-US1) containing overlapping and contiguous HSV-1 sequences into Vero cells with LipofectAMINE according to the manufacturer's protocol (GIBCO Invitrogen, Carlsbad, CA) and cells were incubated at 37° Celcius. Infectious viruses were isolated 3 days later and virus stocks were generated. The structure of the recombinant virus was confirmed by Southern blotting and sequencing (Wong et al., 2002, Hum Gene Ther 13(10):1213-1223).

The three transfected molecules are briefly described as follows. BAC 17-28 and cos 12a contained HSV-1 sequences, UL1 to UL56 and, US1-US12, the terminal repeat, and, UL1 to 9, respectively. pUL56-GFP-US1 contained HSV sequences, UL54 to UL56, and, US1 to US3. A CMV-eGFP cassette, obtained from Genentech, Inc., was cloned between the HSV UL and US sequences. Recombination between the three molecules resulted in generation of a vector deficient for the internal repeat sequences (positions 116 262-131 537) and contained enhanced GFP (eGFP) sequences under the control of the cytomegalovirus (CMV) promoter in the deleted joint (effectively between UL56 and US 1) (Fig.1). The resultant virus is one with high specificity for infection of mouse and human cancer cells and constitutively expresses the marker gene for green fluorescent protein.

### B. Construction of a Vaccinia Virus for use in tumor-specific replication of the present inventions, for example, GLV-1h68, is briefly described below.

GLV-1h68 refers to an engineered vaccinia virus constructed to express eGFP in cancer cells. In brief, a GLV-1h68 virus comprising a gene encoding eGFP was provided using a lister vaccine (LIVP) strain of Vaccinia Virus as a parental virus. LIVP strain is attenuated and has the Thymidine Kinase gene deleted.

Recombinant vaccinia virus rVV-RUC-GFP was constructed by inserting via homologous recombination the RUC-GFP cassette 39 (Yu, et al."Visualization of tumors and metastases in live animals with bacteria and vaccinia virus encoding light-emitting proteins." Nat Biotechnol. 2004 Mar,22(3):313-20), which contains the RUC and GFP cDNA sequences under the control of a synthetic early/late promoter of vaccinia, into the nonessential region of the vaccinia virus genome.

### II. Cancer Cell Lines:

The results from screening one hundred eleven human cancer cell lines from sixteen different primary organs are described herein. (See, Example 2 and Table 8). Human cancer cell lines were obtained from the American Type Culture Collection (ATCC, Rockville, MD) and independent investigators. Cells were maintained in appropriate media as directed by instructions provided by ATCC and investigators and were incubated in a humidified incubator supplied with 5% carbon dioxide at 37° Celcius.

### III. Collection of Normal Cells:

Normal (non-cancerous) cells were collected from the representative organs of the cancer type of origin in mice or humans. Normal cells were collected from lung, esophagus, pleura, peritoneum, liver, kidney, urinary bladder, stomach, and gallbladder of mice without cancer or humans as described or referenced herein. Individual organs of each mouse were collected after euthanasia with minimal contamination of blood, minced with a scalpel, incubated with collagenase *in vitro,* filtered through nylon mesh and isolated by centrifugation. Accurate counts of harvested cell suspension were made using trypan blue staining and manual counting on a hemocytometer. For example, to collect normal bronchial and alveolar cells, mice were euthanized; the trachea was cannulated with a 22-G Angiocath. Through the Angiocath, 1 ml saline was injected and aspirated three times into and from the trachea and lungs. The collected bronchioalveolar fluid was centrifuged at 3000 rpm x 15 min. at 4° C to separate cells from supernatant. NV1066 was shown to infect mouse cancer cells, for example, Wong et al., Hum Gene Ther. 2002 Jul 1;13( 10):1213-23.

### IV. Imaging:

Zeiss LSM 510 confocal laser scanning microscope and Metamorph were used to visualize eGFP expressing cancer cells. Imaging was performed in both bright-field and fluorescent modes. Live cells were identified by Hoechst staining, examined under (4',6-Diamidino-2-phenylindole (DAPI), dimethylsulfoxide) filter, and eGFP expression identified after placement of both excitation and emission filters to detect eGFP. The excitation filter was fixed at passage 470 ± 40 nm wavelength light as eGFP has a minor excitation peak at 475 nm. The emission filter was fixed at 500 nm, to accommodate the emission peak of eGFP at 509 nm. The image-capture system consisted of a Retiga EX digital CCD camera (Qimaging, Burnaby, BC). Mathematical algorithms are used in the computer deconvolution to improve image quality by decreasing the "out-of-focus" fluorescence. *For one example, see,* Figure 10.

### V. Flow Cytometry Methods:

Standard flow cytometry was performed in accordance with guidelines outlined in the 1995 United States-Canadian consensus conference (Stelzer et al., 1997, Cytometry 30(5):214-230). Data acquisition analyses were performed on a FACScan flow cytometer (BD Biosciences). CellQuest software (Becton Dickinson Immunocytometry systems, San Jose, CA) was used for data analysis. Nonviable cells were identified by using 7-amino actinomycin D (7-AAD). Matched isotype controls were used in flow cytometry panels. Voltages were based on unstained cells, and compensation was set using single-stained positive controls for each color. A fixed gating was derived from pilot experiments and was used to accommodate different size and granularity of non-cancerous and cancer cells using forward and side scatter on a linear scale. eGFP expression was identified in FL-1 channel (green fluorescence) using logarithmic scale. Flow cytometry experiments were repeated by two independent investigators to ensure reproducibility. Each experiment was repeated at least three times.

### VI. Comparison of autofluorescence with eGFP fluorescence by Flow Cytometry:

The cancer cell lines (1 x 10⁵ cells of each cell line) were infected with NV1066 at an MOI 0.5 or 1.0 (as defined above, MOI refers to a multiplicity of infection, such as a ratio of viral particles to tumor cells) *in vitro* and incubated for 18 hours). The cells were analyzed for intensity in FL-1 channel (green fluorescence). The mean intensity of the eGFP-positive cells was compared with the mean intensity of the eGFP-negative uninfected cells.

In a separate experiment, cancer cells were mixed with normal cells in serial dilutions infected with 1 x 10⁷ plaque forming units (pfu) NV1 066 and incubated for 18 hours. The mean intensity of eGFP-positive cells (live and/or dead) was compared with the autofluorescence of the cells. In order to test the ability of NV1066 to infect cancer cells and express eGFP in infected cancer cells under different specimen transport conditions, a similar experiment was repeated with a mixture of cancerous and normal cells left for 12 hours at room temperature, in ice, or at 4° Celcius.

### VII. Identifying Limits of detection:

In order to measure the limit of detection of tumor cells in body fluids, serial dilutions of cancer cells were used to mix with normal cells. Accurate counts of harvested cancer cell suspension were made using trypan blue staining and manual counting using a hemocytometer. Cancer cells (0,10, 100, 1000, 10 000, or 100 000) were added to tubes that contained 10 million normal cells. In experiments involving fluorescent detection using microscopy, the samples were directly mixed and incubated in chamber slides (Lab-Tek; Nalge Nunc) to avoid any loss of cells. In experiments involving flow cytometry, samples were mixed and incubated directly in flow cytometry polystyrene tubes. After gentle mixing of the samples, 1x10⁷ pfu of NV1066 was added to the samples except for " negative controls. The samples were incubated for 18 hours in a humidified incubator supplied with 5% CO₂ and kept at a temperature of 37° Celcius. Following the incubation, samples were centrifuged, supernatant discarded, and the cell pellet was brought up in 1 ml of PBS (phosphate buffered saline) for analysis. In the event of small normal cells that could not be sedimented, the entire sample was used for analysis. Following spiking of normal cells with cancer cells; samples were analyzed by both fluorescent microscopy and flow cytometry. Each sample was analyzed in six replicates. For fluorescent microscopy, the slides were labeled randomly.

Ten observers from three different laboratories at the same academic institution reviewed these slides. The individual observer's scientific experience or experience with microscopy, varied between zero to three years. The observers were blinded to the actual concentration of mixture of cancer cells and normal cells and the presence of green cells in each sample. Observers examined the slides under both bright-field and fluorescent microscopy and recorded the presence or absence of green cell in each sample. For flow cytometry, two scientists who were blinded to the treatment analyzed the samples to identify the green cells. Each dilution of spiked sample was performed in six replicates. Data acquisition was performed with CellQuest (BDIS, San Jose, CA), and a multiparameter analysis was performed on FlowJo software.

### VIII. Confirmation of eGFP-expressing cells as cancer cells:

Cells were differentiated from any background artifacts on glass slides by staining live cells with Hoechst stain.

Cancer cells were identified using cancer cell markers. Human lung cancer cells, A549 and mesothelioma cells, NCI-H28, express integrin surface antigen (CD 51/61). Therefore these cells can be identified by red fluorescence when stained with R-Phycoerythrin (R-PE) conjugated anti-human CD51/61 monoclonal antibody (BD Biosciences) and examined under a microscope with TRITC filter. To confirm that green cells were indeed cancer cells, A549, and NCI-H28 cells were spiked with normal cells and incubated for 18 hours with NV1066, 1 x 10⁷ pfu. Following incubation, the mixtures of cells were harvested and washed with PBS and bovine serum albumin (BSA). Cell aggregates were disrupted gently using a pipette, and the resulting single cell suspension was incubated with R-PE conjugated mouse anti-human CD 51/6Imonoclonal antibody for 30 min. on ice. Samples containing antibody-stained cancer cells spiked with normal cells were analyzed both by fluorescent microscopy and by FACScan Flow Cytometer (Becton-Dickinson, San Jose, CA).

Microscopic detection of markers: slides were initially observed under bright-field to identify cells, and then examined for the presence of green cell under fluorescence microscopy; once a green cell was identified, the cell was confirmed to be a cancer cell by changing filters for identifying the nucleus (blue from Hoechst staining when examined under DAPI filter) and then by changing filters again for identifying R-PE conjugated integrin surface antigen (stained red when examined under TRITC filter). Pictures were taken with individual channel filters and were overlapped. For example, see, *Figure* 10.

Similar confirmation was performed by flow cytometry by correlating individually identified green cells on the FL-1 detector with the PE-staining by the FL-3 detector, using methods described supra.

### IX. Correlation of eGFP expression to HSV MOI:

Gastric cancer cells, OCUM-2MD3 (1 x 10⁶) were grown and infected with NV1066 at MOIs of 0.01, 0.1, and 1.0. Cells were harvested at 6, 12, and 24 hours after infection. eGFP-expressing cells were separated from non-GFP-expressing cells by fluorescence-activated cell sorting (MoFlo; Dako Cytomation, Fort Colling, CO) and fixed on slides for immunohistochemistry. Uninfected cells served as negative controls. Slides were stained by the improved biotin-streptavidin amplified method (Biogenex Supersensitive Detection System) using a polyclonal antibody to HSV-1. Slides were examined using a Zeiss Axiovert 200 microscope for the presence of herpes and for eGFP expression. Overlay images were created of the same field viewed under routine light microscopy and eGFP microscopy modes. Each condition was performed in triplicate. Similar methods and results are demonstrated in Figures 4 and 5.

### EXAMPLE 2

### Herpes Simplex Viral Mutant, NV1066, Infects a Range of Cancer Cell Lines In Vitro.

NV 1066 infectivity was tested in numerous cancer cell lines as described in Table 8 below. One hundred and eleven cancer cell lines were infected *in vitro* at an MOI of 0.01, 0.1 and 1.0. The cell lines listed became infected and expressed eGFP that was detected upon examination under fluorescent microscopy and by flow cytometry.

The inventors observed that *in vitro,* NV1066 infected the cell lines listed in Table 8 at MOIs of 0.01, 0.1 and 1.0. Specifically, NV1066 infected cancer cells expressed eGFP within 1 to 2 hours of incubation. After 18 hours incubation, the majority of cancer cells were infected and expressed eGFP. Although the virus was able to infect and express eGFP at a lower MOI of 0.1, at a higher MOI of 1.0, the majority of cancer cells in the sample were infected at an earlier time point and expressed strong eGFP fluorescence. The expressed eGFP was intracellular such that the fluorescence was detected by fluorescent microscope and by flow cytometry.

Thus NV1066 provides easily detectable levels of expressed eGFP in cancer cell lines.

**Table 8: Cancer cell lines that can be infected by NV1066 and express eGFP.**

| **Primary organ of Origin** | **Cell Lines** | **Body Fluid/s from whe cells for analysis can obtained** |
|---|---|---|
| Gastrointestinal | | Ascitic fluid, endoscopic biopsy or needle aspiration, nasogastric tube drainage, feces, rectal swab, needle aspiration cytology from lymph nodes |
| Esophagus | BE3, SKG-T4 | |
| Stomach | OCUM, MKN-1, MKN-45, MKN-74 | |
| Colorectal . | CT26, HCT-8, HCT-116, HT-29, HT- | |
| | 29 MDR | |
| Hepatobiliary | | Ascites, liver biopsy, needle aspiration cytology, biliary drainage |
| Hepatocellular carcinoma | Hep G2, Hep 3B, PLC/PRF/5, SKHep1, SNU-182, SNU-354, SNU-368, SNU-387, SNU-398, SNU-423, SNU-449, SNU-475, SNU-739, SNU-761, SNU-878, SNU-886 | |
| Cholangiocarcinoma | HUCCT-1, KMBC, SK-ChA-1, SNU-1079, SNU-1196, YoMi; | |
| Gall Bladder | MZ-ChA-1, TGBC-1, TGBC-2 | |
| Pancreas | Pan02, AsPC-1, BxPC-3, hs766t, HTB147, Panc-1, MLkPaCa-2, SNU-478, SNU-869 | Pacreatic fluid, ascites, endoscopic biopsy, needle aspiration cytology from lymph nodes |
| Lung | A549, H1299, 2030, H322, H522 | Sputum, bronchial lavage, pleural effusion, mediastinal lymph node biopsy |
| Mesothelioma | JMN, VAMT, MSTO-211H, H-2373, H-2052, H-2452, H-28, Meso, Meso1A, Meso-9, Meso-10 | Pleural effusion, ascites, mediastinal lymph node biopsy |
| Urinary | J82, RT4, T24, UMUC-3, SK UB DU-145, C4-2, CWR-22, CWR-22R, PC-3 | Urine, cystoscopic drainage prostatic secretions, endoscopic biopsy |
| Bladder | | |
| Prostate | | |
| Breast | HCC1500, HCC1937, HCC 1954, MCF-7, MCF-7 MDR, MDA-MB-231, MDA-MB-435, MDA-MB-435LN, MDA-MB-435S, SkBr3, T47D | Breast ductal drainage, nipple discharge, needle aspiration cytology, bone marrow cytology |
| Head and Neck | SCC VII, SCC15, SCC25, SCCQLL1, SCCQLL2, 686, 1586, 1986, 886, MSK922, MSK1493, LN1-LN7, MG1,MG11,MG14 | Oral secretions, lymph node aspiration cytology, oral swab, throat swab |
| Thyroid | NPA-187, WRO 82-1, DR081-1, DR090-1, ARO, KAT-4C, KAT-18 | Needle aspiration cytology |
| Uterine-cervix | HeLa | Pap smear |

### EXAMPLE 3

### Mean intensity of NV1066 infected cancer cells is 11 - 344 -fold higher than background autofluorescence.

In order to determine the level of eGFP expression over background cellular autofluorescence, fifteen representative human cancer cell lines (A- O, described below) were infected *in vitro* at an MOI of 1.0 (multiplicity of infection, ratio of viral particles per cancer cell), incubated for 18 hours, and analyzed by flow cytometry. Compared to background autofluorescence, infected eGFP expressing cancer cells showed higher mean intensity of green fluorescence (11 - 344 -fold higher, represented in logarithmic scale).

Because of this strong expression of eGFP, cancer cells in body fluids can be easily identified, even in a background of millions of cells or in cell clumps. *See,* Figure 2. (A - O cancer cells: lung - A549, H1299; bladder - UMUC-3, KU19-19; stomach - OCUM-2MD3; colorectal - HT29; hepatoma - HepG2; mesothelioma- MSTO-211H, JMN, H-Meso, H-28; breast MCF-7; head and neck - SCCVII, SCC25, MG11).

### EXAMPLE 4

### Fewer than One Cancer Cell per Million Normal Cells Can Be Diagnosed by Fluorescent Detection

The limits of detection of the NV1066 virus (vector) in defined populations of mixed cells ranging from 100% normal cells to 1 cancer cell in 1,000,000 cells are shown in Figure 3 and Table 9. Specifically, lung cancer cells were mixed with normal cells from bronchoalveolar lavage in ratios ranging from 1:10 to 1:1,000,000 and incubated with NV1066 for 18 hours. Cancer cells mixed with NV1066 served as a positive control, and normal cells mixed with NV1066 served as negative controls. The mean intensities, as analyzed by flow cytometry, of eGFP expressing cells in each sample were plotted. Cancer cells were detected by higher intensity of green fluorescence in up to one in a million without any difficulty. The mean intensity at a dilution of one cancer cell in a million normal cells is fifteen times higher than autofluorescence of cells.

The fluorescent intensity of the infected cancer cells is not compromised even if the infected cells are dead. NV1066-infected cells retain eGFP fluorescence and dead cells have a higher mean intensity than the cellular autofluorescence (1572 vs. 56) and hence can be easily detected either by fluorescent microscopy or flow cytometry.

The above set of experiments was repeated, and similar results were reproduced with the following experimental conditions: (a) human bladder cancer cells mixed with normal bladder cells, (b) human mesothelioma cells mixed with normal pleural cells, (c) gastric cancer cells mixed with cells obtained from peritoneal lavage, (d) human breast cancer cells mixed with normal breast epithelial cells or lymphocytes from lymph nodes, (e) human head and neck cancer cells mixed with lymphocytes from lymph nodes, (f) human hepatocellular cancer cells mixed with normal liver cells (data not shown).

Thus, this method of screening cancer cells is reproducible and may detect 1 in 1 million cells. *See,* Figure 3.

**Table 9: Cancer cells were mixed with normal cells at serial dilutions listed and incubated with NV1066 for 18 hours. Slides were labeled randomly including positive (100% green cells) and negative (no NV1066, no green cells, 0% green cells) controls. Ten independent observers who were blinded to the exact dilutions and treatment examined the slides and identified the presence or absence of green cell on each slide.**

| Ratio of Cancer Cells | Number of Slides Observed | Number Incorrect Observations | % Correct Observations (CI) |
|---|---|---|---|
| 0 | 60 | 2 | 97% (89%, 99%) |
| 1:10 | 60 | 0 | 100% |
| 1:100 | 60 | 0 | 100% |
| 1:1 000 | 60 | 0 | 100% |
| 1:10 000 | 60 | 0 | 100% |
| 1:100 000 | 60 | 3 | 95% (87%, 99%) |
| 1:1 000 000 | 60 | 5 | 92% (83%, 97%) |
| 1:10 -1:1 000 000 | 360 | 8 | 98% (96%, 99%) |

### EXAMPLE 5

### NV1066 infective ability is retained in cells preserved in ice or at room temperature for twelve hours

The sensitivity and repeatability for the parameters of this assay were measured. Lung cancer cells, A549 were preserved in incubator (37° C), ice (4° C) or room temperature (22° C) for 12 hours, and then infected with NV1066 at an MOI of 1.0. The percentage of eGFP-positive cells and the mean eGFP intensity of the positive cells were measured at 18 hours post-incubation. There was no statistically significant difference in the percentage of eGFP-positive cells in three experimental conditions (14%, 13% and 18% at 4°, 22°and 37° C). The mean intensity of eGFP positive cancer cells is 8 - 50 fold higher than the mean intensity of the normal cells in these three experimental conditions. Similar results were reproduced after repeating the experiment with different pathological types of cancer cells.

Thus, the infective ability of NV 1066 remains in both cells incubated at room temperature and on ice for providing a reproducible and simple method for identifying cancer cells.

### EXAMPLE 6

### GFP expression is due to No1.066 Infection and Replication

GFP expression corresponds to immunohistochemistry-proven NV1066 infection. After cultured Gastric cancer cells, OCUM-2MD3, were infected with NV1066 at MOIs of 0.01, 0.1, and 1.0, the cells were fluorescence-activated cell-sorted into eGFP-expressing and non-GFP-expressing populations. One hundred percent of eGFP-expressing cells were positive for HSV by immunohistochemistry. Of the cells sorted by flow cytometry that did not express eGFP, none were positive for HSV by immunohistochemistry.

This finding supports that eGFP expressed by the cancer cells is due to specific HSV infection and replication in cancer cells.

### EXAMPLE 7

### NV1066 selectively infects and/or replicates in malignant mesothelioma cancer cells and spares normal cells

In order to confirm that NV1066 selectively infects and/or replicates in cancer cells and spares normal cells, the inventors combined green fluorescence detection with immunohistochemistry for cancer cells. Mixture of malignant mesothelioma cells, NCI-H28 were incubated with NV1066 for 18 hours, then counterstained with R-PE conjugated anti-human CD51/61 monoclonal antibody. The green cells were confirmed as cancer cells because of their staining with R-PE conjugated mouse anti-human CD51/61 (Figure 4).

NV1066 selective infection and/or replication of cancer cells among a mixture of millions of normal cells was confirmed by counterstaining with immunohistochemistry. Human mesothelioma cancer cells were mixed with normal pleural cells (Figure 4A) and were incubated with NV1066 for 18 hours. Examination under fluorescence microscope identified cancer cells by expression of strong green fluorescence (Figure 4B). These cancer cells express integrin (CD 51/61) surface antigen. Incubation with R-Phycoerythrin (R-PE) conjugated mouse anti-human CD51/61 monoclonal antibody confirmed that eGFP expression is selective to cancer cells (identified by red fluorescence, Figure 4C). Overlap of fluorescent pictures with bright-field identifies cancer cells amongst normal cells (Figure 4D). Live cells amongst the cell clumps were identified by nuclear Hoechst staining (blue).

Thus malignant mesothelioma cells are easily identifiable as isolated cells and cells in clumps.

### EXAMPLE 8

### NV1066 selectively infects and/or replicates in lung cancer cells and spares normal cells.

NV1066 selective infection and/or replication in cancer cells among a mixture of millions of normal cells was confirmed by counterstaining with immunohistochemistry. Human lung cancer cells were mixed with normal bronchoalveolar cells (Figure 5A) and were incubated with NV1066 for 18 hours. These cancer cells express integrin (CD 51/61) surface antigen. Incubation with R-Phycoerythrin (R-PE) conjugated mouse anti-human CD51/61 monoclonal antibody identified cancer cells by red fluorescence (Figure 5B, overlap of bright-field and red fluorescence). Cancer cells were detected by expression of strong green fluorescence (Figure 5C, overlap of bright-field and green fluorescence). Overlap of fluorescent pictures with bright-field identifies cancer cells amongst normal cells (Figure 5D). Live cells amongst the cell clumps were identified by nuclear Hoechst staining (blue).

Thus lung cancer cells are easily identifiable as isolated cells and cells in clumps.

### EXAMPLE 9

### GFP positive lung cancer cells, can be identified against a background of millions of bronchoalveolar lavage cells.

A rare cancer cell in a mixture of millions of normal cells is difficult to identify under bright-field microscopy and is time consuming (Panel 6A). Under fluorescent microscopy, eGFP positive NV1066 infected cancer cells can be easily identified by means of green fluorescence (Panel 6B). Overlap of fluorescent picture with bright-field identifies the cancer cell (Panel 6C) for further studies.

Thus lung cancer cells are easily identifiable against a background of millions of bronchoalveolar lavage cells.

### EXAMPLE 10

### GFP positive bladder cancer cells can be identified against a background of millions of normal bladder cells.

A rare cancer cell in a mixture of millions of normal cells is difficult to identify under bright-field microscopy and is time consuming (Figure 7A). Under fluorescent microscopy, eGFP positive NV1066 infected cancer cells can be easily identified by means of green fluorescence (Figure 7B). Overlap of fluorescent picture with bright-field identifies the cancer cell (Figure 7C) for further studies.

Thus bladder cancer cells are easily identifiable against a background of millions of normal bladder cells.

### EXAMPLE 11

### Cancer cells can be easily and accurately diagnosed by Fluorescence Assisted Cytological Testing (FACT).

A rare cancer cell in a mixture of millions of normal cells is difficult to identify under bright-field microscopy (Figure 6A, 7A). Under fluorescent microscopy, eGFP positive NV1066 infected cancer cells can be easily identified by means of green fluorescence (Figure 6B, 7B). Overlap of the fluorescent picture with bright-field identifies the cancer cell (Figure 6C, 7C) for further histological testing.

Thus cancer cells can be easily and accurately identified by FACT.

### EXAMPLE 12

### Rare cancer cells can be detected with high sensitivity in body fluids.

Diagnosis of cancer cells by green fluorescence under fluorescent microscopy is uncomplicated and does not require training. In the previous experiment Example 4, Figure 3, where serial dilutions of cancer cells were spiked with millions of normal cells, ten independent observers identified the presence or absence of green cells on a given slide. For each dilution, there were a total of sixty observations. Appropriate positive and negative controls were included in the analysis.

Presence or absence of green cells was accurately identified (Table 8) with an overall sensitivity of 98% (CI 96 - 99%) by all observers, irrespective of their previous experience with microscopy or fluorescent detection. Even when the concentration (ratio) of cancer cells was one in a million of normal cells, the observers identified the presence of the green cell accurately with a sensitivity of 92% (CI 83 - 97%). In the absence of cancer cell in a negative control slide, two observers (out of ten) identified a green cell, which in fact was an artifact on the glass slide. This error was eliminated after the observers reexamined the greenness with DAPI filter for nuclear staining.

Thus a cancer cell can be easily and accurately identified at a concentration (ratio) of one cancer cell in a million of normal cells.

### EXAMPLE 13

### Rare cancer cells can be identified and sorted out by flow cytometry.

Flow cytometry was used to identify and sort NV1066 infected cancer cells from non-infected non-cancerous cells. Because of the strong emission of green fluorescence by NV1066 infected cancer cells compared to the background autofluorescence of uninfected normal cells, rare cancer cell amongst millions of normal cells can be easily identified by flow cytometry by gating in FL-1 channel. In Panel 8A, two million cells were sorted out by flow cytometry. In Panel 8B, amongst the same cell population, cancer cells were identified by strong green fluorescence in FL-1 channel. These rare cancer cells can be separated out for further histological studies by flow cytometric sorting.

From the above information, it is clear that the invention provides compositions and methods for ultra-sensitive screening for early cancer cell detection wherein a cancer cell may be detected within a mixed population of cancer cells and non-cancer cells.

### EXAMPLE 14

### Isolated cancer cells proliferation rate can be extrapolated from the intensity of the marker.

As shown in Figure 9, the intensity of the reporter molecule within a cancer cell was found to correlate with such cancer cell proliferation rate. Thus, the greater the marker intensity or presence, in a given cancer cell, the more probable that this cancer is rapidly proliferating and hence the more aggressive the cancer is going to be in that patient, which has important clinical implications.

### EXAMPLE 15

### Co-localization of an expressed gene, eGFP, with a cytokeratin cell surface marker for cancer cells.

Samples of tissue and fluid were obtained from pancreatic cancer resections. These cells were then infected with NV 1066 in order to show co-localization of an expressed gene, eGFP, with a cytokeratin cell surface marker for cancer cells.

As shown in Figure 10, samples obtained from pancreatic cancer resections were infected with NV1066. Following infection, samples were then stained with Hoechst (for nucleus - Blue), cytokeratin (for a cancer cell surface marker - Red) and GFP (for a cancer cell, Green). A-D) microscopic pictures showing that human cancer cells expressing cytokeratin are infected by NV1066 producing GFP; E) human pancreatic cancer cells obtained in a sample of a ductal lavage are infected by NV1066 and shown to produce GFP (right panels) compared to cell staining with '"Diff-Quick," a stain used by cytopathologists for cancer cell identification, see below, (left panels); and F & G) a mixture of cells (cell nuclei stained with Hoechst, blue), cancer cells stained with cytokeratin (red) showing cancer cells are infected by NV1066 and produce GFP (green) while normal cells (blue positive, red negative) are not infected by NV1066 and do not produce GFP (blue positive, green negative).

A "Diff Quick" Histological Staining kit refers to a three pack set of 1) Methanol fixative, 2) Buffered Eosin and 3) Phosphate buffered Azure B used with the following general staining procedure; 1. deparaffinize sections and hydrate to deionized water; 2. treat frozen sections and blood smears in Solution A; 3. dip slides 25 times in Solution B for optimum results; 4. dip slides 25 times in Solution C (Do not rinse slides before treating in Solution C); 5. rinse quickly under distilled water, 6. quickly check slides microscopically; repeat steps 3-4 if slides need more enhancement; 7. air dry slides; and 8. clear in Xylene and mount using synthetic mounting medium.

### EXAMPLE 16

### Detecting cancer cells in pancreatic juice (fluid) from pancreatic cancer patients.

Early and accurate diagnosis of periampullary malignancies provides the best chance for cure and spares patients with benign disease the morbidity of a major operation (Khalid, et al., (2005) Clin Lab Med 2005;25(1):101-16). However, current methods of detection are inadequate, as many periampullary tumors remain radiographically occult or are indistinguishable from inflammatory masses (Fukushima et al., (2003) Cancer Biol Ther;2(1):78-83; Bardales et al., (2006) Diagn Cytopathol;34(2):140-75; Prokesch et al., (2003, EurRadio1;13(9):2147-54; Rosty et al., (2002) Cancer Res;62(6):1868-75).

Pancreatic cancer, the most common periampullary malignancy, is a fatal disease with most patients dying within two years of diagnosis. Surgical resection provides the greatest chance for cure, but most patients present with locally advanced or metastatic disease at the time of diagnosis, precluding surgery. Early detection of resectable tumors is necessary to improve patients' outcome. However, despite advances in imaging and endoscopy, periampullary neoplasms are among the most challenging tumors to detect early (Walsh et al., (2003) Surg Endosc; 17(10): 1514-20). Thus, the inventors used fluorescence assisted cytological testing (FACT) in combination with NV1066-transduced eGFP expression to determine the detection level of cancer cells in pancreatic juice from pancreatic periampullary cancer patients.Thirty-eight consecutive patients with periampullary lesions underwent pancreaticoduodenectomy. Patients consented to providing samples under an institutional review board-approved protocol for tissue and fluid collection.

Immediately after resection, a tissue sample (specimen) was transported to the pathology suite, where the pancreatic duct was irrigated with 3 ml of saline. Aspirated pancreatic juice was collected in a sterile polypropylene conical, transported on ice to the laboratory, and centrifuged at 800 rpm x 5 minutes at 4° Celcius. Supernatant was discarded, and the cell pellet was resuspended in RPMI with 10% fetal calf serum, 100 µg/mL penicillin, and 100 µg/mL streptomycin.

One-third of a patient's specimen was incubated without virus as a negative control while the remainder was infected with 4.5 x 10⁵ plaque-forming units (pfu) of NV 1066. Samples were incubated for six hours in polystyrene round-bottom tubes (BD Falcon, San Jose, CA) in a humidified incubator supplied with 5% CO₂ and kept at a temperature of 37° Celcius. After incubation, cells were washed with phosphate-buffered saline (PBS) and cytospun at 1000 rpm x 4 minutes at room temperature onto saline-coated slides (Electron Microscopy Sciences, Hatfield, PA). Slides were fixed in 1% paraformaldehyde for 12 minutes at room temperature, washed twice in PBS, and stained with acidophilic and basophilic stains from the Quick-Dip (Hematology Stain) three-part staining system. For detection of green fluorescence, slides were examined with a Zeiss Axio2Imaging upright microscope with a 100W mercury arc lamp light source and Retiga EX CCD digital camera. Selective excitation of EGFP was produced through a Chroma 41017 filter set. Images were processed and analyzed with IPLab Imaging Software (Scanalytics, Rockville, MD).

NV1066-transduced green fluorescence was expressed exclusively in cancer cells in pancreatic juice. NV1066-mediated green fluorescence detected multiple epithelial pancreatic cancer types. Patient characteristics, final pathologic stage and grade, EGFP expression, and cytologic diagnosis are shown in Table 10. Figure 11 shows representative cells from a patient with pancreatic ductal adenocarcinoma, demonstrating that EGFP positive cells were also malignant by cytologic criteria. EGFP was not detected in inflammatory cells in a patient with chronic pancreatitis (Figure 11b) or in benign epithelial cells in a patient with a benign adenoma (Figure 11c).

After *ex vivo* incubation with NV1066, pancreatic juice specimens were also examined in blinded fashion for EGFP expression. Blinded attending pathologists examined specimens after modified hematoxylin and eosin staining (Diff Quik) for morphologic criteria of malignancy.

For conventional cytologic examination, slides were evaluated by attending pathologists who were blinded to the final histologic diagnosis. Standard morphologic cellular criteria were used to diagnose malignancy (Mitchell et al., (1985) Am J Clin Pathol;83(2):171-6; (Robin et al., (1995) Acta Cytologica;39(1):1-10 ). Green fluorescence and cytology interpretations were compared with the final histologic diagnoses of the pancreaticoduodenectomy specimens.

To confirm that green fluorescent cells were identifying malignant cells, a sample of the tissue was stained with monoclonal antibody B72.3, a murine IgG1 that is reactive with a wide range of carcinomas while demonstrating little or no reactivity to normal adult tissues. Immunohistochemistry for a Tumor-associated glycoprotein B72.3, TAG-72, (Biogenex Labs, San Ramon, CA), monoclonal antibody which recognizes pancarcinoma antigen was performed using a streptavidin-biotin peroxidase detection method at a 1:200 dilution to produce a brown reaction product (for example, see, Carrasquillo et al., (1988) Radiology, Apr;167(1):35-40; Canasquillo, et al., (1988) J Nucl Med. 1988 Jun;29(6):1022-30).

On final histologic diagnosis, 31 patients had invasive periampullary tumors, 2 had noninvasive pancreatic carcinoma, and 5 had benign pancreatic lesions. Using compositions and methods of the present inventions, cancer was diagnosed positive by green fluorescence in 18 of 24 (75%) patients with invasive pancreatic or bile duct tumors, while 12 of 24 (50%) patients were diagnosed positive by conventional cytology. Benign specimens did not express green fluorescence. Green fluorescent cells were confirmed as being malignant by conventional cytology and immunohistochemistry. Untrained observers diagnosed cancer by observing green fluorescence in cells with 97% agreement between observers.

**Table 10. Patient characteristics; EGFP and cytology results.**

| **Case** | **Age/sex** | **Tumor type** | **Stage**** | **Grade** | **EGFP** | **Cytology** |
|---|---|---|---|---|---|---|
| 1 | 81/F | PDAC | 2B (T3N1) | G2 | + | + |
| 2 | 83/M | PDAC | 2A (T3N0) | G2 | + | + |
| 3 | 73/F | PDAC | 2B (T3N1) | G1-2 | + | + |
| 4 | 76/F | PDAC | 2B(T3N1) | G2 | + | + |
| 5 | 64/F | PDAC | 2B (T3N1) | G1 | - | - |
| 6 | 48/M | PDAC | 2B (T3N1) | G3 | + | + |
| 7 | 66/F | PDAC | 2B(T3N1) | G2 | + | + |
| 8 | 79/F | PDAC | 2A (T3N0) | G2 | + | + |
| 9 | 79/F | PDAC | 2B (T3N1) | G2-3 | + | - |
| 10 | 82/F | PDAC | 2A (T3N0) | G2-3 | - | - |
| 11 | 80/F | PDAC | 2A (T3N0) | G2-3 | + | + |
| 12 | 61/M | PDAC | 2A (T3N0) | G3 | + | - |
| 13 | 67/M | PDAC | 2A (T3N0) | G2 | - | - |
| 14 | 73/M | PDAC | 2A (T3N0) | G2 | + | + |
| 15 | 79/M | PDAC | 2B (T3N1) | G2-3 | + | - |
| 16 | 64/F | PDAC | 2B (T3N1) | G2 | - | - |
| 17 | 62/M | PDAC | 2B (T3N1) | G3 | + | - |
| 18 | 78/M | PDAC | 2B (T3N1) | G3 | - | - |
| 19 | 77/F | PDAC | 1B (T2N0) | G3 | + | + |
| 20 | 68/M | Invasive IPMC | 2B (T3N1) | | + | - |
| 21 | 64/M | Invasive IPMC | 1A (T1N0) | | + | + |
| 22 | 66/M | Noninvasive IPMC | 0 (Tis) | | + | + |
| 23 | 50/M | Noninvasive IPMC | 0 (Tis) | | - | - |
| 24 | 66/F | IPMN adenoma | | | - | - |
| 25 | 67/M | IPMN adenoma | | | - | - |
| 26 | 78/M | Cholangiocarcinoma | 1B (T2N0) | G2 | + | + |
| 27 | 59/F | Cholangiocarcinoma | 2B (T3N1) | G2 | - | - |
| 28 | 54/M | Cholangiocarcinoma | 1B (T2N0) | G2-3 | + | - |
| 29 | 56/F | Ampullary adenocarcinoma | 2B (T2N1) | G2 | + | + |
| 30 | 50/M | Ampullary | 3 (T4N1) | G3 | - | - |
| 31 | 85/M | adenocarcinoma Ampullary adenocarcinoma | 1B (T2N0) | G2 | - | - |
| 32 | 46/M | Ampullary adenocarcinoma | 3 (T4N1) | G1 | - | - |
| 33 | 70/M | Duodenal adenocarcinoma | 1 (T1N0) | G1 | - | - |
| 34 | 55/M | Duodenal adenocarcinoma | 3 (T3N1) | G3 | - | - |
| 35 | 66/M | Duodenal adenocarcinoma | 2 (T3N0) | G3 | - | - |
| 36 | 49/M | Localized neuroendocrine tumor* | | | - | - |
| 37 | 45/F | Mucinous cystadenoma* | | | - | - |
| 38 | 74/F | Chronic pancreatitis | | | - | - |

| | | | | | | |
|---|---|---|---|---|---|---|
| ** Degree of differentiation and stages were assigned following the recommendations of the American Joint Committee on Cancer (AJCC) after examination by pathology (American Joint Committee on Cancer. Exocrine Pancreas. AJCC Cancer Staging Manual. 6th ed. New York: Springer; 2002. p. 157-64; herein incorporated by reference). PDAC = pancreatic ductal adenocarcinoma. IPMN = intraductal papillary mucinous neoplasm. IPMC = intraductal papillary mucinous carcinoma.* = of the pancreas. | | | | | | |

EGFP expression did not depend upon tumor grade, stage, or patient characteristics. The sensitivities of green fluorescence and conventional cytology for detecting neoplastic cells from various periampullary tumor types are shown in Table 11. Twenty-four of 38 patients had invasive tumors of the pancreas or bile duct; 19 had pancreatic ductal adenocarcinoma, 2 had invasive intraductal papillary mucinous carcinoma, and 3 had cholangiocarcinoma. Among these 24 patients, cancer was diagnosed by green fluorescence in 18 (75%) patients, whereas conventional cytology identified cancer cells in 12 (50%) patients. Seven patients had ampullary or duodenal adenocarcinoma, with one (14%) sample positive by both green fluorescence and conventional cytology. One of two patients with noninvasive intraductal papillary mucinous carcinoma had tumor cells detected by both green fluorescence and cytology. Benign adenomas (n=3), chronic pancreatitis (n=1), and nonmetastatic pancreatic neuroendocrine tumor (n=1) were negative for malignancy by green fluorescence and cytology.

**Table 11. Sensitivity of eGFP and conventional cytology for the detection of various periampullary tumor types from pancreatic juice.**

| Tumor type | eGFP | Conventional cytology |
|---|---|---|
| Pancreatic ductal adenocarcinoma, n=19 | 74% | 53% |
| Invasive intraductal papillary mucinous neoplasm, n=2 | 100% | 50% |
| Cholangiocarcinoma, n=3 | 66% | 33% |
| Ampullary adenocarcinoma, n=4 | 25% | 25% |
| Duodenal adenocarcinoma, n=3 | 0% | 0% |
| Total, n=31 | 61% | 42% |

### Blinded review.

Blinded observers accurately and reliably diagnosed malignancy based on eGFP detection. Fifteen observers without pathology training examined 10 slides each of NV1066-infected pancreatic juice, for a total of 150 observations (Table 12). Five slides contained pancreatic ductal adenocarcinoma cells for a total of 75 observations, while five slides represented patients with benign pancreatic lesions, for another 75 observations. Of the 75 observations of pancreatic ductal adenocarcinoma, 73 (97%) were correctly identified as positive for malignancy based on green fluorescence. Of the 75 observations of benign pancreatic lesions, 74 (99%) were correctly identified as negative for malignancy based on the absence of green fluorescence.

To evaluate interobserver reliability and ease of eGFP detection, 15 blinded observers without pathology training examined 10 cytospun slides of pancreatic juice; 5 slides from patients with pancreatic ductal adenocarcinoma and 5 slides of benign pancreatic lesions. They interpreted the slides as positive or negative for malignancy based on the presence or absence of green fluorescence.

**Table 12. Blinded observers' diagnosis of malignancy based on the detection of green fluorescence.**

| | Histopathologic diagnosis | |
|---|---|---|
| Observers' diagnosis | Pancreatic ductal | Benign pancreatic lesion |
| | adenocarcinoma (n=75) | (n=75) |
| Positive for malignancy | 73 (97%) | 1 (1%) |
| Negative for malignancy | 2 (3%) | 74 (99%) |

FACT diagnosed malignancy in samples that were indeterminate by conventional cytology.

Pancreatic juice from a patient with pancreatic ductal adenocarcinoma displayed prominent green fluorescence (Figure 12a). However, conventional cytologic examination yielded an inconclusive diagnosis by three independent attending pathologists (Figure 12b). To confirm green fluorescent cells were malignant, immunohistochemistry was performed for B72.3, an antibody against tumor-associated glycoprotein 72 that is specific for carcinoma but absent in benign epithelium (Adsay et al., (1996) Am J Surg Pathol;20(8):980-94; Klimstra et al., (1994) International Journal of Pancreatology;16(2-3):224-5; Suriawinata et al., (2000) Modem Pathology;15(2):294A ). Green fluorescent cells stained positively with B72.3 (Figure 12c), thus validating EGFP as a marker of malignancy.

### EXAMPLE 17

### Detecting cancer cells in pleural fluid from lung cancer patients.

The inventors further tested compositions and methods of the present inventions on clinical specimens obtained from lung cancer patients. Test specimens were obtained from patients known to have cancer cells in their pleural fluids. Further, these patients were being treated for malignant pleural effusion by having their pleural fluid drained by chest tube placement. For comparison, control fluid specimens with no cancer cells were obtained from patients with a chest tube placed for mechanical reasons.

NV1066 (2 X 10⁵ particles) detected lung cancer in all specimens tested from patients with malignant pleural effusions. No fluoresce was detected in control samples. Representative microscope slides are shown in Figure 13. None of the cells, such as benign mesothelial cells (Fig 13a), showed green fluorescence. Specimens with non-small cell lung cancer clearly expressed eGFP green fluorescence (Fig. 13c-f).

### Patients' pleural fluid samples.

Studies were performed after consent under an institutional review board-approved protocol for tissue and fluid collection. Pleural fluid was collected from two patients with malignant pleural effusions being treated with tube thoracostomy for the effusion, and one patient was studied who did not have cancer in the pleural fluid but was subjected to placement of a chest tube intraoperatively. Specimens were obtained from the tube drainage and immediately transported on ice to the laboratory. Ten separate aliquots from each patient were studied (total samples with suspected cancer=20, total controls=10). Pleural fluid specimens were centrifuged at 800 rpm x 5 minutes at 4° C, and resuspended in RPMI with 10% fetal calf serum, 100 µg/mL penicillin, and 100 µg/mL streptomycin. Cells were counted using a hemocytometer, and 5 x 10⁵ cells were aliquoted into polystyrene round-bottom tubes (BD Falcon, San Jose, CA). Ten aliquots were incubated without virus as negative controls at 37° C in a 5% CO₂ incubator. The thirty experimental aliquots were incubated each with 2 x 10⁵ plaque-forming units of NV 1066.

After an eight-hour incubation, cells were washed with phosphate-buffered saline (PBS) and cytospun for 4 minutes at 1000 rpm onto silane-coated slides (Electron Microscopy Sciences, Hatfield, PA). Slides were fixed with 1% paraformaldehyde at room temperature for 12 minutes, washed twice with PBS, and stained with acidophilic and basophilic stains from the Quick-Dip staining system (Mercedes Medical, Sarasota, FL). Bright field and fluorescence microscopy was performed using a Zeiss Axio2Imaging upright microscope with a 100W mercury arc lamp light source and Retiga EX CCD digital camera. For detection of green fluorescence, selective excitation of eGFP was produced through a Chroma 41017 filter set. Images were processed and analyzed with Volocity Imaging Software (Improvision Inc., Lexington, MA). Conventional cytologic assessment was performed by an attending pathologist blinded to the clinical condition and to the treatment.

### EXAMPLE 18

### Detecting cancer cells in pleural fluid from lung cancer patients using a vaccia virus.

The inventors' duplicated compositions and methods of the present inventions on human lung cancer cell lines, using similar methods as described above, with the substitution of a vaccinia virus, GLV-1h68.

The inventors showed that GLV-lh68 provided sensitive and replicable cancer cell detection of at least one cancer cell in a background of one mi Ilion non-cancer cells. Further, the inventors showed that GLV-1h68 was capable of detecting one cancer cell in a background of one million non-cancer cells at Mol's of 0.0001.

Specifically, Figure 14 shows an exemplary experiment of detecting cancer cells in a mixture of 1 x 10² (le2) MSTO211H mesothelioma cancer cells (human) mixed with 1 x 10⁸ (1e8) rat hepatocytes, plated and infected with 1x10⁴ (1e4) pfus of GLV-1h68 (MOI=0.0001), 24-48 hours after infection, visualized under fluorescent microscopy.

Further, Figure 15 shows exemplary 1 x 10³ (1e3) MSTO211H mesothelioma cancer cells mixed with 1x10⁶ (1e6) rat hepatocytes, plated and infected with 1x10³ (1e3) pfus of GLV-1h68 (MOI=0.0001), 24-48 hours after infection, visualized under fluorescent microscopy.

Even further, Figure 16 shows exemplary 1x10³ (1e3) MSTO211 mesothelioma cancer cells mixed with 1x10⁶ (1e6) rat hepatocytes, plated and infected with 1x10³ (1e3) pfus of GLV-1h68 (MOI=0.0001), 24-48 hours after infection, visualized under fluorescent microscopy.

This study was performed on pancreatic juice obtained by lavaging the pancreatic duct from resected pancreaticoduodenectomy specimens to simulate cytologic biopsy. In addition, the inventors contemplate applying viral detection compositions and methods of the present inventions combined with FACT to other cytology specimens such as urine, sputum, and peritoneal fluid for detection of a wide range of tumor types.

Thus, tissue and fluid obtained from pancreatic cancer resections can be used to identify cancer cells and can be used to co-localize cell markers in relation to cancer cells. Further, the results shown herein, demonstrated that FACT, through *ex vivo* incubation of pancreatic juice with NV1066, was used to diagnose periampullary malignancies. Thus, NV1066 in combination with FACT, is a facile, accurate, and reliable method of cancer diagnosis and a potentially powerful, widely applicable adjunctive tool to conventional cytology.

## Claims

1. A method for detection of a cancer cell in a human cell sample, comprising,
a) providing,
i) a virus that is replication-competent conditional to a cancer cell comprising a reporter gene capable of expressing a reporter molecule, and
ii) a human cell sample comprising primary cells, and
b) contacting at least a portion of said cell sample in vitro with said virus to create a treated sample, and
c) detecting the expression of the reporter molecule for detecting a cancer cell in said treated sample, wherein said cancer cell is in a background of at least ten thousand non-cancer cells.

2. The method of claim 1, wherein said virus is a herpes virus, preferably NV1066.

3. The method of claim 2, wherein said contacting with said herpes virus is at a multiplicity of infection of 0.1-5.0, preferably 0.5-1.0.

4. The method of claim 1, wherein said virus is a vaccinia virus.

5. The method of claim 1, wherein said reporter gene encodes a protein selected from the group consisting of Green Fluorescent Protein, enhanced Green Fluorescent Protein, Blue Fluorescent Protein, Cyan Fluorescent Protein, Yellow Fluorescent Protein, firefly luciferase, renilla luciferase, β-galactosidase, chloramphenicol acetyltransferase, alkaline phosphatase, and horseradish peroxidase, wherein the reporter gene is under the control of a constitutive promoter, wherein said promoter is preferably selected from the group consisting of a cytomegalovirus promoter, a Simian Virus 40 promoter, and a synthetic vaccinia virus early/late promoter.

6. The method of claim 1, wherein the reporter gene is under the control of a promoter from the replication-competent virus, wherein said replication-competent virus promoter is a herpes simplex virus-1 promoter, wherein said herpes simplex virus-1 promoter is selected from the group consisting of a thymidine kinase β-promoter, a unique short₁₁ promoter, and an α-promoter of the infected cell protein₄ gene or a vaccinia promoter, wherein said vaccinia promoter is a synthetic early/late promoter.

7. The method of claim 1, wherein said cell sample comprises a cancer cell, wherein said cancer cell is preferably selected from the group consisting of a gastro-intestinal cancer cell, a hepatobiliary cancer cell, a gall bladder cancer cell, a pancreatic cancer cell, a lung cancer cell, a mesothelioma cancer cell, a bladder cancer cell, a prostate cancer cell, a breast cancer cell, a head cancer cell, a neck cancer cell, a thyroid cancer cell, a uterine cancer cell, a cervix cancer cell, a uterine-cervix cancer cell, a blood cancer cell, a white blood cancer cell, a bone marrow cancer cell, a pleural cancer cell, and a pleural fluid cancer cell.

8. The method of claim 1, further comprising the step of contacting the treated human cell sample with an antibody recognizing a molecule selected from the group consisting of a cytokeratin molecule, integrin molecule CD51/61, a Tag72, and a p53 molecule.

9. The method of claim 1, further comprising one of the steps of contacting the treated human cell sample with a nuclear stain, or counterstaining said treated human cell sample, wherein said counterstaining preferably comprises contacting said cell sample with a counterstain selected from the group consisting of a Hoechst stain, a trypan blue stain, an ethidium bromide stain, a 7-amino actinomycin D stain and an antibody stain.

10. The method of claim 1, wherein said reporter molecule is detected between one and eighteen hours after contacting said cell sample, further preferred between one and six hours after contacting said cell sample.

11. The method of claim 1, wherein said detecting comprises fluorescence assisted cytological testing, or wherein said detecting comprises using an instrument selected from the group consisting of a microscope, a luminometer, a fluorescent microscope, a confocal laser scanning microscope, and a flow cytometer.

12. The method of claim 1, wherein said human is a patient, wherein said patient is suspected of having cancer.

13. The method of claim 1, wherein said cell sample was obtained from a bodily fluid.

14. The method of claim 1, wherein said cell sample may derive from the group consisting of saliva, sputum, mucus, amniotic fluid urine, cerebrospinal fluid, blood, plasma, and serum.

15. The method of claim 1, wherein said cell sample may derive from blood.

16. The method of claim 1, wherein said cell sample may derive from pleural fluid.

17. The method of claim 1, wherein said detected cancer cell is an aggressive cancer cell.

## Patentansprüche

1. Ein Verfahren zur Detektion einer Krebszelle in einer humanen Zellprobe, umfassend,
a) die Bereitstellung,
i) eines Virus der für eine Krebszelle bedingt replikations-kompetent ist und ein Reportergen umfasst das in der Lage ist ein Reportermolekül zu exprimieren, und
ii) einer humanen Zellprobe umfassend primäre Zellen, und
b) in Kontakt bringen zumindest eines Teils der Zellprobe in vitro mit dem Virus um eine behandelte Probe zu erhalten, und
c) die Detektion der Expression des Reportermoleküls um eine Krebszelle in der behandelten Probe zu detektieren, wobei die Krebszelle in einem Hintergrund von mindestens zehntausend Nicht-Krebszellen ist.

2. Das Verfahren nach Anspruch 1, wobei das Virus ein Herpesvirus ist, vorzugsweise NV1066.

3. Das Verfahren nach Anspruch 2, wobei das in Kontakt bringen mit dem Herpesvirus bei einer Multiplizität der Infektion von 0,1 - 5,0 erfolgt, vorzugsweise von 0,5 - 1,0.

4. Das Verfahren nach Anspruch 1, wobei das Virus ein Vaccinia-Virus ist.

5. Das Verfahren nach Anspruch 1, wobei das Reportergen für ein Protein kodiert ausgewählt aus der Gruppe bestehend aus Grün Fluoreszierenden Protein, enhanced Grün Fluoreszierenden Protein, Blau Fluoreszierenden Protein, Cyan Fluoreszierenden Protein, Gelb Fluoreszierenden Protein, Firefly-Luciferase, Renilla-Luciferase, β-Galactosidase, Chloramphenicol-Acetyltransferase, alkalische Phosphatase und Meerrettichperoxidase, wobei das Reportergen unter der Kontrolle eines konstitutiven Promotors steht und der Promotor vorzugsweise ausgewählt ist aus der Gruppe bestehend aus einem Cytomegalovirus-Promotor, einem Simian-Virus 40-Promotor, und einem synthetischen Vaccinia-Virus früh/spät-Promotor.

6. Das Verfahren nach Anspruch 1, wobei das Reportergen unter der Kontrolle eines Promotors von dem replikations-kompetenten Virus steht, wobei der replikationskompetente Viruspromotor ein Herpes simplex Virus-1-Promotor ist, wobei der Herpes simplex Virus-1-Promotor ausgewählt ist aus der Gruppe bestehend aus einem Thymidinkinase β-Promotor, einem unikalen short₁₁ Υ-Promotor und einem α-Promotor des infizierten Zellprotein₄-Gens oder ein Vaccinia-Promotor, wobei der Vaccinia-Promotor ein synthetischer früh/spät-Promotor ist.

7. Das Verfahren nach Anspruch 1, wobei die Zellprobe eine Krebszelle umfasst, wobei die Krebszelle vorzugsweise ausgewählt ist aus der Gruppe bestehend aus einer Magen-Darm-Krebszelle, einer hepatobiliären Krebszelle, einer Gallenblasenkrebszelle, einer Bauchspeicheldrüsenkrebszelle, einer Lungenkrebszelle, einer Mesotheliom-Krebszelle, einer Blasenkrebszelle, einer Prostata-Krebszelle, einer Brustkrebszelle, einer Kopf-Krebszelle, einer Halskrebszelle, einer Schilddrüsenkrebszelle, einer Gebärmutterkrebszelle, einer Muttermundkrebszelle, einer Gebärmutterhalskrebszelle, einer Blutkrebszelle, einer weißen Blutkörperchernkrebszelle, einer Knochenmarkblutkrebszelle, einer Pleurakrebszelle und einer Pleuraflüssigkeitskrebszelle.

8. Das Verfahren nach Anspruch 1, welches des Weiteren den Schritt des in Kontakt bringen der behandelten humanen Zellprobe mit einem Antikörper umfasst welcher ein Molekül erkennt ausgewählt aus der Gruppe bestehend aus einem Zytokeratinmolekül, einem Integrinmolekül CD51/61, einem TAG-72 und einem p53 Molekül.

9. Das Verfahren nach Anspruch 1, welches des Weiteren einen der Schritte des Kontaktierens der behandelten humanen Zellprobe mit einem Kernfarbstoff oder die Gegen- bzw. Kontrastfärbung der behandelten humanen Zellprobe umfasst, wobei die Gegen- bzw. Kontrastfärbung vorzugsweise das Kontaktieren der Zellprobe mit einem Gegenfärbungs- bzw. Kontrastmittel umfasst welches ausgewählt ist aus der Gruppe bestehend aus einem Höchst-Farbstoff, einem Trypanblau-Farbstoff, einem Ethidiumbromid-Farbstoff, einem 7-Aminoactinomycin D-Farbstoff und einem Antikörperfarbstoff.

10. Das Verfahren nach Anspruch 1, wobei das Reportermolekül zwischen einer und achtzehn Stunden nach dem Kontaktieren der Zellprobe detektiert wird, des Weiteren vorzugsweise zwischen ein und sechs Stunden nach dem Kontaktieren der Zellprobe.

11. Das Verfahren nach Anspruch 1, wobei das Detektieren das Fluoreszenz-basierte zytologische Testen (FACT) umfasst oder wobei das Detektieren die Verwendung eines Geräts umfasst ausgewählt aus der Gruppe bestehend aus einem Mikroskop, einem Luminometer, einem Fluoreszenzmikroskop, einem konfokalen Laserrastermikroskop und einem Durchflusszytometer.

12. Das Verfahren nach Anspruch 1, wobei der Mensch ein Patient ist, wobei bei dem Patienten Verdacht auf Krebs besteht.

13. Das Verfahren nach Anspruch 1, wobei die Zellprobe von einer Körperflüssigkeit stammt.

14. Das Verfahren nach Anspruch 1, wobei die Zellprobe stammen kann aus einer Gruppe bestehend aus Speichel, Auswurf, Schleim, amniotische Urinflüssigkeit, zerebrospinale Flüssigkeit, Blut, Plasma und Serum.

15. Das Verfahren nach Anspruch 1, wobei die Zellprobe von Blut stammen kann.

16. Das Verfahren nach Anspruch 1, wobei die Zellprobe von Pleuraflüssigkeit stammen kann.

17. Das Verfahren nach Anspruch 1, wobei die detektierte Krebszelle eine aggressive Krebszelle ist.

## Revendications

1. Procédé de détection d'une cellule cancéreuse dans un échantillon cellulaire humain, consistant à :
a) fournir
i) un virus comprenant un gène rapporteur capable d'exprimer une molécule rapporteur et dont la réplication est conditionnée par une cellule cancéreuse, et
ii) un échantillon cellulaire humain comprenant des cellules primaires, et
b) mettre en contact au moins une partie dudit échantillon cellulaire in vitro avec ledit virus pour créer un échantillon traité, et
c) détecter l'expression de la molécule rapporteur pour détecter une cellule cancéreuse dans ledit échantillon traité, ladite cellule cancéreuse pouvant être détectée parmi au moins dix mille cellules non cancéreuses.

2. Procédé selon la revendication 1, dans lequel ledit virus est un virus herpès, de préférence, NV1066.

3. Procédé selon la revendication 2, dans lequel ladite mise en contact avec ledit virus herpès se fait à une multiplicité d'infection de 0,1 à 5,0, de préférence, 0,5 à 1,0.

4. Procédé selon la revendication 1, dans lequel ledit virus est un virus de la vaccine.

5. Procédé selon la revendication 1, dans lequel ledit gène rapporteur code pour une protéine choisie dans le groupe constitué par la protéine verte fluorescente, la protéine verte fluorescente améliorée, la protéine bleue fluorescente, la protéine cyan fluorescente, la protéine jaune fluorescente, la luciférase de la luciole, la luciférase de rénilla, la β-galactosidase, la chloramphénicole acétyl transférase, la phosphatase alcaline et la péroxydase de raifort, dans lequel le gène rapporteur est sous le contrôle d'un promoteur constitutif, dans lequel ledit promoteur est de préférence choisi dans le groupe constitué par un promoteur du cytomégalovirus, un promoteur du virus Simien 40 et un promoteur précoce/tardif du virus de la vaccine synthétique.

6. Procédé selon la revendication 1, dans lequel le gène rapporteur est sous le contrôle d'un promoteur issu du virus réplicatif, dans lequel ledit promoteur de virus réplicatif est un promoteur de l'herpès simplex virus de type 1, dans lequel ledit promoteur de l'herpès simplex virus de type 1 est choisi dans le groupe constitué par un β-promoteur de la thymidine kinase, un unique promoteur γ court₁₁, et un promoteur α du gène de la protéine₄ de cellule infectée ou un promoteur de la vaccine, ledit promoteur de la vaccine étant un promoteur précoce/tardif synthétique.

7. Procédé selon la revendication 1, dans lequel ledit échantillon cellulaire comprend une cellule cancéreuse, dans lequel ladite cellule cancéreuse est de préférence choisie dans le groupe constitué par une cellule du cancer gastro-intestinal, une cellule du cancer hépatobiliaire, une cellule cancéreuse de la vésicule biliaire, une cellule cancéreuse pancréatique, une cellule cancéreuse pulmonaire, une cellule cancéreuse de mésothéliome, une cellule cancéreuse de la vessie, une cellule cancéreuse de la prostate , une cellule cancéreuse du sein, une cellule cancéreuse de la tête, une cellule cancéreuse du cou, une cellule cancéreuse de la thyroïde, une cellule cancéreuse de l'utérus, une cellule cancéreuse du col de l'utérus, une cellule cancéreuse appartenant au col utérin et à l'utérus, une cellule cancéreuse sanguine, une cellule cancéreuse leucocytaire, une cellule cancéreuse de la moelle osseuse, une cellule cancéreuse pleurale et une cellule cancéreuse du liquide pleural.

8. Procédé selon la revendication 1, comprenant en outre l'étape consistant à mettre en contact l'échantillon cellulaire humain traité avec un anticorps reconnaissant une molécule choisie dans le groupe constitué par la cytokératine, l'intégrine CD51/61, la TAG-72 et la p53.

9. Procédé selon la revendication 1, comprenant en outre l'une des étapes de mise en contact de l'échantillon cellulaire humain traité avec un colorant nucléaire, ou de contre-coloration dudit échantillon cellulaire humain traité, dans lequel ladite contre-coloration consiste de préférence à mettre en contact ledit échantillon cellulaire avec un contre-colorant choisi dans le groupe constitué par le colorant de Hoechst, le bleu trypan, le bromure d'éthidium, la 7-amino actinomycine D et un anticorps.

10. Procédé selon la revendication 1, dans lequel ladite molécule rapporteur est détectée entre une et dix-huit heures après la mise en contact avec ledit échantillon cellulaire, de manière davantage préférée entre une et six heures après la mise en contact avec ledit échantillon cellulaire.

11. Procédé selon la revendication 1, dans lequel ladite détection comprend l'essai cytologique assisté par fluorescence, ou dans lequel ladite détection comprend l'utilisation d'un instrument choisi dans le groupe constitué par un microscope, un luminomètre, un microscope à fluorescence, un microscope confocal à balayage laser et un cytomètre de flux.

12. Procédé selon la revendication 1, dans lequel ledit humain est un patient, ledit patient étant suspecté d'avoir un cancer.

13. Procédé selon la revendication 1, dans lequel ledit échantillon cellulaire a été obtenu à partir d'un fluide corporel.

14. Procédé selon la revendication 1, dans lequel ledit échantillon cellulaire peut provenir du groupe constitué par de la salive, un crachat, du mucus, un liquide amniotique, de l'urine, un liquide céphalorachidien, du sang, du plasma et du sérum.

15. Procédé selon la revendication 1, dans lequel ledit échantillon cellulaire peut provenir du sang.

16. Procédé selon la revendication 1, dans lequel ledit échantillon cellulaire peut provenir de liquide pleural.

17. Procédé selon la revendication 1, dans lequel ladite cellule cancéreuse détectée est une cellule cancéreuse agressive.
